# EUROPÄISCHE PATENTSCHRIFT

(11) **EP 0 874 645 B1**
(45) Veröffentlichungstag und Bekanntmachung des Hinweises auf die Patenterteilung: **03.04.2002**
(21) Anmeldenummer: 97902179.7
(22) Anmeldetag: 16.01.1997
(51) Int. Cl.: A61K 49/00

(54) **PERFLUORALKYLHALTIGE METALLKOMPLEXE UND IHRE VERWENDUNG IN DER NMR-DIAGNOSTIK**
PERFLUOROALKYL-CONTAINING METAL COMPLEXES AND THEIR USE IN NMR DIAGNOSTICS
COMPLEXES METALLIQUES CONTENANT DU PERFLUOROALKYLE ET LEUR UTILISATION DANS LE DIAGNOSTIC PAR RMN

(30) Priorität: 19.01.1996 DE 19603033
(43) Veröffentlichungstag der Anmeldung: 04.11.1998
(73) Patentinhaber: SCHERING AKTIENGESELLSCHAFT, 13353 Berlin (DE)
(72) Erfinder: PLATZEK, Johannes, D-12621 Berlin (DE); NIEDBALLA, Ulrich, D-14195 Berlin (DE); RADÜCHEL, Bernd, D-13465 Berlin (DE); SCHLECKER, Wolfgang, D-12047 Berlin (DE); WEINMANN, Hanns-Joachim, D-14129 Berlin (DE); FRENZEL, Thomas, D-12247 Berlin (DE); MISSELWITZ, Bernd, D-13439 Berlin (DE); EBERT, Wolfgang, D-12203 Berlin (DE)
(86) Internationale Anmeldenummer: EP9700209
(87) Internationale Veröffentlichungsnummer: WO9726017

(56) Entgegenhaltungen:
- EP-A- 0 592 306
- EP-A- 0 628 316
- US-A- 5 248 498
- US-A- 5 401 493
- LEE H ET AL: "In vivo fluorine-19 MR imaging: relaxation enhancement with Gd-DTPA." J MAGN RESON IMAGING, JUL-AUG 1994, VOL. 4, NO. 4, PAGE(S) 609-13, XP002041354
- THOMAS SR ET AL: "Evaluation of the influence of the aqueous phase bioconstituent environment on the F-19 T1 of perfluorocarbon blood substitute emulsions." J MAGN RESON IMAGING, JUL-AUG 1994, VOL. 4, NO. 4, PAGE(S) 631-5, XP002041355
- GONG B ET AL: "PARAMETER OPTIMIZATION AND CALIBRATION OF FLUORINE-19 MAGNETIC RESONANCE IMAGING AT 1.5 TESLA" MAGN RESON IMAGING, 1991, VOL. 9, NO. 1, PAGE(S) 101-106., XP002041356
- RATNER A V ET AL: "FLUORINE-19 RELAXATION RATE ENHANCEMENT AND FREQUENCY SHIFT WITH GADOLINIUM-DTPA" INVEST RADIOL, 1989, VOL. 24, NO. 3, PAGE(S) 224-227., XP002041357

## Beschreibung

Die Erfindung betrifft die in den Patentansprüchen gekennzeichneten Gegegstände, d. h. neue, monomere, perfluoralkylsubstituierte, paramagnetische Metallkomplexe und Komplexsalze, pharmazeutische Mittel enthaltend diese Metallkomplexe, Verfahren zu deren Herstellung, und ihre Verwendung als Kontrastmittel in der ¹H-NMR-Diagnostik und -Spektroskopie, Röntgendiagnostik, Radiodiagnostik sowie als Radiotherapeutika.

Die kernmagnetische Resonanz (NMR) ist heute eine breit angewendete , für die in vivo-Bildgebung ausgenutzte Methode der medizinischen Diagnose, mit der über die Messung der magnetischen Eigenschaften der Protonen im Körperwasser Körpergefäße und Körpergewebe (einschließlich Tumore) dargestellt werden können. Es werden hierzu z. B. Kontrastmittel eingesetzt, die durch Beeinflussung bestimmter NMR-Parameter der Körperprotonen (z. B. der Relaxationszeiten T¹ und T²) eine Kontrastverstärkung in den resultierenden Bildern bewirken bzw. diese Bilder erst lesbar machen. Vor allem kommen Komplexe paramagnetischer Ionen, wie z. B. Gadolinium-enthaltende Komplexe (z. B. Magnevist®) aufgrund des Effektes der paramagnetischen Ionen auf die Verkürzung der Relaxationszeiten zur Anwendung. Ein Maß für die Verkürzung der Relaxationszeit ist die Relaxivity, die in mM⁻¹ · sec ⁻¹angegebenen wird.

Paramagnetische Ionen, wie z. B. Gd³⁺, Mn²⁺, Cr³⁺, Fe³⁺ und Cu²⁺ könnnen nicht in freier Form als Lösungen verabreicht werden, da sie hoch toxisch sind. Um diese Ionen für eine in vivo-Anwendung geeignet zu machen, werden sie in der Regel komplexiert, was erstmalig in EP 0 071 564 A1 beschrieben wird (Komplexierung mit Aminopolycarbonsäuren, z. B. mit Diethylentriamin-pentaessigsäure [DTPA]). Das Di-N-methylglucaminsalz des Gd-DTPA-Komplexes ist unter dem Namen Magnevist® bekannt und wird u.a. zur Diagnose von Tumoren im menschlichen Gehirn und in der Niere angewandt.

Das in der französischen Patentschrift 25 39 996 beschriebene Megluminsalz der Gd-DOTA (Gadolinium-III-Komplex des 1,4,7,10-Tetracarboxymethyl-1,4,7,10- tetraazacyclododecans) ist ein weiteres Kontrastmittel, das sich in der Kernspintomographie sehr gut bewährt hat und unter dem Namen Dotarem® registriert wurde.

Diese Kontrastmittel sind jedoch nicht für alle Anwendungsfälle befriedigend einzusetzen. So verteilen sich die zur Zeit klinisch eingesetzten Kontrastmittel für die modernen bildgebenden Verfahren Kernspintomographie (MRI) und Computertomographie (CT) wie z.B. Magnevist® , Pro Hance® , Ultravist® und Omniscan ® im gesamten extrazellulären Raum des Körpers (im Intravasalraum und im Interstitium).

Besonders zur Darstellung von Gefäßen sind jedoch Konstrastmittel wünschenswert, die sich bei Applikation in den vasalen Raum (Gefäßraum) auch ausschließlich in diesem verteilen und ihn damit markieren (sog. blood-pool-agents).

Es wurde versucht, diese Probleme durch Verwendung von Komplexbildnern, die an Makro- oder Biomoleküle gebunden sind, zu lösen. Damit war man bisher nur sehr begrenzt erfolgreich.

So ist beispielsweise die Anzahl paramagnetischer Zentren in den Komplexen, die in den EP 0 088 695 A1 und EP 0 150 844 A1 beschrieben sind, für eine zufriedenstellende Bildgebung nicht ausreichend.

Erhöht man die Anzahl der benötigten Metallionen durch mehrfache Einführung komplexierender Einheiten in ein makromolekulares Biomolekül, so ist das mit einer nicht tolerierbaren Beeinträchtigung der Affinität und/oder Spezifizität dieses Biomoleküls verbunden [J. Nucl. Med. 24, 1158 (1983)].

Makromolekulare Kontrastmittel für die Angiographie, wie Albumin-Gd-DTPA, werden in Radiology 1987; 162: 205 beschrieben. Albumin-Gd-DTPA zeigt jedoch 24 Stunden nach intravenöser Injektion bei der Ratte eine Anreicherung im Lebergewebe, die fast 30 % der Dosis ausmacht. Außerdem werden in 24 Stunden nur 20 % der Dosis eliminiert.

Das Makromolekül Polylysin-Gd-DTPA (EP 0 233 619 A1) kann ebenfalls als blood-pool-agent eingesetzt werden. Diese Verbindung besteht jedoch herstellungsbedingt aus einem Gemisch von Molekülen verschiedener Größe. Bei Ausscheidungsversuchen bei der Ratte konnte gezeigt werden, daß dieses Makromolekül unverändert durch glomeruläre Filtration über die Niere ausgeschieden wird. Synthese-bedingt enthält Polylysin-Gd-DTPA aber auch Makromoleküle, die so groß sind, daß sie bei der glomerulären Filtration die Kapillaren der Niere nicht passieren können und somit im Körper zurückbleiben.

Auch makromolekulare Kontrastmittel auf der Basis von Kohlenhydraten, z.B. Dextran, sind beschrieben worden (EP 0 326 226 A1). Der Nachteil dieser Verbindungen liegt darin, daß diese in der Regel nur ca. 5 % des signalverstärkenden paramagnetischen Kations tragen.

Aufgabe der Erfindung war es daher, neue ¹H-NMR-Kontrastmittel zur Verfügung zu stellen, die die genannten Nachteile nicht aufweisen und insbesondere eine höhere Protonen-Relaxivity zeigen und damit bei einer Steigerung der Signalintensität eine Reduzierung der Dosis erlauben. Ferner sollen die Kontrastmittel stabil sein, gut verträglich und vor allem organspezifische Eigenschaften aufweisen, wobei einerseits ihre Retention in den zu untersuchenden Organen ausreichend sein soll, um bei geringer Dosierung die für eine zweifelsfreie Diagnose notwendige Anzahl an Bildern zu erhalten, andererseits aber anschließend eine möglichst schnelle und weitestgehend vollständige Ausscheidung der Metalle aus dem Körper gewährleistet sein soll.

Die Aufgabe der Erfindung wird mit den monomeren, perfluoralkylhaltigen Verbindungen der allgemeinen Formel I gemäß Anspruch 1 gelöst, die eine überraschend hohe Protonen-Relaxivity von 20 - 50 [mM⁻¹ · sec⁻¹, 39 °C, 0,47 T ] zeigen. Im Vergleich dazu liegt die Protonen-Relaxivity für die im Handel befindlichen ¹H-NMR-Kontrastmittel Magnevist® , Dotarem® , Omniscan® und Pro Hance® bei Werten zwischen 3,5 - 4,9 [mM⁻¹ · sec⁻¹, 39 °C, 0,47 T].

Daneben sind die erfindungsgemäßen Verbindungen in hervorragender Weise zur Erkennung und Lokalisierung von Gefäßkrankheiten geeignet, da sie sich bei Applikation in den Intravasalraum auch ausschließlich in diesem verteilen. Die erfindungsgemäßen Verbindungen ermöglichen es, mit Hilfe der Kernspintomographie gut durchblutetes von schlecht durchblutetem Gewebe abzugrenzen und somit eine Ischämie zu diagnostizieren. Auch infarziertes Gewebe läßt sich aufgrund seiner Anämie vom umliegenden gesunden oder ischämischen Gewebe abgrenzen, wenn die erfindungsgemäßen Kontrastmittel angewandt werden. Dies ist von besonderer Bedeutung, wenn es z.B. darum geht, einen Herzinfarkt von einer Ischämie zu unterscheiden.

Gegenüber den bisher als blood-pool-agents eingesetzten makromolekularen Verbindungen, wie beispielsweise Gd-DTPA-Polylysin, zeigen die erfindungsgemäßen Verbindungen ebenfalls eine höhere T¹-Relaxivity (s. Tab. 3) und zeichnen sich somit durch eine hohe Steigerung der Signalintensität bei der NMR-Bildgebung aus. Da sie daneben eine verlängerte Retention im Blutraum haben, können sie auch in relativ kleinen Dosierungen (von z. B. ≤ 50 mmol Gd/kg Körpergewicht) appliziert werden. Vor allem aber werden die erfindungsgemäßen Verbindungen, die keine polymeren Verbindungen sind, rasch und weitestgehend vollständig aus dem Körper eliminiert.

Ferner zeigte sich, daß die Verbindungen der vorliegenden Erfindung nicht nur als blood-pool-agents geeignet sind, sondern auch als lymphspezifische MRT-Kontrastmittel (Lymphographika) in hervorragender Weise eingesetzt werden können.

Die Darstellung von Lymphknoten ist von zentraler Bedeutung für die frühe Erkennung des metastatischen Befalls bei Krebspatienten. Die erfindungsgemäßen Kontrastmittel erlauben es, kleine Metastasen in nicht-vergrößerten Lymphknoten (<2 cm) von Lymphknotenhyperplasien ohne malignen Befall zu unterscheiden.

Dabei können die Kontrastmittel intravasal oder interstitiell/intrakutan appliziert werden. Die Applikation interstitiell/intrakutan hat den Vorteil, daß die Substanz direkt vom streuenden Herd (z.B. Primärtumor) durch die entsprechenden Lymphwege in die potentiell betroffenen, regionalen Lymphknotenstationen transportiert wird. Gleichfalls kann mit einer geringen Dosis eine hohe Konzentration des Kontrastmittels in den Lymphknoten erreicht werden.

Die erfindungsgemäßen Verbindungen erfüllen alle Voraussetzungen, die von Kontrastmitteln in der indirekten MRT-Lymphographie verlangt werden: gute lokale Verträglichkeit, rasche Elimination vom Injektionsort, rasche und weitestgehend vollständige Ausscheidung aus dem Gesamtorganismus. Ferner zeigen sie eine hohe Anreicherung über mehrere Lymphknotenstationen und erlauben damit relevante diagnostische Aussagen. So konnte am Meerschweinchenmodell eine hohe Anreicherung über mehrere Lymphknotenstationen (popliteal, inguinal, iliakal) nach s.c. Gabe (2,5 - 10 µmol/kg Körpergewicht, Injektion in Zwischenzehenräume der Hinterpfote) gezeigt werden. In besonders geeigneten Fällen wurden so in der zweiten (inguinal) und dritten (iliakal) Station noch Gadoliniumkonzentrationen von jeweils ≥ 200 bzw. ≥ 300 µmol/l erhalten. Üblicherweise sind mit den erfindungsgemäßen Verbindungen Lymphknotenkonzentrationen im Bereich von 100 bis 1000 µmol/l zu erhalten.

In MR-Bildgebungsstudien an Meerschweinchen konnte die besondere Eignung der erfindungsgemäßen Verbindungen bestätigt werden. So wurde 120 Minuten nach subkutaner Applikation von 10 µmol/kg Körpergewicht eines perfluorhaltigen Gadolinium-Komplexes (Meerschweinchen, Hinterpfote, Zwischenzehenraum) in T¹-gewichteten Spin-Echo-Aufnahmen (TR 400 ms, TE 15 ms) ein deutliches Enhancement der poplitealen Lymphknoten (270 %) sowie der inguinalen (104 %) beobachtet (vgl. Bild 1).

Beim Menschen können die erfindungsgemäßen Verbindungen lokal (entweder subkutan oder direkt perkutan in das interessierende Gewebe) injiziert werden. Es sind mehrere Injektionsorte (Quaddeln) mit einem jeweiligen Injektionsvolumen von 0,2 bis 1 ml gruppiert um den interessierenden Bereich herum (z.B. Tumor) möglich. Das injizierte Gesamtvolumen sollte dabei 5 ml in keinem Fall übersteigen. Das bedeutet, daß in der Formulierung eine Metallkonzentration von 75 - 100 mmol/l vorliegen muß, damit eine potentielle klinische Dosis von 5 - 10 µmol/kg Körpergewicht mit diesem Volumen appliziert werden kann. Der Applikationsort hängt davon ab, ob ein bestimmtes Lymphabflußgebiet aus dem dazu zugeordneten Gewebe spezifisch angefärbt werden soll (z.B. bei gynäkologischen oder Rektumtumoren), oder ob das unbekannte Abflußgebiet einer bestimmten Läsion (ergo der Bereich für eine mögliche therapeutische Intervention z.B. beim Melanom oder Mammakarzinom) dargestellt werden soll.

Für die MR-Bildgebung werden im normalen Lymphknotengewebe, wo die Anreicherung der Verbindung erfolgt, Gadoliniumkonzentrationen von mindestens 50 µmol/l und höchstens 2500 µmol/l benötigt. Die Bildgebung kann (je nach Injektionsort und Gewebe) nach 30 Minuten oder bis zu 4 - 6 Stunden nach Injektion der erfindungsgemäßen Verbindungen erfolgen. Da mit den erfindungsgemäßen Verbindungen von Gadoliniumkomplexen vor allem die T¹-Relaxationszeiten der Protonen des Wassers des Lymphknotengewebes beeinflußt werden, sind T¹-gewichtete Sequenzen am besten in der Lage, ein MRT-Enhancement der Lymphknotenstationen nachzuweisen. Da Lymphknoten sehr häufig in Fettgewebe eingebettet sind, und dieses eine sehr hohe Signalintensität auf solchen Sequenzen besitzt, bieten sich fett-unterdrückte Meßmethoden an. Paramagnetische Gadoliniumkomplexe in Verbindung mit fettunterdrückten, T¹-gewichteten Meßsequenzen haben gegenüber Formulierungen von superparamagnetischen Eisenoxidpartikeln den großen Vorteil, daß sie MRT-Bilder mit höherer räumlicher Auflösung, mit geringeren Distorsionsartefakten (aufgrund von Suszeptibilitätsartefakten) und mit kürzerer Aufnahmezeit erlauben.

Da eine positive Markierung der Lymphknoten erfolgt (d.h. Signalanstieg), sind auch MRT-Aufnahmen ohne Kontrastmittel zum Vergleich nicht mehr zwingend notwendig, und die Gesamt-Untersuchungszeit pro Patient kann verkürzt werden.

Die neuen erfindungsgemäßen perfluoralkylhaltigen Verbindungen der allgemeinen Formel I gemäß Patentanspruch 1 umfassen sowohl Komplexbildner als auch Metallkomplexe. Verbindungen der allgemeinen Formel I mit Z¹ als Wasserstoffatom werden als Komplexbildner bezeichnet und Verbindungen mit mindestens einem der möglichen Substituenten Z¹ als Metallionenäquivalent werden als Metallkomplexe bezeichnet.

Die erfindungsgemäßen Verbindungen der allgemeinen Formel I beinhalten als bevorzugte Reste L die folgenden:

-CH₂-

-CH₂CH₂-

-(CH₂)ₛ- s = 3 - 15

-CH₂-O-CH₂CH₂-

-CH₂-(O-CH₂-CH₂-)ₜ t = 2 - 6

-CH₂-NH-CO-

-CH₂-NH-CO-CH₂-N(CH₂COOH)-SO₂-

-CH₂-NH-CO-CH₂-N(C₂H₅)-SO₂-

-CH₂-NH-CO-CH₂-N(C₁₀H₂₁)-SO₂-

-CH₂-NH-CO-CH₂-N(C₆H₁₃)-SO₂-

-CH₂-NH-CO-(CH₂)₁₀-N(C₂H₅)-SO₂-

-CH₂-NH-CO-CH₂-N(-CH₂-C₆H₅)-SO₂-

-CH₂-NH-CO-CH₂-N(-CH₂-CH₂-OH)SO₂-

-CH₂-NHCO-(CH₂)₁₀-S-CH₂CH₂-

-CH₂NHCOCH₂-O-CH₂CH₂-

-CH₂NHCO(CH₂)₁₀-O-CH₂CH₂-

-CH₂-C₆H₄-O-CH₂CH₂-

-CH₂-O-CH₂-C(CH₂-OCH₂CH₂-C₆F₁₃)₂-CH₂-OCH₂-CH₂-

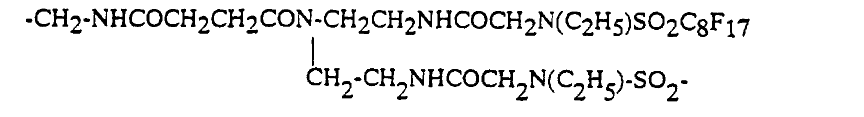

-CH₂-O-CH₂-CH(OC₁₀H₂₁)-CH₂-O-CH₂CH₂-

-(CH₂NHCO)₄-CH₂O-CH₂CH₂-

-(CH₂NHCO)₃-CH₂O-CH₂CH₂-

-CH₂-OCH₂C(CH₂OH)₂-CH₂-O-CH₂CH₂-

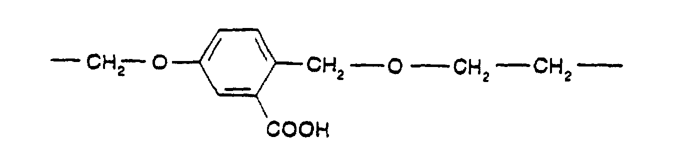

-CH₂NHCOCH₂N(C₆H₅)-SO₂-

-NHCO-CH₂-CH₂-

-NHCO-CH₂-O-CH₂CH₂-

-NH-CO-

-NH-CO-CH₂-N(CH₂COOH)-SO₂-

-NH-CO-CH₂-N(C₂H₅)-SO₂-

-NH-CO-CH₂-N(C₁₀H₂₁)-SO₂-

-NH-CO-CH₂-N(C₆H₁₃)-SO₂-

-NH-CO-(CH₂)₁₀-N(C₂H₅)-SO₂-

-NH-CO-CH₂-N(-CH₂-C₆H₅)-SO₂-

-NH-CO-CH₂-N(-CH₂-CH₂-OH)SO₂-

-NH-CO-CH₂-

-CH₂-O-C₆H₄-O-CH₂-CH₂-

-CH₂-C₆H₄-O-CH₂-CH₂-

-N(C₂H₅)-SO₂-

-N(C₆H₅)-SO₂-

-N(C₁₀H₂₁)-SO₂-

-N(C₆H₁₃)-SO₂-

-N(C₂H₄OH)-SO₂-

-N(CH₂COOH)-SO₂-

-N(CH₂C₆H₅)-SO₂-

-N-[CH(CH₂OH)₂]-SO₂-

-N-[CH(CH₂OH)CH(CH₂OH)]-SO₂-

Erfindungsgemäß ganz besonders bevorzugt sind die Reste L der in den Beispielen der vorliegenden Erfindungsbeschreibung genannten Verbindungen.

Weitere bevorzugte Verbindungen sind solche, in denen X aus der Formel -CₙF₂ₙX Fluor bedeutet, und n für die Zahlen 4 bis 15 steht.

Verbindungen der allgemeinen Formel I mit A in der Bedeutung der allgemeinen Formel IX, wobei L mindestens eine -NHCO-Gruppe enthält, kann man aus Verbindungen der allgemeinen Formel 14 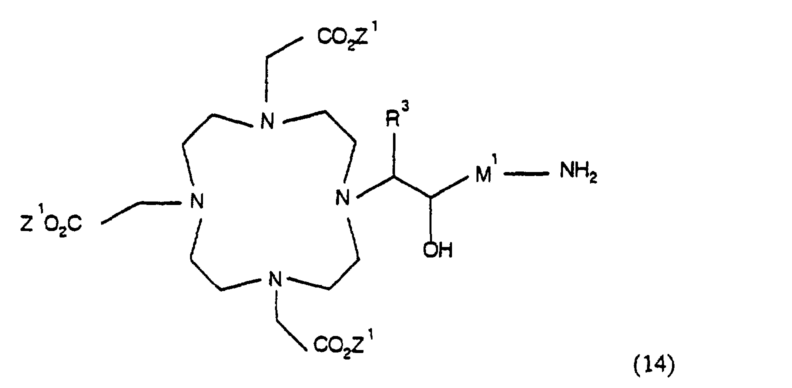 worin
- R³: in der oben genannten Bedeutung steht, Z¹, in der Bedeutung eines Metallionenequivalents der Ordnungszahlen 21-29, 39, 42, 44 oder 57-83, steht und
- M¹: in der Bedeutung von L steht,
durch Umsetzung mit Verbindungen der allgemeinen Formel 15 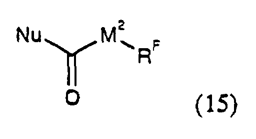 worin
- RF: die oben genannte Bedeutung hat,
- M²: in der Bedeutung von L steht und
- Nu: in der Bedeutung eines Nucleofugs steht, erhalten.

Als Nucleofug dienen vorteilhafterweise die Reste:
Cl, F, ―OTs , ―OMs ,

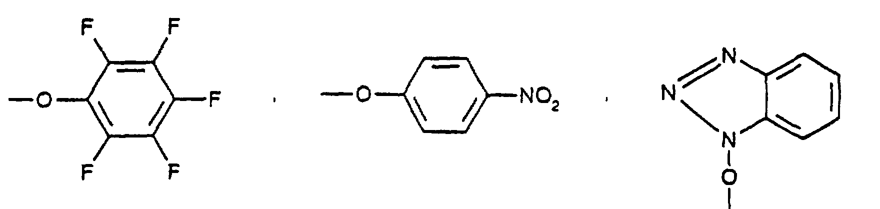

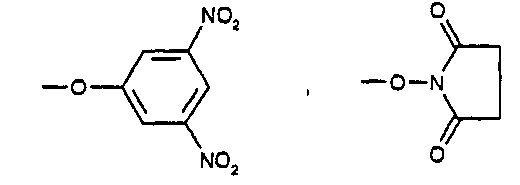

Die Umsetzung wird im Gemisch von Wasser und organischen Lösungsmitteln wie: Isopropanol, Ethanol, Methanol, Butanol, Dioxan, Tetrahydrofuran, Dimethylformamid, Dimethylacetamid, Formamid oder Dichlormethan durchgeführt. Bevorzugt sind ternäre Gemische aus Wasser, Isopropanol und Dichlormethan.

Die Umsetzung wird in einem Temperaturintervall zwischen -10 °C - 100 °C, vorzugsweise zwischen 0 °C - 30 °C durchgeführt.

Als Säurefänger dienen anorganische und organische Basen wie Triethylamin, Pyridin, N-Methylmorpholin, Diisopropylethylamin, Dimethylaminopyridin. Alkali und Endalkalihydroxyde, ihre Carbonate oder Hydrogencarbonate wie Lithiumhydroxid, Natriumhydroxid, Kaliumhydroxid, Natriumcarbonat, Natriumhydrogencarbonat, Kaliumhydrogencarbonat.

Die Verbindungen der allgemeinen Formel 15 werden aus Verbindungen der allgemeinen Formel 16

HO₂C-M²-R^{F} (16)

in der
R^{F}, M² die oben genannte Bedeutung haben, nach den dem Fachmann allgemein bekannten Verfahren der Säureaktivierung wie durch Umsetzung der Säure mit Dicyclohexylcarbodiimid, N-Hydroxysuccinimid/Dicyclohexylcarbodiimid, Carbonyldiimidazol, 2-Ethoxy-1-ethoxycarbonyl-1,2-dihydrochinolin, Oxalsäuredichlorid oder Chlorameisensäurisobutylester nach den in der in der Literatur beschriebenen Verfahren erhalten:
◆ Aktivierung von Carbonsäuren. Übersicht in Houben-Weyl, Methoden der Organischen Chemie, Band XV/2, Georg Thieme Verlag Stuttgart, 19.
◆ Aktivierung mit Carbodiimiden. R. Schwyzer u. H. Kappeler, Helv. 46: 1550 (1963).
◆ E. Wünsch et al., B. 100: 173 (1967).
◆ Aktivierung mit Carbodiimiden/Hydroxysuccinimid: J. Am. Chem. Soc. 86: 1839 (1964) sowie J. Org. Chem. 53: 3583 (1988). Synthesis 453 (1972).
◆ Anhydridmethode, 2-Ethoxy-1-ethoxycarbonyl-1,2-dihydrochinolin: B. Belleau et al., J. Am. Chem. Soc., 90: 1651 (1986), H. Kunz et al., Int. J. Pept. Prot. Res., 26: 493 (1985) und J. R. Voughn, Am. Soc. 73: 3547 (1951).
◆ Imidazolid-Methode: B.F. Gisin, R.B. Menifield, D.C. Tosteon, Am. Soc. 91: 2691 (1969).
◆ Säurechlorid-Methoden, Thionylchlorid: Helv., 42: 1653 (1959).
◆ Oxalylchlorid: J. Org. Chem., 29: 843 (1964).

Die Verbindungen der allgemeinen Formel 16 sind Kaufware (Fluorochem, ABCR) oder werden aus Verbindungen der allgemeinen Formel 17

H-Q-M³-R^{F} (17)

mit
- M3: in der Bedeutung von L und
- Q: in der Bedeutung von Sauerstoff, Schwefel, einer >CO-Gruppe, >N-R³, 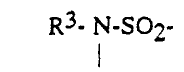 mit einer Bindung vom Stickstoffatom zum Wasserstoffatom,
durch Umsetzen mit Verbindungen der allgemeinen Formel 18 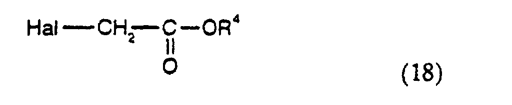 mit
- Hal: in der Bedeutung, Cl, Br, I und
- R⁴: in der Bedeutung von H, Methyl, Ethyl, t-Butyl, Benzyl, Isopropyl, dargestellt beispielsweise nach C.F. Ward, Soc. 121, 1161 (1922), nach den dem Fachmann bekannten Methoden wie Alkylierung von Alkoholen mit Alkylhalogeniden [Houben-Weyl, Methoden der Organischen Chemie, Sauerstoffverbindungen 1, Teil 3, Methoden zur Herstellung und Umwandlung von Ethern, Georg Thieme Verlag, Stuttgart 1965, Alkylierung von Alkoholen mit Alkylhalogeniden S. 24, Alkylierung von Alkoholen mit Alkylsulfaten S. 33] oder N-Alkylierung eines Sulfonamids mit Alkylsulfonaten [Houben-Weyl, Methoden der organischen Chemie, XI/2 Stickstoffverbindungen, Georg Thieme Verlag Stuttgart, 1957, S. 680; J.E. Rickman and T. Atkins, Am. Chem. Soc., 96: 2268, 1974, 96: 2268; F. Chavez and A.D. Sherry, J. Org. Chem. 1989, 54: 2990]
erhalten.

Für den Fall daß Q eine >CO-Gruppe bedeutet, wird die Umsetzung mit einem Wittig-Reagenz der Struktur 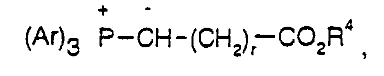 wobei r die Zahlen 0 - 16 bedeutet, vorgenommen.
Die dabei entstandene -CH=CH-Doppelbindung kann als Bestandteil der Struktur erhalten bleiben oder durch katalytische Hydrierung (Pd 5 %/C) in eine -CH₂-CH₂-Gruppierung überführt werden.

Die Verbindungen der allgemeinen Formel 18 sind Kaufware (Fluorochem, ABCR).

Alternativ können Verbindungen der allgemeinen Formel I mit A in der Bedeutung der allgemeinen Formel IX aus Verbindungen der allgemeinen Formel 19 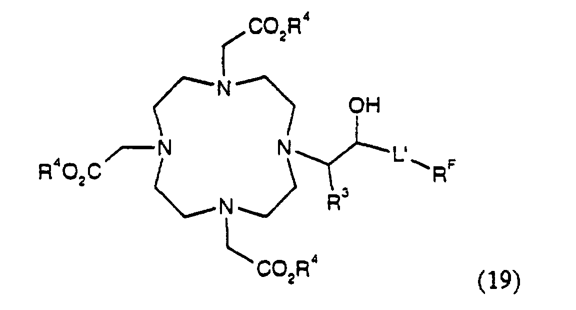 mit
- R^{F}, R³ und R⁴: in der oben genannten Bedeutung und
- L': in der Bedeutung von L, gegebenenfalls mit geschützten Hydroxyl- oder Carboxylfunktionen,
erhalten werden, indem man, falls erforderlich, vorhandene Schutzgruppen abspaltet und die so erhaltenen Komplexbildner mit den nach dem Fachmann bekannten Methoden (EP 250358, EP 255471) mit Metalloxiden oder Metallsalzen bei Raumtemperatur oder erhöhter Temperatur umsetzt, und anschließend - falls gewünscht - vorhandene acide Wasserstoffatome durch Kationen anorganischer und/oder organischer Basen, Aminosäuren oder Aminosäureamide substituiert.

Die Verbindungen der allgemeinen Formel 19 werden erhalten aus Verbindungen der allgemeinen Formel 20 (D03A bzw. der Ester) 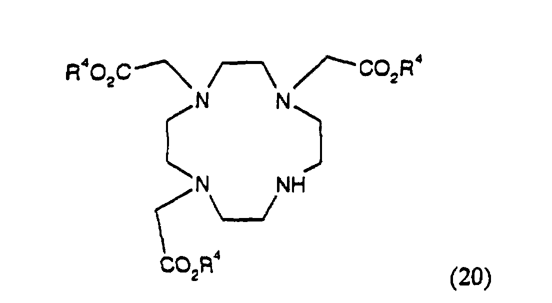 mit
- R⁴: in der oben genannten Bedeutung
durch Umsetzen mit Verbindungen der allgemeinen Formel 21 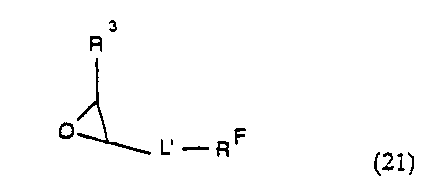 worin
R³ die Bedeutung von R¹, gegebenenfalls in geschützter Form, oder -(CH₂)ₘ-L'-R^{F} hat, wobei m 0, 1 oder 2 ist und L' und R^{F} die oben genannte Bedeutung' haben. Die Umsetzung wird in Alkoholen wie Methanol, Ethanol, Isopropanol, Butanol, Ethern wie Dioxan, Tetrahydrofuran, Dimethoxyethem oder in Wasser oder in Mischungen aus Wasser und einem der genannten organischen Lösungsmittel, sowie auch Acetonitril, Aceton, Dimethylformamid, Dimethylacetamid oder Dimethylsulfoxid, Dichlormethan, Dichlorethan, Chloroform bei Temperaturen zwischen -10 °C und 180 °C, vorzugsweise bei 20 ° - 100 °C durchgeführt. Als vorteilhaft hat sich der Zusatz von organischen oder anorganischen Basen wie Triethylamin, Pyridin, Dimethylaminopyridin, N-Methylmorpholin, Diisopropylamin, Alkali- oder Endalkalihydroxiden oder ihren Carbonaten oder Hydrogencarbonaten wie Lithiumhydroxid, Natriumhydroxid, Kaliumhydroxid, Natriumcarbonat, Kaliumcarbonat, Natriumhydrogencarbonat, Kaliumhydrogencarbonat, erwiesen. Im Falle niedrigsiedender Epoxide wird die Umsetzung im Autoklaven durchgeführt.

Die Verbindungen der allgemeinen Formel 21 sind Kaufware (Fluorochem, ABCR) oder aus Verbindungen der allgemeinen Formel 22

R³-CH=CH-L'-R^{F} (22)

durch Epoxidierung nach den dem Fachmann bekannten Methoden erhältlich, beispielsweise die Wolframat-katalysierte Oxidation mit H₂O₂ nach Payne, die Cyclisierung von Halogenhydrinen oder die alkalische H₂O₂-Oxidation in Gegenwart von Nitrilen.

Besonders geeignet für diese Reaktion ist 3-Chlorperbenzoesäure in Dichlormethan bei Raumtemperatur. Houben-Weyl, Methoden der Organischen Chemie, Sauerstoffverbindungen I, Teil 3, Methoden zur Herstellung und Umwandlung dreigliedriger cyclische Ether (1,2-Epoxide), Georg Thieme Verlag, Stuttgart, 1965; G. B. Payne and P.H. Williams, J. Org. Chem., 159, 24: 54; Y. Ogata and Y. Samaki, Tetrahedron 1964, 20: 2065; K.B. Sharpless et al., Pure Appl. Chem. 55, 589 (1983).
Verbindungen der allgemeinen Formel 22 werden vorzugsweise durch Wittig-Reaktion, bzw. durch die Varianten nach Horner, Schlosser oder Bestmann, Houben-Weyl, Methoden der Organischen Chemie XII/1, Organische Phosphorverbindungen Teil 1, Georg Thieme Verlag, Stuttgart, 1963, Phosphoniumsalze S. 79, Phosphoniumylide S. 112, Wittig-Reaktion S. 121; A.W. Johnson, Ylides and Imines of Phosphorus, John Wiley & Sons, Inc., New York, Chichester, Brisbane, Toronto, Singapore, 1993, Wittig-Reaktion S. 221; Schlosser-Modifikation der Wittig-Reaktion S. 240; Wadsworth-Emmons-Reaktion S. 313; Horner Reaktion S. 362, durch Umsetzung eines Triarylphosphoniums Ylids 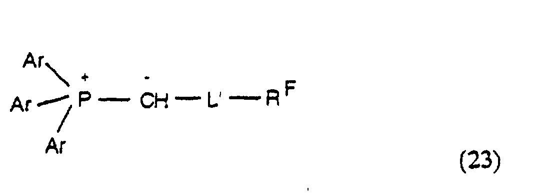 mit L' und R^{F} in der oben genannten Bedeutung und Ar in der Bedeutung Aryl, insbesondere Phenyl, mit käuflichen (Merck, Fluka) oder nach den dem Fachmann bekannten Methoden, beispielsweise der Oxidation von primären Alkoholen mit Chromtrioxid/Pyridin, Houben-Weyl, Methoden der Organischen Chemie, Sauerstoffverbindungen II, Teil 1, Aldehyde, Georg Thieme Verlag, Stuttgart, 1954, darstellbaren Aldehyden der allgemeinen Formel 20

OHC-R³ (24)

wobei
R³ auch H sein kann, erhalten.

Die Triarylphosphoniumylide 23 werden aus den entsprechenden Halogeniden der allgemeinen Formel 25

Hal-CH₂-L'-R^{F} (25)

mit Hal, L' und R^{F} in der oben genannten Bedeutung nach den dem Fachmann bekannten Methoden, beispielsweise durch Erwärmen des Triarylphosphins mit dem Alkylhalogenid, Houben-Weyl, Methoden der Organischen Chemie XII/1, Organische Phosphorverbindungen Teil 1, Georg Thieme Verlag, Stuttgart, 1963, oder A.W. Johnson, Yldes and Imines of Phosphorus, John Wiley & Sons, Inc., New York, Chichester, Brisbane, Toronto, Singapore, 1993, dargestellt. Die Verbindungen der allgemeinen Formel 25 sind Kaufware (Fluorochem, ABCR, 3M).

Die Verbindungen der allgemeinen Formel 21 mit R³ = H werden bevorzugt aus Verbindungen der allgemeinen Formel 17

H-Q'-M³-R^{F} (17)

worin
- Q': in der Bedeutung von Q steht, aber keine >CO-Gruppe bedeuten kann,
- M³: die Bedeutung von L mit Ausnahme der direkten Bindung hat und
- R^{F}: die o. g. Bedeutung hat,
durch Umsetzen nach der dem Fachmann bekannten Weise der Veretherung oder Sulfonamidalkylierung mit Epihalogenhydrinen: (Houben-Weyl, Methoden der Organischen Chemie, Sauerstoffverbindungen I, Teil 3, Methoden zur Herstellung und Umwandlung von Ethern, Georg Thieme Verlag, Stuttgart, 1965, Alkylierung von Alkoholen, S. 24, 33; Houben-Weyl, Methoden der Organischen Chemie, XI/2 Stickstoffverbindungen, Georg Thieme Verlag, Stuttgart, 1957, S. 680; J.E. Rickman and T.J.J. Atkins, Am. Chem. Soc. 1974, 96: 2268; F. Chavez and A.D. Sherry, 1989, 54: 2990) der allgemeinen Formel 26 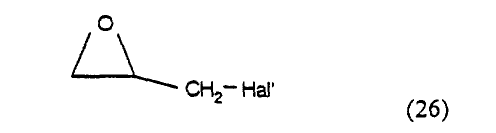 mit
- Hal': in der Bedeutung Hal, F, -OTs, OMs erhalten.

Im Falle niedrigsiedender Epoxide wird die Umsetzung im Autoklaven durchgeführt.

Verbindungen der allgemeinen Formel I mit A in der Bedeutung der allgemeinen Formel VIII erhält man aus Verbindungen der allgemeinen Formel 27 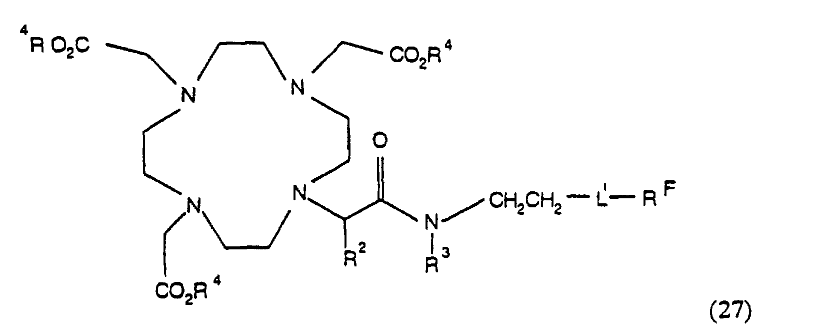 mit R², R³, R⁴, L'und R^{F} in der oben genannten Bedeutung durch Abspalten gegebenenfalls vorhandener Schutzgruppen und Komplexieren in der dem Fachmann bekannten Art.

Verbindungen der allgemeinen Formel 27 erhält man durch Alkylierung der Verbindungen der allgemeinen Formel 20 mit Verbindungen der allgemeinen Formel 28 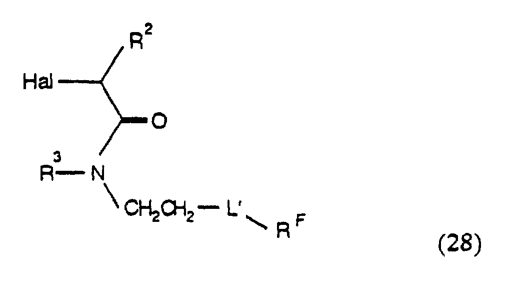 in der Hal, R², R³, L' und R^{F} die o. g. Bedeutung haben,
in an sich bekannter Weise, beispielsweise wie unter EP 0 232 751 B1 (Squibb), beschrieben.

Verbindungen der allgemeinen Formel 28 werden aus Verbindungen der allgemeinen Formel 29 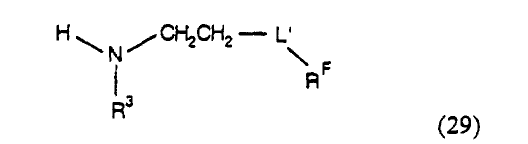 mit L', R³ und R^{F} in der oben genannten Bedeutung und einer aktivierten Halogencarbonsäure der allgemeinen Formel 30 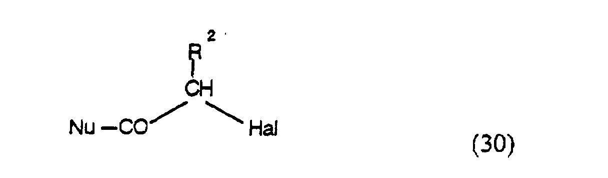 mit Nu, R² und Hal in der oben genannten Bedeutung
nach den dem Fachmann bekannten Methoden der Amidbildung über aktivierte Carbonsäuren [vgl. Lit. S. 11] dargestellt.

Verbindungen der allgemeinen Formel 30 sind aus den Säuren erhältlich nach C. Hell, B. 14: 891 (1881); J. Volhard, A 242, 141 (1887); N. Zelinsky, B. 20: 2026, (1887) oder aus den Halogensäuren nach den Aktivierungsmethoden wie sie bei der allgemeinen Formel 15 beschrieben werden.
Die Verbindungen der allgemeinen Formel 29 lassen sich nach den dem Fachmann bekannten Methoden der Aminsynthese [Houben-Weyl, Methoden der Organischen Chemie, Stickstoffverbindungen II, Amino, 1. Herstellung, Georg Thieme Verlag, Stuttgart, 1957] aus den käuflichen Verbindungen (Fluorochem, ABCR) der allgemeinen Formel 31

Hal-CH₂CH₂-L'-R^{F} (31)

oder 32

HO-CH₂CH₂-L'-R^{F} (32)

leicht darstellen, beispielsweise durch Alkylierung einer Verbindung 31 mit einem Amin PhCH₂NHR³ und anschließender Deprotektion der Aminogruppe durch katalytische Hydrierung oder durch Mitsunobu-Reaktion [H. Loibner und E. Zbiral, Helv. 59, 2100 (1976), A.K. Bose und B. Lal, Tetrahedron Lett. 3973 (1973)] einer Verbindung 32 mit Kaliumphthalimid und Deprotektion mit Hydrazinhydrat.

Verbindungen der allgemeinen Formel I mit A in der Bedeutung der allgemeinen Formel VII erhält man aus Verbindungen der allgemeinen Formel 33 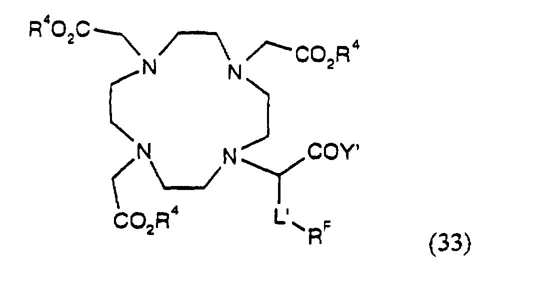 mit
L', R^{F} und R⁴ in der oben genannten Bedeutung und
Y' in der Bedeutung Y, gegebenenfalls mit Schutzgruppen, durch Abspalten gegebenenfalls vorhandener Schutzgruppen und Komplexieren nach den dem Fachmann bekannten Methoden (Protective Groups in Organic Synthesis, 2nd Edition, T.W. Greene and P.G.M. Wuts, John Wiley & Sons, Inc., New York, 1991; EP 0 130 934, EP 0 250 358).

Verbindungen der allgemeinen Formel 33 erhält man aus Verbindungen der allgemeinen Formel 20 und Verbindungen der allgemeinen Formel 34 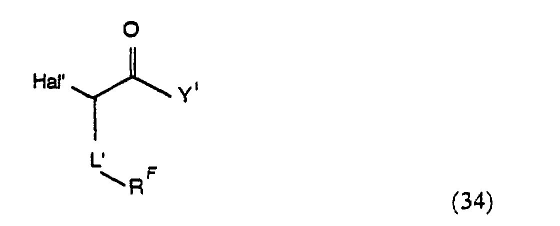 worin
Hal', L', R^{F} die oben genannte Bedeutung haben und Y' für die Reste
―OH, 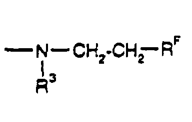 steht, in an sich bekannter Weise, beispielsweise wie in EP 0 232 751 B1, EP 0 292 689 A2 (beide Squibb) oder EP 0 255 471 A1 (Schering) beschrieben.

Die Darstellung von Verbindungen der allgemeinen Formel 34 erfolgt nach bekannten Methoden, beispielsweise nach Hell-Volhard-Zelinsky aus käuflichen Vorstufen (ABCR).

Verbindungen der allgemeinen Formel I mit A in der Bedeutung der allgemeinen Formel VI erhält man aus Verbindungen der allgemeinen Formel 35 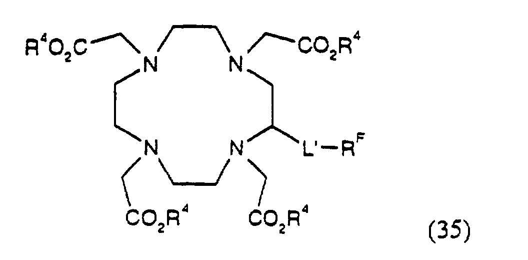 worin L', R⁴ und R^{F} die oben genannte Bedeutung haben durch gegebenenfalls Abspalten von Schutzgruppen und Komplexieren in an sich bekannter Weise [Protective Groups in Organic Synthesis, 2nd Edition, T.W. Greene and P.G.M. Wuts, John Wiley & Sons, Inc., New York, 1991 (EP 0 130 934, EP 0 250 358)].

Verbindungen der allgemeinen Formel 35 erhält man durch Umsetzen von α-Halogencarbonsäureestern oder -säuren der allgemeinen Formel 18 mit Verbindungen der allgemeinen Formel 36 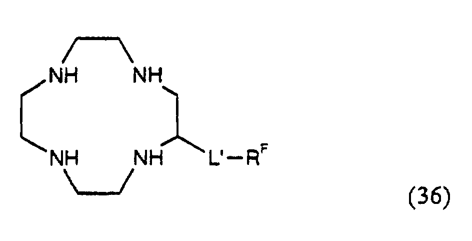 mit L' und R^{F} in der oben genannten Bedeutung, nach den dem Fachmann bekannten Methoden, wie beispielsweise in EP 0 255 471 oder US 4,885,363 beschrieben.

Verbindungen der allgemeinen Formel 36 sind durch Abspalten gegebenenfalls vorhandener Schutzgruppen und anschließender Reduktion mit Diboran nach den bekannten Verfahren aus Verbindungen der allgemeinen Formel 37 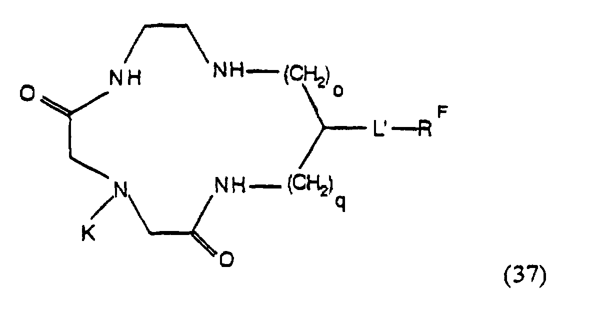 worin
L', R^{F}, o, q, die oben genannte Bedeutung haben und
K die Bedeutung einer Schutzgruppe hat, erhältlich.

Die Verbindungen der allgemeinen Formel 37 sind durch eine Kondensationsreaktion aus einer aktivierten, N-geschützten Iminodiessigsäure 38 und dem Amin 39 zugänglich: 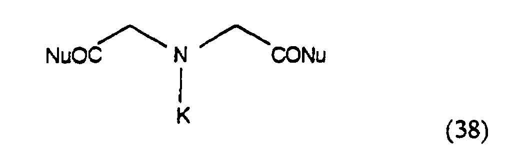 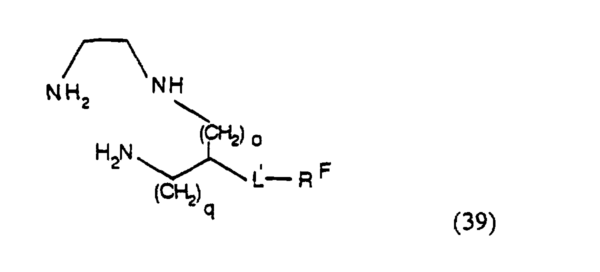 worin
L', R^{F}, o, q, Nu und K die oben genannte Bedeutung haben. Als Nucleofug dient bevorzugt das N-Hydroxysuccinimid, als Schutzgruppe die Benzyloxycarbonyl-, Trifluoracety oder t-Butyloxycarbonylgruppe.

Verbindungen der allgemeinen Formel 38 sind nach den dem Fachmann bekannten Verfahren des Schutzes der Aminogruppe und der Aktivierung der Carbonsäure [Protective Groups, Aktivierung von Carboxylgruppen, S. 11] über die geschützte Iminodiessigsäure 40 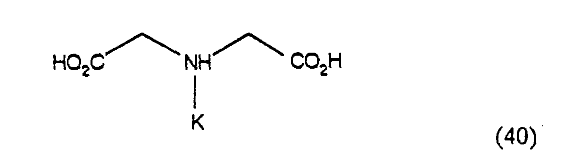 worin
K die Bedeutung einer Schutzgruppe hat aus Iminodiessigsäure 41 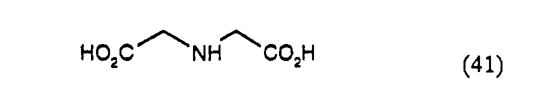 erhältlich.

Alternativ sind Verbindungen der allgemeinen Formel 36 durch gegebenenfalls Abspalten von Schutzgruppen und Reduktion mit Diboran nach dem bei 37 beschriebenen Verfahren aus Verbindungen der allgemeinen Formel 42 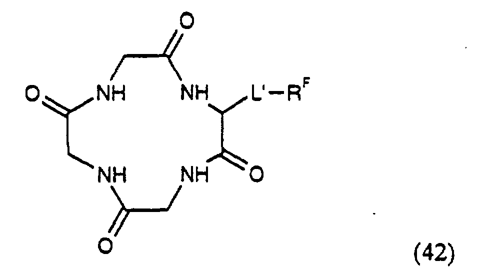 zugänglich.

Verbindungen der allgemeinen Formel 42 sind durch Ringschluß der Secc-Verbindungen 43 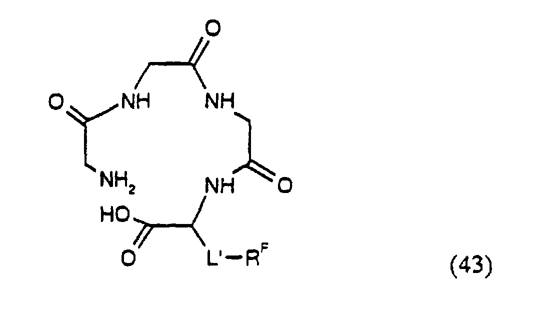 worin
L' und R^{F} die oben genannte Bedeutung haben, nach Standardverfahren erhältlich; beispielsweise durch Umsetzen mit dem Mukaiyama-Reagenz 2-Fluor-1-methylpyridinium-tosylat 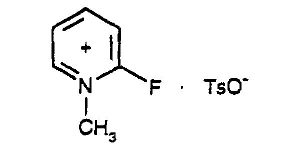 [J. Org. Chem. 1994, 59, 415; Synthetic Commununications 1995, 25, 1401] oder mit dem Phosphorsäurediphenylester-azid 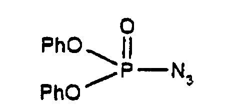 [J. Am. Chem. Soc. 1993, 115, 3420; WO 94/15925].

Verbindungen der allgemeinen Formel 43 sind durch Kondensation der aktivierten Säure 44 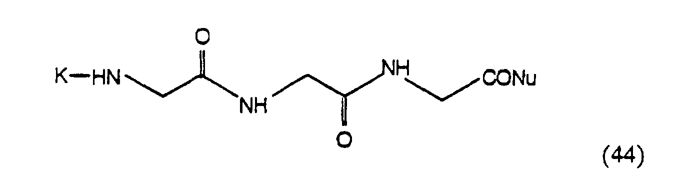 mit Nu und K in der oben genannten Bedeutung mit einer Verbindungen der allgemeinen Formel 45 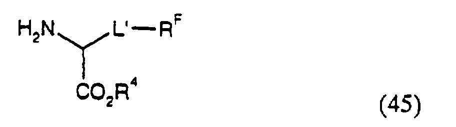 worin
L', R⁴ und R^{F} die oben genannte Bedeutung haben,
nach den beschriebenen Verfahren zugänglich.

Verbindungen der allgemeinen Formel 44 sind aus dem käuflichen Triglycin (Bachem, Fluka) 46 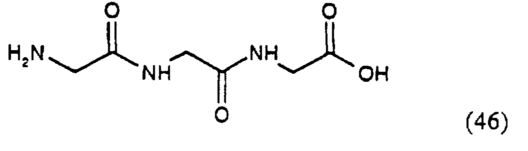 durch Schutz der Aminogruppe mit nachfolgender Aktivierung der Säurefunktion nach den dem Fachmann bekannten Verfahren für Aminschutz und Carbonsäureaktivierung (Lit. S. 12) zugänglich.

Die Verbindungen der allgemeinen Formel 45 sind aus Verbindungen der allgemeinen Formel 62 durch Einführen der Schutzgruppe R⁴ nach den dem Fachmann bekannten Methoden - beispielsweise Umesterung eines Sulfitesters - leicht erhältlich.

Verbindungen der allgemeinen Formel I mit A in der Bedeutung der allgemeinen Formel II erhält man aus Verbindungen der allgemeinen Formel 47 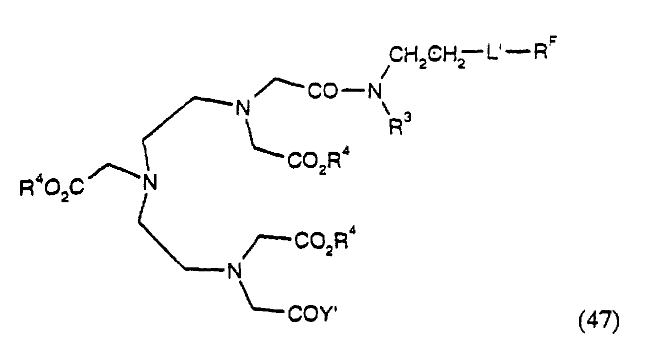 mit L', R³, R⁴, R^{F} und Y' in der oben genannten Bedeutung, durch gegebenenfalls Abspalten von Schutzgruppen und Komplexieren in einer dem Fachmann wohlbekannten Weise (Protective Groups, EP 0 250 358, EP 0 130 934),

Für den Fall, daß Y' in der allgemeinen Formel 47 eine OH-Gruppe bedeutet, erhält man die Verbindungen durch Umsetzen einer Verbindung 48 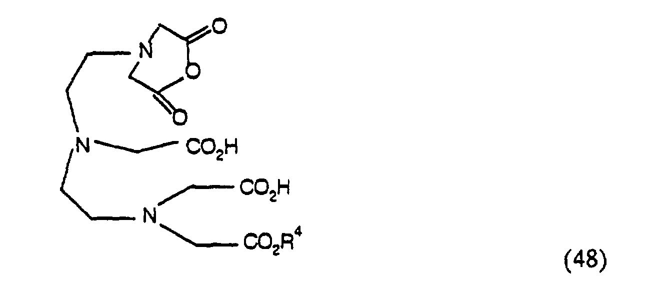 mit R⁴ in der oben genannten Bedeutung, dargestellt nach DE 3 633 243, mit einem Amin der allgemeinen Formel 29 unter den bereits beschriebenen Bedingungen und anschließender Abspaltung der Schutzgruppen.

Ist Y' in Formel 47 jedoch die Gruppe 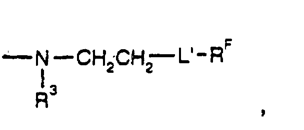 dann wird die Umsetzung mit dem DTPA-Bisanhydrid (Kaufware, Merck) 49 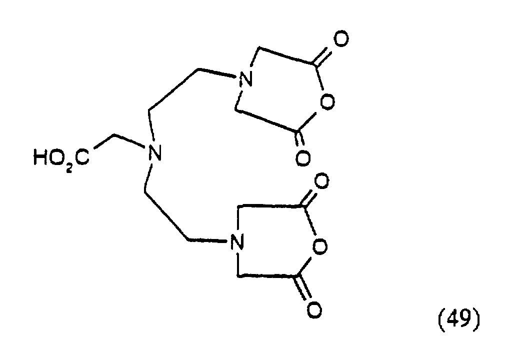 unter analogen Bedingungen vorgenommen.

Verbindungen der allgemeinen Formel I, mit A in der Bedeutung der allgemeinen Formel III erhält man aus Verbindungen der allgemeinen Formel 50 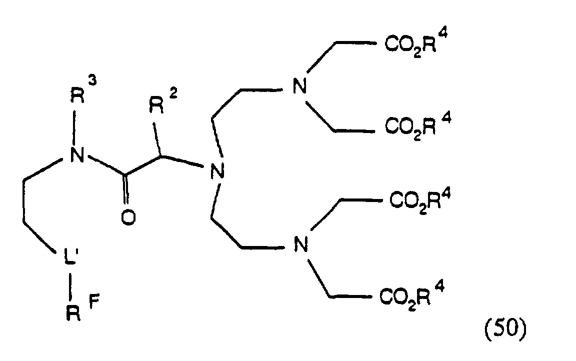 worin
L', R², R³, R⁴ und R^{F} die oben genannte Bedeutung haben, durch gegebenenfalls Abspalten von Schutzgruppen und Komplexieren in einer dem Fachmann wohlbekannten Weise [Protective Groups, EP 0 071564, EP 0 130 934, DE-OS 3 401 052].

Verbindungen der allgemeinen Formel 50 werden nach dem in J. Org. Chem. 1993, 58: 1151, beschriebenen Verfahren aus Verbindungen der allgemeinen Formel 51 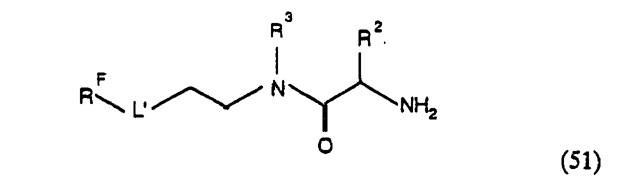 und Halogencarbonsäurederivaten der Formel 52 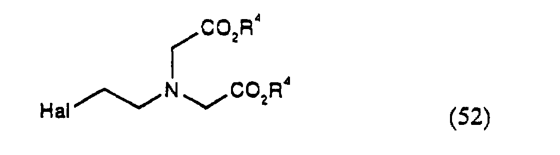 in der R⁴ und Hal die bereits beschriebene Bedeutung haben, erhalten. Die Verbindungen der allgemeinen Formel 51 sind durch Acylierung eines Amins der allgemeinen Formel 29 mit einer aktivierten N-geschützten Aminosäure der allgemeinen Formel 53 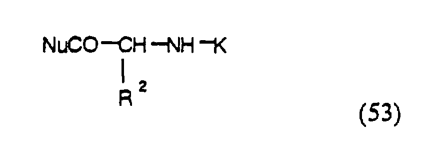 in der Nu die oben genannte Bedeutung hat und K in der Bedeutung einer Schutzgruppe wie Z, -BOC, FMOC, -COCF₃ steht, und anschließender Abspaltung der Schutzgruppe, dargestellt.

Verbindungen der allgemeinen Formel I mit A in der Bedeutung der allgemeinen Formel IV erhält man aus Verbindungen der allgemeinen Formel 54 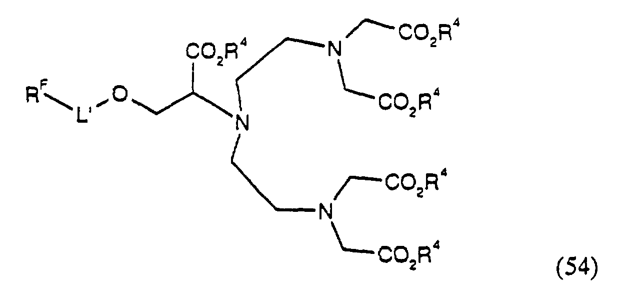 worin
L', R^{F} und R⁴ die oben genannte Bedeutung haben durch gegebenenfalls Abspalten der Schutzgruppen und Komplexieren nach einer dem Fachmann bekannten Methode, wie sie bereits beschrieben [Protective Groups, EP 0 071 564, EP 0 130 934, DE-OS 3 401 052].

Verbindungen der allgemeinen Formel 54 lassen sich in bekannter Weise aus den Halogenverbindungen der allgemeinen Formel 55

Hal-L'-R^{F} (55)

die als Kaufware erhältlich sind (Fluorochem, ABCR), durch Umsetzen mit den Hydroxysäuren 56 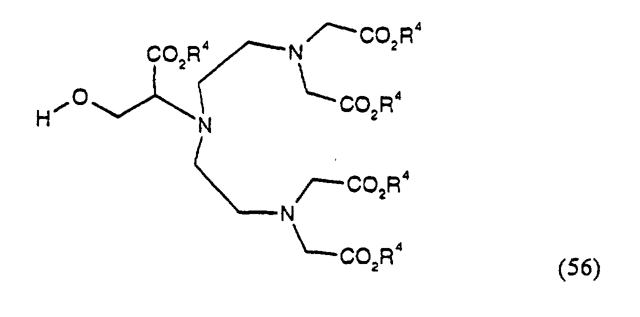 worin
R⁴ die oben genannte Bedeutung hat, erhalten. Die Verbindungen der Formel 56 sind in an sich bekannter Weise nach J. Org. Chem. 58, 1151 (1993), aus dem käuflichen Serinester 57 (Bachem, Fluka) 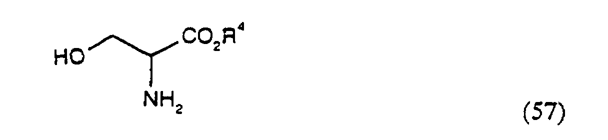 mit R⁴ in der oben genannten Bedeutung und den Halogencarbonsäureestern 58 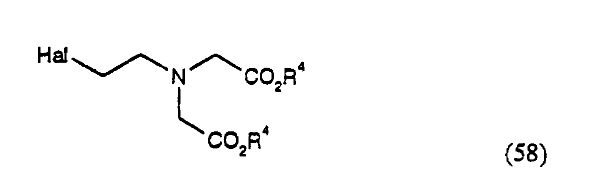 erhältlich.

Verbindungen der allgemeinen Formel I mit A in der Bedeutung der allgemeinen Formel V erhält man aus Verbindungen der allgemeinen Formel 59 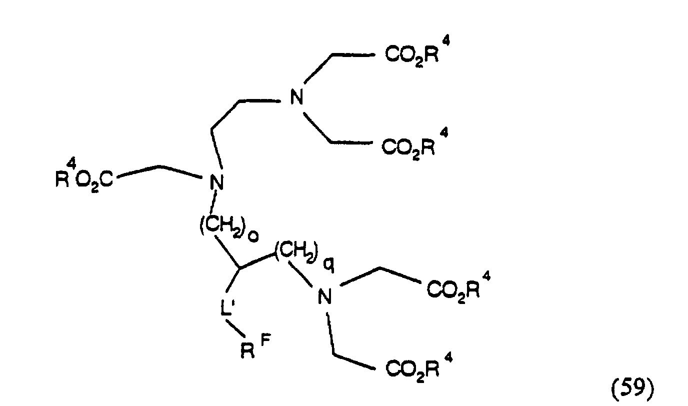 worin
L', o, q, R⁴ und R^{F} die oben genannte Bedeutung haben, durch gegebenenfalls Abspalten von Schutzgruppen und Komplexieren nach einer dem Fachmann bekannten Methode. [Protective Groups, EP 0 071 564, EP 0 130 934, DE-OS 3 401 052].

Verbindungen der allgemeinen Formel 59 lassen sich in bekannter Weise, beispielsweise nach J. Org. Chem., 58, 1151 (1993) durch Umsetzen von Halogencarbonsäureestern 18

Hal-CH₂CO₂R⁴ (18)

mit Hal und R⁴ in der oben genannten Bedeutung, und einer Verbindung der allgemeinen Formel 39 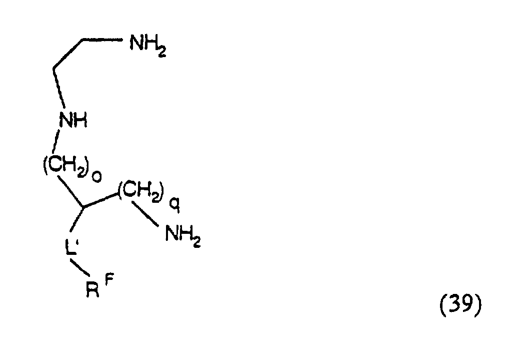 worin
L', o, q, und R^{F} die oben genannte Bedeutung haben, darstellen.

Die Verbindungen der allgemeinen Formel 39 werden für den Fall q = 0 aus den Verbindungen der allgemeinen Formel 60 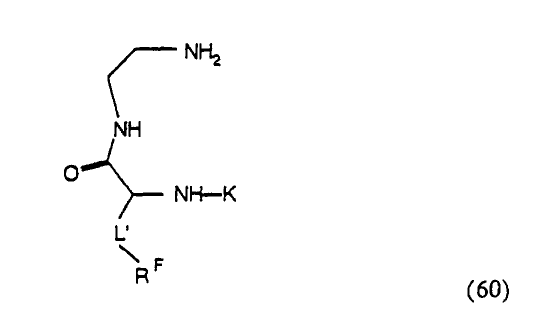 mit
L', R^{F} und K in der oben genannten Bedeutung in an sich bekannter Weise [Helv. Chim. Acta, 77: 23 (1994)] durch Reduktion mit Diboran und Abspaltung der Schutzgruppen gewonnen. Die Verbindungen der allgemeinen Formel 60 werden durch Aminolyse der aktivierten Verbindungen der allgemeinen Formel 61 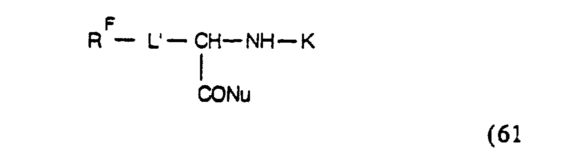 worin
L', Nu, R^{F} und K die oben genannte Bedeutung haben
mit Ethylendiamin erhalten.

Die Verbindungen der allgemeinen Formel 61 werden nach den bekannten Methoden der Schutzgruppenchemie [Protective Groups] aus der ungeschützten Säure der allgemeinen Formel 62 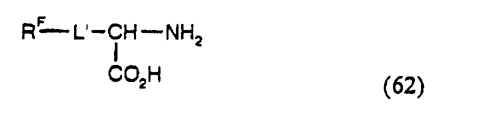 dargestellt, und zwar wird in einem ersten Schritt die Aminogruppe geschützt, gefolgt von der Aktivierung der Säuregruppe im zweiten Schritt.

Die Verbindungen der allgemeinen Formel 62 lassen sich nach dem Methoden der Aminosäuresynthese [Houben-Weyl, Methoden der organischen Chemie, XI/2 Stickstoffverbindungen II und III, II Aminosäuren; Georg Thieme Verlag Stuttgart, 1958, Strecker-Reaktion, S. 305; Erlenmeyer-Reaktion, S. 306; Aminolyse von α-Halogencarbonsäuren, S. 309] aus den käuflichen Aldehyden der allgemeinen Formel 63

HOC―L'―R^{F} (63)

darstellen, beispielsweise nach Strecker, über das Azlacton oder über das Cyanhydrin.

Die Verbindungen der allgemeinen Formel 39 werden für den Fall o = 0 aus den Verbindungen der allgemeinen Formel 64 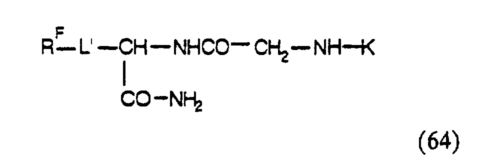 mit R^{F}, L' und K in den genannten Bedeutungen,
in an sich bekannter Weise durch Abspalten der Schutzgruppen und Reduktion mit Diboran gewonnen.

Verbindungen der allgemeinen Formel 64 sind durch Aminolyse der N-geschützten, aktivierten Glycine 53 mit Verbindungen der allgemeinen Formel 65 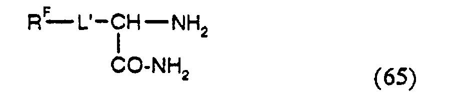 in der R^{F} und L' die genannten Bedeutungen haben,
zugänglich.

Die Verbindungen der allgemeinen Formel 65 sind in einfacher Weise aus Verbindungen der allgemeinen Formel 61 durch Amidbildung mit Ammoniak und anschließender Abspaltung der Schutzgruppe erhältlich.

Verbindungen der allgemeinen Formel XIII können analog den Verbindungen der allgemeinen Formel III hergestellt werden, indem man Halogencarbonsäurederivate der allgemeinen Formel 52 mit einer Verbindung der allgemeinen Formel 66 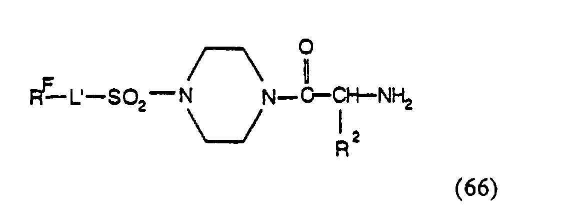 in der R^{F}, L' und R² die o. g. Bedeutungen haben, umsetzt.

Die Verbindungen der allgemeinen Formel 66 werden durch Umsetzung einer Verbindung der allgemeinen Formel 67 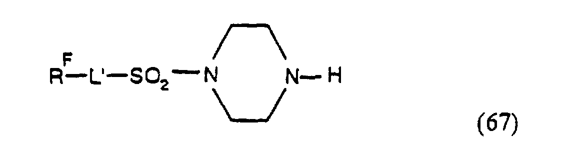 mit der aktivierten, N-geschützten Aminosäure der allgemeinen Formel 53 in Analogie zur Umsetzung des Amins 29 mit der Verbindung 53 hergestellt.

Die Verbindungen der allgemeinen Formel 67 können durch Umsetzung von Piperazin - frei oder gegebenfalls partiell geschützt - mit Perfluoralkylsulfonsäurefluoriden oder -chloriden erhalten werden. (Die Sulfonamidbildung aus Amin und Sulfofluorid ist beschrieben in DOS 2 118 190, DOS 2 153 270, beide Bayer AG).

Verbindungen der allgemeinen Formel XI mit q in der Bedeutung der Ziffern 0 bzw. 1 werden analog zu Verbindungen der allgemeinen Formel VIII hergestellt, indem man Verbindungen der allgemeinen Formel 20 mit Verbindungen der allgemeinen Formel 68 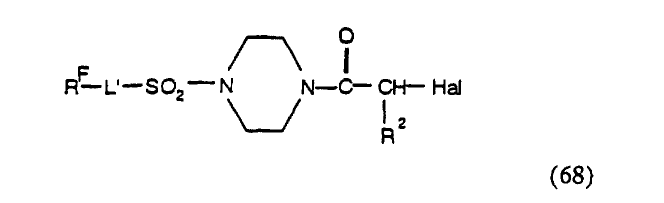 in der R^{F}, L', R² und Hal die o.g. Bedeutung haben,
bzw. mit Verbindungen der allgemeinen Formel 68a 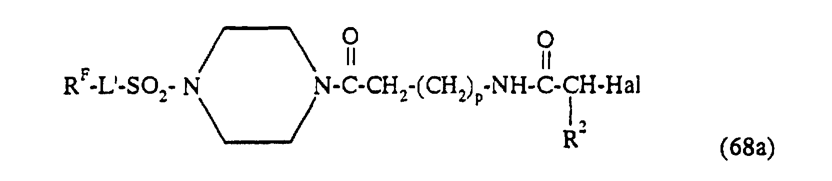 in der R^{F}, L', R², p und Hal die oben genannte Bedeutung haben, umsetzt.

Verbindungen der allgemeinen Formel 68 sind aus Verbindungen der allgemeinen Formel 30 und Piperazinderivaten der allgemeinen Formel 67 in an sich bekannter Weise erhältlich.

Verbindungen der allgemeinen Formel 68a sind aus Verbindungen der allgemeinen Formel 67 durch Amidkupplung mit Verbindungen der allgemeinen Formel 68 b

HOOC-CH₂―(CH₂)ₚ―NH-CO-CHR²-Hal (68b)

erhältlich

Verbindungen der allgemeinen Formel XII werden analog zu Verbindungen der allgemeinen Formel II hergestellt, z.B. durch Umsetzen von Verbindungen der Formel 49 mit Piperazinderivaten der allgemeinen Formel 67.

Verbindungen der allgemeinen Formel I mit A in der Bedeutung der allgemeinen Formel X erhält man aus Verbindungen der allgemeinen Formel 69 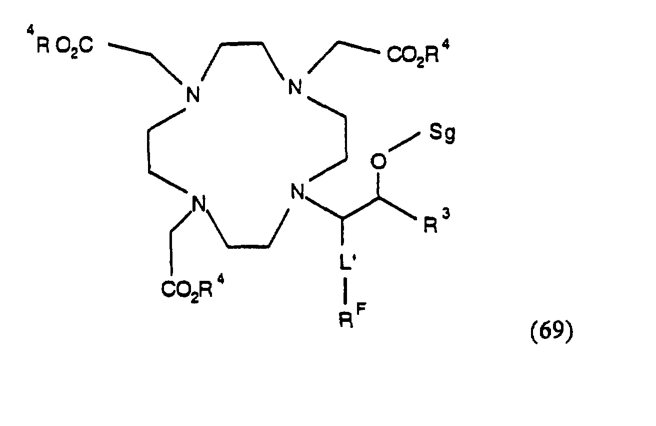 worin
L', R³, R⁴ und R^{F} die oben beschriebene Bedeutung haben und Sg in der Bedeutung einer Schutzgruppe steht,
durch gegebenenfalls Abspalten von Schutzgruppen und Komplexieren in an sich bekannter Weise [Protective Groups in Organic Synthesis, 2nd Edition, T. W. Greene and P. G. M. Wuts, John Wiley & Sons, Inc., New York, 1991 (EP 0 130 934, EP 0 250 358)].

Verbindungen der allgemeinen Formel 69 erhält man durch Umsetzen von a-Halogencarbonsäureestern oder -säuren der allgemeinen Formel 18 mit Verbindungen der allgemeinen Formel 70 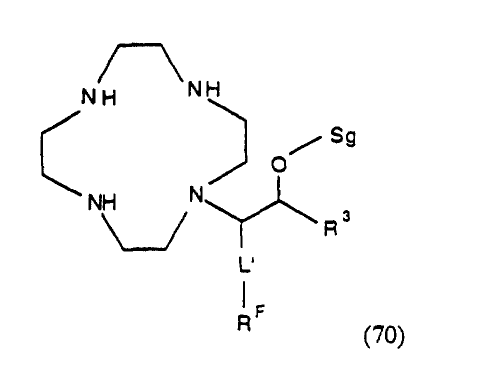 mit L', R^{F,} R³ und Sg in der oben genannten Bedeutung
nach den dem Fachmann bekannten Methoden wie beispielsweise in EP 0 255 471 oder US 4,885,363 beschrieben.

Verbindungen der allgemeinen Formel 70 sind durch Abspalten gegebenenfalls vorhandener Schutzgruppen und anschließender Reduktion mit Diboran nach den bekannten Verfahren aus Verbindungen der allgemeinen Formel 71 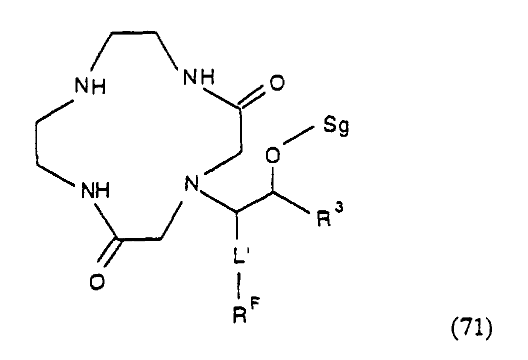 worin
L', R^{F}, R³ und Sg die oben genannte Bedeutung haben, erhältlich.

Die Verbindungen der allgemeinen Formel 71 sind durch eine Kondensationsreaktion aus einem aktivierten Iminodiessigsäurederivat der allgemeinen Formel 72 und dem Diethylentriamin der Formel 73 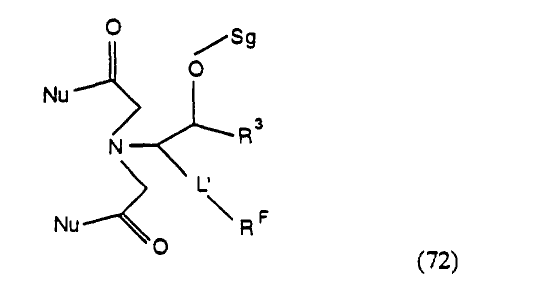 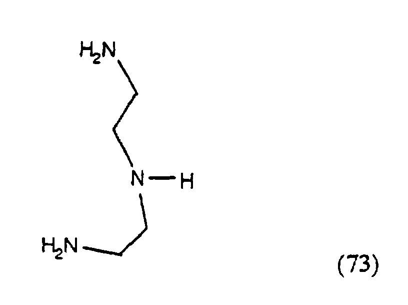 worin
L', R^{F}, R³, Sg und Nu die oben genannte Bedeutung haben, erhältlich.

Als Nucleofug Nu dient bevorzugt das N-Hydroxysuccinimid.

Verbindungen der allgemeinen Formel 72 sind aus Verbindungen der allgemeinen Formel 74 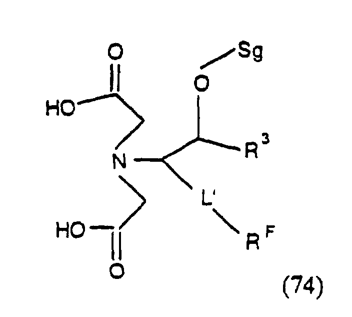 worin
L', R^{F} und Sg die oben genannte Bedeutung haben, durch Aktivierung der Carbonsäuren, wie auf Seite 11 beschrieben, erhältlich.

Verbindungen der allgemeinen Formel 74 erhält man durch Umsetzen von a-Halogencarbonsäureestern oder -säuren der allgemeinen Formel 18 mit Verbindungen der allgemeinen Formel 75 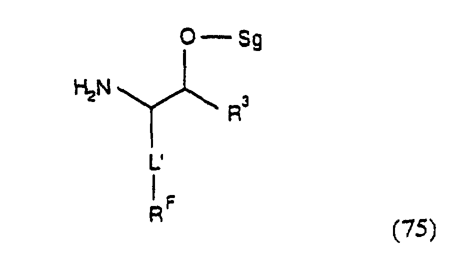 worin
L', R^{F}, R³ , und Sg die oben genannte Bedeutung haben,
wobei gegebenenfalls vorhandene Estergruppen verseift werden.

Verbindungen der allgemeinen Formel 75 erhält man aus Verbindungen der allgemeinen Formel 76 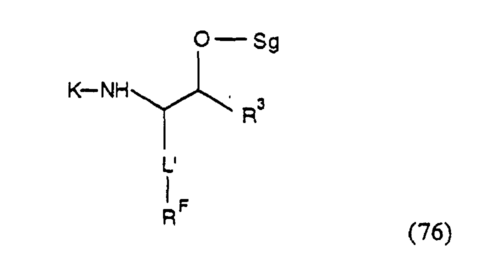 worin
L', R^{F}, R³, Sg und K die oben genannte Bedeutung haben,
durch Abspaltung der Schutzgruppe K nach den bekannten Verfahren.

Verbindungen der allgemeinen Formel 76 erhält man aus Verbindungen der allgemeinen Formel 77 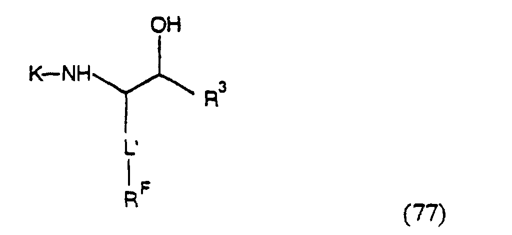 worin
L', R^{F}, R³ und K die oben genannte Bedeutung haben,
durch Einführen einer Schutzgruppe Sg in der dem Fachmann bekannten Weise.

Verbindungen der allgemeinen Formel 77 erhält man aus den Verbindungen der allgemeinen Formel 78 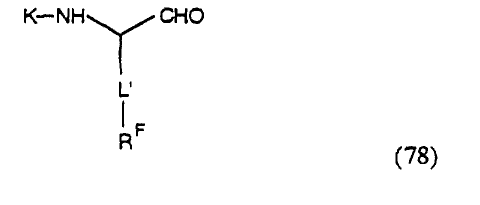 worin
L', R^{F} und K die oben genannten Bedeutung haben,
nach den dem Fachmann wohlbekannten Methoden (Houben-Weyl, Methoden der Organischen Chemie, XIII 2a, Metallorganische Verbindungen, Georg Thieme Verlag Stuttgart, 1973, S. 285 ff, Umsetzung magnesiumorganischer Verbindungen mit Aldehyden; S. 809 ff, Umsetzung von zinkorganischen Verbindungen mit Aldehyden; Houben-Weyl, Methoden der Organischen Chemie XIII/1, Metallorganische Verbindungen, Georg Thieme Verlag Stuttgart, 1970; S. 175 ff, Umsetzung lithiumorganischer Verbindungen mit Aldehyden) durch Umsetzen mit den aus Verbindungen der allgemeinen Formel 79

Hal - R³ (79)

worin
Hal und R³ die oben genannte Bedeutung haben,
erhältlichen metallorganischen Verbindungen, wie Magnesium-, Lithium- oder Zinkverbindungen.

Verbindungen der allgemeinen Formel 79 sind Kaufware (ABCR, Fluka).

Verbindungen der allgemeinen Formel 78 werden aus Verbindungen der allgemeinen Formel 80 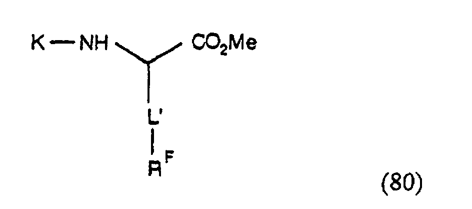 worin
L', R^{F} und K die oben genannte Bedeutung haben,
durch Reduktion mit Diisobutylaluminiumhydrid (Tett. Lett., 1962, 619; Tett. Lett., 1969, 1779; Sythesis, 1975, 617). hergesteilt.

Verbindungen der allgemeinen Formel 80 werden aus Verbindungen der allgemeinen Formel 45 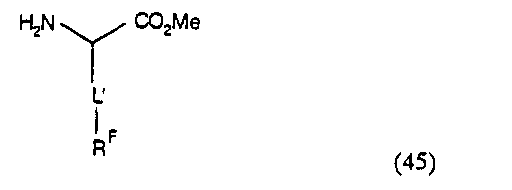 worin
L' und R^{F} die oben genannte Bedeutung haben,
auf eine dem Fachmann bekannte Weise durch Einführen der Schutzgruppe K hergestellt.

Die Neutralisation eventuell noch vorhandener freier Carboxygruppen erfolgt mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl-und N,N-Dimethylglucamin, sowie basischer Aminosäuren, wie zum Beispiel Lysin, Arginin und Ornithin oder von Amiden ursprünglich neutraler oder saurer Aminosäuren.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Gegenstand der Erfindung sind ferner pharmazeutische Mittel, die mindestens eine physiologisch verträgliche Verbindung der allgemeinen Formel I enthalten, ggf. mit den in der Galenik üblichen Zusätzen.

Die Herstellung der erfindungsgemäßen pharmazeutischen Mittel erfolgt in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern oder schwachen Komplexen (wie zum Beispiel Diethylentriaminpentaessigsäure oder die zu den erfindungsgemäßen Metallkomplexen korrespondierenden Ca-Komplexe) oder - falls erforderlich - Elektrolyte wie zum Beispiel Natriumchlorid oder - falls erforderlich - Antioxidantien wie zum Beispiel Ascorbinsäure.

Sind für die enterale bzw. parenterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) [zum Beispiel Methyl-cellulose, Lactose, Mannit] und/oder Tensid(en) [zum Beispiel Lecithine, Tween® , Myrj® ] und/oder Aromastoff(en) zur Geschmackskorrektur [zum Beispiel ätherischen Ölen] gemischt.

Prinzipiell ist es auch möglich, die erfindungsgemäßen pharmazeutischen Mittel ohne Isolierung der Komplexe herzustellen. In jedem Fall muß besondere Sorgfalt darauf verwendet werden, die Chelatbildung so vorzunehmen, daß die erfindungsgemäßen Komplexe praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexes.

Die erfindungsgemäßen pharmazeutischen Mittel enthalten vorzugsweise 0,1µ Mol - 1 Mol/l des Komplexes und werden in der Regel in Mengen von 0,0001 - 5 mMol/kg dosiert. Sie sind zur enteralen und parenteralen Applikation bestimmt. Die erfindungsgemäßen Komplexverbindungen kommen zur Anwendung
1. für die NMR- und Röntgen-Diagnostik in Form ihrer Komplexe mit den Ionen der Elemente mit den Ordnungszahlen 21 - 29, 39, 42, 44 und 57 - 83;
2. für die Radiodiagnostik und Radiotherapie in Form ihrer Komplexe mit den Radioisotopen der Elemente mit den Ordnungszahlen 27, 29, 31, 32, 37 - 39, 43, 49, 62, 64, 70, 75 und 77.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,0001 - 5 mMol/kg, vorzugsweise 0,005 - 0,5 mMol/kg, dosiert. Besonders niedrige Dosierungen (unter 1 mg/kg Körpergewicht) von organspezifischen NMR-Diagnostika sind zum Beispiel zum Nachweis von Tumoren und von Herzinfarkten einsetzbar.

Ferner können die erfindungsgemäßen Komplexverbindungen vorteilhaft als Suszeptibilitäts-Reagenzien und als shift-Reagenzien für die in-vivo-NMR-Spektroskopie verwendet werden.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details einer solchen Anwendung und Dosierung werden z.B. in "Radiotracers for Medical Applications", CRC-Press, Boca Raton, Florida, beschrieben.
Die erfindungsgemäßen Verbindungen und Mittel können auch in der Positronen-Emissions-Tomographie, die positronenemittierende Isotope wie z.B. ⁴³Sc, ⁴⁴Sc, ⁵²Fe, ⁵⁵Co und ⁶⁸Ga verwendet (Heiss, W.D.; Phelps, M.E.; Positron Emission Tomography of Brain, Springer Verlag Berlin, Heidelberg, New York 1983), eingesetzt werden.

Die erfindungsgemäßen Verbindungen sind überraschenderweise auch zur Differenzierung von malignen und benignen Tumoren in Bereichen ohne Blut-Hirn-Schranke geeignet.

Sie zeichnen sich auch dadurch aus, daß sie vollständig aus dem Körper eliminiert werden und somit gut verträglich sind.

Da sich die erfindungsgemäßen Substanzen in malignen Tumoren anreichern (keine Diffusion in gesunde Gewebe, aber hohe Durchlässigkeit von Tumorgefäßen), können sie auch die Strahlentherapie von malignen Tumoren unterstützen. Diese unterscheidet sich von der entsprechenden Diagnostik nur durch die Menge und Art des verwendeten Isotops. Ziel ist dabei die Zerstörung von Tumorzellen durch energiereiche kurzwellige Strahlung mit einer möglichst geringen Reichweite. Hierzu werden Wechselwirkungen der in den Komplexen enthaltenen Metalle (wie z.B. Eisen oder Gadolinium) mit ionisierenden Strahlungen (z.B. Röntgenstrahlen) oder mit Neutronenstrahlen ausgenutzt. Durch diesen Effekt wird die lokale Strahlendosis am Ort, wo sich der Metallkomplex befindet (z.B. in Tumoren) signifikant erhöht. Um die gleiche Strahlendosis im malignen Gewebe zu erzeugen, kann bei Anwendung solcher Metallkomplexe die Strahlenbelastung für gesunde Gewebe erheblich reduziert und damit belastende Nebenwirkungen für die Patienten vermieden werden. Die erfindungsgemäßen Metallkomplex-Konjugate eignen sich deshalb auch als radiosensibilisierende Substanz bei Strahlentherapie von malignen Tumoren (z.B. Ausnutzen von Mössbauer-Effekten oder bei Neutroneneinfangtherapie). Geeignete β-emittierende Ionen sind zum Beispiel ⁴⁶Sc, ⁴⁷Sc, ⁴⁸Sc, ⁷²Ga, ⁷³Ga und ⁹⁰Y. Geeignete geringe Halbwertzeiten aufweisende α-emittierende Ionen sind zum Beispiel ²¹¹Bi, ²¹²Bi, ²¹³Bi und ²¹⁴Bi, wobei ²¹²Bi bevorzugt ist. Ein geeignetes Photonen- und Elektronen-emittierendes Ion ist ¹⁵⁸Gd, das aus ¹⁵⁷Gd durch Neutroneneinfang erhalten werden kann.

Ist das erfindungsgemäße Mittel zur Anwendung in der von R.L. Mills et al. (Nature Vol. 336, (1988), S. 787] vorgeschlagenen Variante der Strahlentherapie bestimmt, so muß sich das Zentralion von einem Mößbauer-Isotop wie beispielsweise ⁵⁷Fe oder ¹⁵¹Eu ableiten.

Bei der in-vivo-Applikation der erfindungsgemäßen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung, dem benutzten Metallion und der Art der bildgebenden Methode.

Die erfindungsgemäßen Mittel werden üblicherweise parenteral, vorzugsweise i.v., appliziert. Sie können auch - wie bereits erörtert - intravasal oder interstitiell/intrakutan appliziert werden, je nachdem, ob Körpergefäße oder - gewebe untersucht werden sollen.

Die erfindungsgemäßen Mittel sind hervorragend als Röntgenkontrastmittel geeignet, wobei besonders hervorzuheben ist, daß sich mit ihnen keine Anzeichen der von den jodhaltigen Kontrastmitteln bekannten anaphylaxieartigen Reaktionen in biochemisch-pharmakologischen Untersuchungen erkennen lassen. Besonders wertvoll sind sie wegen der günstigen Absorptionseigenschaften in Bereichen höherer Röhrenspannungen für digitale Substraktionstechniken.

Im allgemeinen werden die erfindungsgemäßen Mittel für die Anwendung als Röntgenkontrastmittel in Analogie zum Beispiel Meglumin-Diatrizoat in Mengen von 0,1 - 5 mMol/kg, vorzugsweise 0,25 - 1 mMol/kg, dosiert.

Insgesamt ist es gelungen, neue Komplexbildner, Metallkomplexe und Metallkomplexsalze zu synthetisieren, die neue Möglichkeiten in der diagnostischen und therapeutischen Medizin erschließen.

Die nachfolgenden Beispiele dienen zur näheren Erläuterung des Erfindungsgegenstands:

### Beispiel 1

### a) N-Ethyl-N-(perfluoroctylsulfonyl)-amino-essigsäure-t-butylester

20 g (37,94 mmol) N-Ethylperfluorooctylsulfonamid und 15,73 g (113,8 mmol) Kaliumcarbonat werden in 200 ml Aceton suspendiert und bei 60°C 14,80 g (75,87 mmol) Bromessigsaure-tert.-butylester zugetropft. Man rührt 3 Stunden bei 60°C. Man filtriert von den Salzen ab und dampft das Filtrat im Vakuum zur Trockne ein. Der Rückstand wird an Kieselgel chromatographiert (Laufmittel: Hexan/Dichlormethan/Aceton= 10/10/1). Nach Eindampfen der produkthaltigen Fraktionen, kristallisiert man den Rückstand aus Methanol/Ether um.
Ausbeute: 21,66 g (89 % d. Th.) eines wachsartigen farblosen Feststoffes

| Elementaranalyse: | | | | | |
|---|---|---|---|---|---|
| ber. | C 29,96 | H 2,51 | F 50,36 | N 2,18 | S 5,00 |
| gef. | C 29,81 | H 2,70 | F 50,15 | N 2,30 | S 4,83 |

### b) N-Ethyl-N-(perfluoroctylsulfonyl)-amino-essigsäure

20 g (31,18 mmol) der Titelverbindung aus Beispiel 1a) werden in 200 ml Trifluoressigsäure gelöst und über Nacht bei Raumtemperatur gerührt. Man dampft im Vakuum zur Trockne ein. Der Rückstand wird aus Methanol/Ether umkristallisiert.
Ausbeute: 17,34 g (95 % d. Th.) eines farblosen kristallinen Feststoffes

| Elementaranalyse: | | | | | |
|---|---|---|---|---|---|
| ber. | C 24,63 | H 1,38 | F 55,19 | N 2,39 | S 5,48 |
| gef. | C 24,48 | H 1,50 | F 55,01 | N 2,17 | S 5,59 |

### c) Gadolinium-Komplex von 10-[2-Hydroxy-4-aza-5-oxo-7-aza-7-(perfluoroctylsulfonyl)-nonyl]-1,4,7-tris(carboxymethyl)-1,4,7,10-tetraazacyclododecan

10 g (17,09 mmol) der Titelverbindung aus Beispiel 1b) und 1,97 g (18,79 mmol) N-Hydroxysuccinimid werden in einer Mischung aus 50 ml Dimethylformamid/50 ml Chloroform gelöst. Bei 0°C gibt man 3,88 g (18,79 mmol) Dicyclohexylcarbodiimid zu und rührt 1 Stunde bei 0°C, anschließend 3 Stunden bei Raumtemperatur. Man kühlt erneut auf 0°C und gibt 5,19 g (51,27 mmol) Triethylamin/50 ml 2-Propanol zu. Anschließend werden 10,78 g (18,79 mmol) Gadolinium-Komplex von 10-(3-Amino-2-hydroxy-propyl)-1,4,7-tris(carboxymethyl)-1,4,7,10-tetraazacyclododecan (WO 95/17451) gelöst in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylharnstoff ab. Das Filtrat wird zur Trockne eingedampft und durch RP-Chromatographie gereinigt (RP-18/ Laufmittel: Gradient aus Wasser/n-Propanol/ Acetonitril).
Ausbeute: 16,37 g (78 % d. Th.) eines farblosen glasigen Feststoffes
Wassergehalt: 7,1 %
T₁-Relaxivität (L/mmol sec) bei 20 MHz, 37°C:
41 (Wasser)
49 (Humanplasma)

| Elementaranalyse (bezogen auf wasserfreie Substanz): | | | | | | |
|---|---|---|---|---|---|---|
| ber. | C 30,58 | H 3,18 | F 28,31 | Gd 13,78 | N 7,37 | S 2,81 |
| gef. | C 30,40 | H 3,29 | F 28,14 | Gd 13,55 | N 7,28 | S 2,65 |

### d) 10-[2-Hydroxy-4-aza-5-oxo-7-aza-7-(perfluoroctylsulfonyl)-nonyl]-1,4,7-tris(carboxymethyl)-1,4,7,10-tetraazacyclododecan

10 g (8,76 mmol) der Titelverbindung aus Beispiel 1c) werden in einer Mischung aus 100 ml Wasser/100 ml Ethanol gelöst und 1,73 g (13,71 mmol) Oxalsäure-Dihydrat zugesetzt. Man erhitzt 8 Stunden auf 80°C. Es wird auf 0°C abgekühlt und vom ausgefallenem Gadoliniumoxalat abfiltriert. Das Filtrat wird zur Trockne eingedampft und der Rückstand an RP-18 gereinigt (RP-18/Laufmittel: Gradient aus Wasser/i-Propanol/Acetonitril).
Ausbeute: 8,96 g (94 % d. Th.) eines glasigen Feststoffes
Wassergehalt: 9,3 %

| Elementaranalyse (bezogen auf wasserfreie Substanz): | | | | | |
|---|---|---|---|---|---|
| ber. | C 35,30 | H 3,98 | F 32,73 | N 8,52 | S 3,25 |
| gef. | C 35,10 | H 4,15 | F 32,51 | N 8,35 | S 3,15 |

### e) Mangan-Komplex von 10-[2-Hydroxy-4-aza-5-oxo-7-aza-7-(perfluoroctylsulfonyl)-nonyl]-1,4,7-tris(carboxymethyl)-1,4,7,10-tetraazacyclododecan (als Natriumsalz)

5 g (5,07 mmol) der Titelverbindung aus Beispiel 1d) werden in 100 ml Wasser gelöst und 0,58 g (5,07 mmol) Mangan(II) -carbonat zugegeben. Man rührt 3 Stunden bei 80°C. Die Lösung wird filtriert und das Filtrat mit 1 N Natronlauge auf pH 7,2 gestellt, anschließend wird gefriergetrocknet.
Ausbeute: 5,87 g (quantitativ) eines farblosen amorphen Pulvers
Wassergehalt: 8,4 %
T₁-Relaxivität (L/mmol·sec) bei 20 MHz, 37°C:
2,7 (Wasser)
4,2 (Humanplasma)

| Elementaranalyse (bezogen auf wasserfreie Substanz): | | | | | | | |
|---|---|---|---|---|---|---|---|
| ber. | C 32,81 | H 3,42 | F 30,42 | Mn 5,17 | N 7,92 | Na 2,17 | S 3,02 |
| gef. | C 32,62 | H 3,57 | F 30,21 | Mn 5,06 | N 7,80 | Na 2,01 | S 2,90 |

### f) Ytterbium-Komplex von 10-[2-Hydroxy-4-aza-5-oxo-7-aza-7-(perfluoroctylsulfonyl)-nonyl]-1,4,7-tris(carboxymethyl)-1,4,7,10-tetraazacyclododecan

Zu 5 g (5,07 mmol) der Titelverbindung aus Beispiel 1 d) in 100 ml Wasser/30 ml Ethanol gibt man 1,33 g (2,53 mmol) Ytterbiumcarbonat und rührt 3 Stunden bei 80 °C. Die Lösung wird filtriert und das Filtrat im Vakuum zur Trockne eingedampft.
Ausbeute: 6,36 g (quantitativ) eines glasigen Feststoffs.
Wassergehalt: 7,8 %.

| Elementaranalyse (bezogen auf wasserfreie Substanz): | | | | | | |
|---|---|---|---|---|---|---|
| ber. | C 30,11 | H 3,14 | F 27,92 | N 7,27 | S 2,77 | Yb 14,96 |
| gef. | C 30,02 | H 3,27 | F 27,80 | N 7,10 | S 2,68 | Yb 14,75 |

### g) Dysprosium-Komplex von 10-[2-Hydroxy-4-aza-5-oxo-7-aza-7-(perfluoroctylsulfonyl)-nonyl]-1,4,7-tris(carboxymethyl)-1,4,7,10-tetraazacyclododecan

Zu 5 g (5,07 mmol) der Titelverbindung aus Beispiel 1 d) in 100 ml Wasser/30 ml Ethanol gibt man 0,95 g (2,53 mmol) Dysprosiumoxid und rührt 3 Stunden bei 80 °C. Die Lösung wird filtriert und das Filtrat im Vakuum zur Trockne eingedampft.
Ausbeute: 6,35 g (quantitativ) eines farblosen glasigen Feststoffs.
Wassergehalt: 8,5 %.

| Elementaranalyse (bezogen auf wasserfreie Substanz): | | | | | | |
|---|---|---|---|---|---|---|
| ber. | C 30,39 | H 3,17 | F 28,18 | N 7,33 | S 2,80 | Dy 14,18 |
| gef. | C 30,17 | H 3,25 | F 28,03 | N 7,21 | S 2,65 | Dy 14,00 |

### Beispiel 2

### a) 13,13,13,12,12,11,11,10,10,9,9,8,8,7,7,6,6-Heptadecafluor-3-oxa-tridecansäure-t.-butylester

Zu einer Mischung aus 10 g (21,55 mmol) 1H, 1H, 2H, 2H-Perfluordecan-1-ol und 0,73 g (2,15 mmol) Tetrabutylammoniumhydrogensulfat in 100 ml 60 %iger Kalilauge/50 ml Toluol tropft man unter starkem Rühren bei 0°C 10,51 g (53,9 mmol) Bromessigsäure-tert.-butylester zu. Man rührt 1 Stunde bei 0°C. Es werden 200 ml Toluol zugegeben, die wässrige Phase abgetrennt und 2 mal mit je 50 ml Toluol extrahiert. Die vereinigten organischen Phasen werden über Magnesiumsulfat getrocknet und im Vakuum eingedampft. Der Rückstand wird an Kieselgel chromatographiert (Laufmittel: Hexan/Dichlormethan/Aceton= 20/10/1).
Ausbeute: 9,72 g (78 % d. Th.) eines farblosen viskosen Öls

| Elementaranalyse: | | | |
|---|---|---|---|
| ber. | C 33,23 | H 2,61 | F 55,85 |
| gef. | C 33,09 | H 2,78 | F 55,71 |

### b) 13,13,13,12,12,11,11,10,10,9,9,8,8,7,7,6,6-Heptadecafluor-3-oxa-tridecansäure

9,0 g (15,56 mmol) der Titelverbindung aus Beispiel 2a) werden in 180 ml Trifluoressigsäure gelöst und über Nacht bei Raumtemperatur gerührt. Man dampft im Vakuum zur Trockne ein. Der Rückstand wird aus Methanol/Ether umkristallisiert.
Ausbeute: 7,80 g (96 % d. Th.) eines farblosen Feststoffes

| Elementaranalyse: | | | |
|---|---|---|---|
| ber. | C 27,60 | H 1,35 | F 61,85 |
| gef. | C 27,48 | H 1,49 | F 61,66 |

### c) Gadolinium-Komplex von 10-[2-Hydroxy-4-aza-5-oxo-7-oxa-10,10,11,11,12,12,13,13,14,14,15,15,16,16,17,17,17-heptadecafluorheptadecyl]-1,4,7-tris(carboxymethyl)-1,4,7,10-tetraazacyclododecan

7,0 g (13,41 mmol) der Titelverbindung aus Beispiel 2b) und 1,70 g (14,75 mmol) N-Hydroxysuccinimid werden in einer Mischung aus 30 ml Dimethylformamid/ 20 ml Chloroform gelöst. Bei 0°C gibt man 3,04 g (14,75 mmol) Dicyclohexylcarbodiimid zu und rührt 1 Stunde bei 0°C, anschließend 3 Stunden bei Raumtemperatur. Man kühlt erneut auf 0°C und gibt 4,48 g (44,25 mmol) Triethylamin/50 ml 2-Propanol zu. Anschließend werden 8,46 g (14,75 mmol) Gadolinium-Komplex von 10-(3-Amino-2-hydroxy-propyl)-1,4,7-tris(carboxymethyl)-1,4,7,10-tetraazacyclododecan gelöst in 40 ml Wasser zugegeben und 3 Stunden bei Raumtemperatur gerührt. Man dampft zur Trockne ein, nimmt den Rückstand in einer Mischung aus 100 ml Methanol/30 ml Chloroform auf und filtriert vom Dicyclohexylharnstoff ab. Das Filtrat wird zur Trockne eingedampft und durch RP-Chromatographie gereinigt (RP-18/Laufmittel: Gradient aus Wassern-Propanol/Acetonitril).
Ausbeute: 11,8 g (75 % d. Th.) eines farblosen glasigen Feststoffes
Wassergehalt: 8,2 %
T₁-Relaxivität (L/mmol·sec) bei 20 MHz, 37°C:
19 (Wasser)
33 (Humanplasma)

| Elementaranalyse: | | | | | |
|---|---|---|---|---|---|
| ber. | C 32,32 | H 3,27 | F 29,96 | Gd 14,59 | N 6,50 |
| gef. | C 32,16 | H 3,42 | F 29,78 | Gd 14,39 | N 6,40 |

### Beispiel 3

### a) 1,2-Epoxy-4-oxa-1H,1H,2H,3H,3H,5H,5H,6H,6H-perfluor-tetradecan

Zu einer Mischung aus 20 g (43,09 mmol) 1H,1H,2H,2H-Perfluordecan-1-ol und 0,79 g (2,32 mmol) Tetrabutylammoniumhydrogensulfat in 200 ml 60%iger Kalilauge/100 ml Toluol tropft man unter starkem Rühren bei 10°C 7,97 g (86,18 mmol) Epichlorhydrin zu und achtet darauf, daß die Temperatur der Reaktionslösung nicht größer wie 20°C wird. Man läßt 2 Stunden bei 15°C rühren und tropft anschließend wie oben beschrieben 3,99 g (43,09 mmol) Epichlorhydrin zu. Dann wird über Nacht bei Raumtemperatur gerührt. Man gibt 100 ml Methyltert.-butylether zu und trennt die wässrige Phase ab. Diese wird 2 mal mit je 50 ml Toluol nachextrahiert. Die organischen Phasen werden vereinigt, über Magnesiumsulfat getrocknet und im Vakuum eingedampft. Der Rückstand wird an Kieselgel chromatographiert (Laufmittel: Dichlormethan/Hexan/Aceton= 20/10/1).
Ausbeute: 19,05 g (85 % d. Th.) eines farblosen Öls

| Elementaranalyse: | | | |
|---|---|---|---|
| ber. | C 30,02 | H 1,74 | F 62,09 |
| gef. | C 29,87 | H 1,95 | F 61,81 |

### b) 10-[-2Hydroxy-4-oxa-1H,1H,2H,3H,3H,5H,5H,6H,6H-perfluor-tetradecyl]-1,4,7-tris(carboxymethyl)-1,4,7,10-tetraazacyclododecan

Zu 12,0 g (34,60 mmol) 1,4,7-Tris(carboxymethyl)-1,4,7,10-tetraazacyclododecan in 50 ml Wasser gibt man 8,3 g (207,6 mmol) Natriumhydroxid. Hierzu tropft man eine Lösung aus 18,0 g (34,60 mmol) der Titelverbindung aus Beispiel 3a) gelöst in 60 ml n-Butanol/60 ml 2-Propanol und erwärmt die Lösung über Nacht auf 70°C. Man dampft im Vakuum zur Trockne ein, nimmt den Rückstand in 300 ml Wasser auf und stellt mit 3N Salzsäure auf pH 3. Dann wird 2 mal mit 200 ml n-Butanol extrahiert. Die vereinigten Butanolphasen werden im Vakuum zur Trockne eingeengt und der Rückstand durch RP-Chromatographie gereinigt (RP-18/Laufmittel: Gradient aus Wasser/ n-Butanol/Acetonitril).
Ausbeute: 26,61 g (79 % d. Th.)
Wassergehalt: 11,0%

| Elementaranalyse (bezogen auf wasserfreie Substanz): | | | | |
|---|---|---|---|---|
| ber. | C 37,42 | H 4,07 | F 37,27 | N 6,47 |
| gef. | C 37,25 | H 4,19 | F 37,08 | N 6,30 |

### c) Gadolinium-Komplex von 10-[-2Hydroxy-4-oxa-1H,1H,2H,3H,3H,5H,5H,6H,6H-perfluor-tetradecyl]-1,4,7-tris(carboxymethyl)-1,4,7,10-tetraazacyclododecan

10 g (11,54 mmol) der Titelverbindung aus Beispiel 3b) werden in einer Mischung aus 100 ml Wasser/50 ml 2-Propanol gelöst und 2,09 g (5,77 mmol) Gadoliniumoxid zugegeben. Man rührt 3 Stunden bei 80°C. Die Lösung wird filtriert und im Vakuum zur Trockne eingedampft.
Ausbeute: 12,48 g (quantitativ) eines glasigen Feststoffes
Wassergehalt: 5,6 %
T₁-Relaxivität (L/mmol·sec) bei 20 MHz, 37°C:
15,2 (Wasser)
27,5 (Humanplasma)

| Elementaranalyse (bezogen auf wasserfreie Substanz): | | | | | |
|---|---|---|---|---|---|
| ber. | C 31,77 | H 3,16 | F 31,64 | Gd 15,40 | N 5,49 |
| gef. | C 31,55 | H 3,30 | F 31,49 | Gd 15,28 | N 5,35 |

### Beispiel 4

### a) 1,2-Epoxy-4-oxa-1H,1H,2H,3H,3H,5H,5H,6H,6H-perfluordodecan

Zu einer Mischung aus 20 g (54,93 mmol) 1H,1H,2H,2H-Perfluoroctan-1-ol und 1,87 g (5,5 mmol) Tetrabutylammoniumhydrogensulfat in 200 ml 60%iger aqu. Kalilauge/100 ml Toluol tropft man unter starkem Rühren bei 10°C 10,17 g (109,9 mmol) Epichlorhydrin zu und achtet darauf, daß die Temperatur der Reaktionslösung nicht größer wie 20°C wird. Man läßt 2 Stunden bei 15°C rühren und tropft anschließend wie oben beschrieben, 5,08 g (54,93 mmol) Epichlorhydrin zu. Dann wird über Nacht bei Raumtemperatur gerührt. Man gibt 100 ml Toluol und 100 ml Methyl-tert.-butylether zu und trennt die wässrige Phase ab. Diese wird 2 mal mit je 50 ml Toluol nachextrahiert. Die organischen Phasen werden vereinigt, über Magnesiumsulfat getrocknet und im Vakuum eingedampft. Der Rückstand wird an Kieselgel chromatographiert (Laufmittel: Dichlormethan/Hexan/Aceton= 20/10/1).
Ausbeute: 19,15 g (83 % d. Th.) eines farblosen Öls

| Elementaranalyse: | | | |
|---|---|---|---|
| ber. | C 31,44 | H 2,16 | F 58,78 |
| gef. | C 31,40 | H 2,29 | F 58,55 |

### b) 10-[2-Hydroxy-4-oxa-1H,1H,2H,3H,3H,5H,5H,6H,6H-perfluordodecyl]-1,4,7-tris(carboxymethyl)-1,4,7,10-tetraazacyclododecan

Zu 14,84 g (42,84 mmol) 1,4,7-Tris(carboxymethyl)-1,4,7,10-tetraazacyclododecan (DO3A) in 70 ml Wasser gibt man 10,3 g (257 mmol) Natriumhydroxid. Hierzu tropft man eine Lösung aus 18 g (42,84 mmol) der Titelverbindung aus Beispiel 4a) gelöst in 80 ml n-Butanol/60 ml 2-Propanol und erwärmt die Lösung über Nacht auf 70°C. Man dampft im Vakuum zur Trockne ein, nimmt den Rückstand in 300 ml Wasser auf und stellt mit 3N Salzsäure auf pH 3. Dann wird 2 mal mit 200 ml n-Butanol extrahiert. Die vereinigten Butanolphasen werden im Vakuum zur Trockne eingeengt und der Rückstand durch RP-Chromatographie gereinigt (RP-18/Laufmittel: Gradient aus Wasser/ n-Butanol/Acetonitril).
Ausbeute: 27,4 g (75 % d. Th.) eines glasigen Feststoffes
Wassergehalt: 10,1 %

| Elementaranalyse (bezogen auf wasserfreie Substanz): | | | | |
|---|---|---|---|---|
| ber. | C 39,17 | H 4,60 | F 32,22 | N 7,31 |
| gef. | C 39,05 | H 4,85 | F 32,05 | N 7,19 |

### c) Gadolinium-Komplex von 10-[2-Hydroxy-4-oxa-1H,1H,2H,3H,3H,5H,5H,6H,6H-perfluordodecyl]-1,4,7-tris(carboxymethyl)-1,4,7,10-tetraazacyclododecan

10 g (13,04 mmol) der Titelverbindung aus Beispiel 4b) werden in einer Mischung aus 100 ml Wasser/50 ml 2-Propanol gelöst und 2,36 g (6,52 mmol) Gadoliniumoxid zugegeben. Man rührt 3 Stunden bei 80°C. Die Lösung wird filtriert und im Vakuum zur Trockne eingedampft.
Ausbeute: 12,77 g (quantitativ) eines glasigen Feststoffes
Wassergehalt: 6,1 %

| Elementaranalyse (bezogen auf wasserfreie Substanz): | | | | | |
|---|---|---|---|---|---|
| ber. | C 32,61 | H 3,50 | F 26,82 | Gd 17,08 | N 6,08 |
| gef. | C 32,43 | H 3,69 | F 26,67 | Gd 16,85 | N 5,91 |

### Beispiel 5

### a) 9,9,9,8,8,7,7,6,6-Nonafluor-3-oxa-nonansäure-t.-butylester

Zu einer Mischung aus 20 g (75,73 mmol) 1H,1H,2H,2H-Perfluorhexan-1-ol und 2,57 g (7,57 mmol) Tetrabutylammoniumhydrogensulfat in 300 ml 60 %iger aqu. Kalilauge/200 ml Toluol tropft man unter starkem Rühren bei 0°C 29,54 g (151,5 mmol) Bromessigsäure-tert.-butylester zu. Man rührt 1 Stunde bei 0°C. Es werden 100 ml Toluol zugegeben, die wässrige Phase abgetrennt und 2 mal mit 50 ml Toluol extrahiert. Die vereinigten organischen Phasen werden über Magnesiumsulfat getrocknet und im Vakuum eingedampft. Der Rückstand wird an Kieselgel chromatographiert (Laufmittel: Hexan/Dichlormethan/Aceton= 20/10/1).
Ausbeute: 21,48 g (75 % d. Th.) eines farblosen Öls

| Elementaranalyse: | | | |
|---|---|---|---|
| ber. | C 38,11 | H 4,00 | F 45,21 |
| gef. | C 37,95 | H 4,18 | F 45,03 |

### b) 9,9,9,8,8,7,7,6,6-Nonanfluor-3-oxa-nonansäure

20 g (52,88 mmol) der Titelverbindung aus Beispiel 5a) werden in 300 ml Trifluoressigsäure gelöst und über Nacht bei Raumtemperatur gerührt. Man dampft im Vakuum zur Trockne ein. Der Rückstand wird aus Hexan/Ether umkristallisiert.
Ausbeute: 14,82 g (87 % d. Th.) eines farblosen kristallinen Feststoffes

| Elementaranalyse: | | | |
|---|---|---|---|
| ber. | C 29,83 | H 2,19 | F 53,08 |
| gef. | C 29,71 | H 2,40 | F 52,90 |

### c) Gadolinium-Komplex von 10-[2-Hydroxy-4-aza-5-oxo-7-oxa-10,10,11,11,12,12,13,13,13-nonafluor-tridecyl]-1,4,7-tris(carboxymethyl)-1,4,7,10-tetraazacyclododecan

7,41 g (23,01 mmol) der Titelverbindung aus Beispiel 5b) und 2,91 g (25,31 mmol) N-Hydroxysuccinimid werden in einer Mischung aus 40 ml Dimethylformamid/ 20 ml Chloroform gelöst. Bei 0°C gibt man 5,22 g (25,31 mmol) Dicyclohexylcarbodiimid zu und rührt 1 Stunde bei 0°C, anschließend 3 Stunden bei Raumtemperatur. Man kühlt erneut auf 0°C und gibt 6,98 g (69 mmol) Triethylamin/30 ml 2-Propanol zu. Anschließend werden 13,2 g (23,01 mmol) Gadolinium-Komplex von 10-(3-Amino-2-hydroxy-propyl)-1,4,7-tris(carboxymethyl)-1,4,7,10-tetraazacyclododecan gelöst in 40 ml Wasser zugegeben und 3 Stunden bei Raumtemperatur gerührt. Man dampft zur Trockne ein, nimmt den Rückstand in einer Mischung aus 200 ml Methanol/50 ml Chloroform auf und filtriert vom Dicyclohexylharnstoff ab. Das Filtrat wird zur Trockne eingedampft und durch RP-Chromatographie gereinigt (RP-18/Laufmittel: Gradient aus Wasser/n-Propanol/ Acetonitril).
Ausbeute: 15,20 g (71 % d. Th.) eines farblosen glasigen Feststoffes
Wassergehalt: 5,7 %

| Elementaranalyse (bezogen auf wasserfreie Substanz): | | | | | |
|---|---|---|---|---|---|
| ber. | C 34,21 | H 4,02 | F 19,48 | Gd 17,91 | N 7,98 |
| gef. | C 34,09 | H 4,18 | F 19,31 | Gd 17,74 | N 7,87 |

### Beispiel 6

### a) N-Ethyl-N-(perfluoroctylsulfonyl)-amino-essigsäure-N-(2-aminoethyl)-amid

15 g (25,63 mmol) der Titelverbindung aus Beispiel 1b) und 3,24 g (28,19 mmol) N-Hydroxysuccinimid werden in 80 ml Dimethylformamid gelöst und bei 0°C 5,82 g (28,19 mmol) Dicyclohexylcarbodiimid zugegeben. Man rührt 1 Stunde bei 0°C, anschließend 2 Stunden bei Raumtemperatur. Man filtriert vom ausgefallenem Dicyclohexylharnstoff ab und tropft das Filtrat innerhalb 30 Minuten in eine Lösung aus 46,21 g (768,9 mmol) Ethylendiamin in 300 ml Dichlormethan. Man rührt 5 Stunden bei Raumtemperatur. Man setzt 1000 ml H₂O zu und trennt die organische Phase ab. Diese wird 2 mal mit je 500 ml Wasser gewaschen, dann über Magnesiumsulfat getrocknet und im Vakuum zur Trockne eingeengt. Die Reinigung erfolgt durch Chromatographie an Kieselgel. (Laufmittel: Dichlormethan/2-Propanol= 15/1).
Ausbeute: 11,79 g (75 % d. Th.) eines farblosen, wachsartigen Feststoffes

| Elementaranalyse: | | | | | |
|---|---|---|---|---|---|
| ber. | C 27,42 | H 2,30 | F 52,66 | N 4,57 | S 5,23 |
| gef. | C 27,20 | H 2,41 | F 52,48 | N 4,38 | S 5,10 |

### b) N-Ethyl-N-(perfluoroctylsulfonyl)-amino-essigsäure-N-[2-(bromacetyl)-aminoethyl]-amid

10 g (16,3 mmol) der Titelverbindung aus Beispiel 6a) und 2,02 g (20 mmol) Triethylamin werden in 40 ml Dichlormethan gelost. Bei -10°C tropft man innerhalb von 30 Minuten 3,29 g (16,3 mmol) Bromacetylbromid zu und rührt 2 Stunden bei 0°C. Man gießt die Lösung in 300 ml 1 N Salzsäure und rührt gut durch. Die organische Phase wird abgetrennt, über Magnesiumsulfat getrocknet und im Vakuum eingeengt. Der Rückstand wird an Kieselgel chromatographiert (Laufmittel: Dichlormethan/Aceton= 20/1).
Ausbeute: 11,1 g (91 % d. Th.) eines leicht gelbgefärbten wachsartigen Feststoffes

| Elementaranalyse: | | | | | | |
|---|---|---|---|---|---|---|
| ber. | C 25,68 | H 2,02 | Br 10,68 | F 43,16 | N 5,62 | S 4,29 |
| gef. | C 25,47 | H 2,18 | Br 10,45 | F 43,29 | N 5,47 | S 4,10 |

### c) 10-[2-Oxo-3-aza-6-aza-7-oxo-9-aza-9-(perfluoroctylsulfonyl)-undecyl]-1,4,7-tris(carboxymethyl)- 1,4,7,10-tetraazacyclododecan

Zu 10 g (13,36 mmol) der Titelverbindung aus Beispiel 6b) in 180 ml Methanol gibt man 4,63 g (13,36 mmol) 1,4,7-Tris(carboxymethyl)-1,4,7,10-tetraazacyclododecan (DO3A) und 18,5 g (133,6 mmol) Kaliumkarbonat. Man kocht 12 Stunden unter Rückfluss. Die anorganischen Salze werden abfiltriert und das Filtrat zur Trockne eingedampft. Der Rückstand wird in 100 ml Wasser aufgenommen und mit 5 N Salzsäure auf pH 3 gestellt. Man extrahiert 2 mal mit 150 ml n-Butanol. Die vereinigten organischen Phasen werden im Vakuum zur Trockne eingeengt und der Rückstand durch RP-Chromatographie gereinigt (RP-18/Laufmittel= Gradient aus Wasser/n-Butanol/Acetonitril).
Ausbeute: 10,43 g (67 % d. Th.) eines farblosen Feststoffes
Wassergehalt: 13,0%

| Elementaranalyse (bezogen auf wasserfreie Substanz): | | | | | |
|---|---|---|---|---|---|
| ber. | C 35,55 | H 3,98 | F 31,86 | N 9,67 | S 3,16 |
| gef. | C 35,37 | H 3,75 | F 31,64 | N 9,78 | S 3,25 |

### d) Gadolinium-Komplex von 10-[2-Oxo-3-aza-6-aza-7-oxo-9-aza-9-(perfluoroctylsulfonyl)-undecyl]-1,4,7-tris(carboxymethyl)-1,4,7,10-tetraazacyclododecan

10 g (9,86 mmol) der Titelverbindung aus Beispiel 6c) werden in einer Mischung aus 50 ml Wasser/20 ml Ethanol gelöst und 1,79 g (4,93 mmol) Gadoliniumoxid zugegeben. Man rührt 4 Stunden bei 80°C. Die Lösung wird filtriert und im Vakuum zur Trockne eingedampft.
Ausbeute: 12,4 g (quantitativ)
Wassergehalt: 7,1 %

| Elementaranalyse (bezogen auf wasserfreie Substanz): | | | | | | |
|---|---|---|---|---|---|---|
| ber. | C 30,85 | H 3,19 | F 27,65 | Gd 13,46 | N 8,39 | S 2,75 |
| gef. | C 30,64 | H 3,35 | F 27,58 | Gd 13,29 | N 8,28 | S 2,65 |

### Beispiel 7

### a) 1H,1H,2H,2H-Perfluordecan-1-ol-p-toluolsulfonsäureester

Zu 30 g (64,64 mmol) 1H,1H,2H,2H-Perfluordecan-1-ol in 300 ml Dichlormethan und 10,12 g (100 mmol) Triethylamin gibt man bei 0°C 12,57 g (65,93 mmol) p-Toluolsulfonsäurechlorid. Man rührt 2 Stunden bei 0°C, anschließend 2 Stunden bei Raumtemperatur. Die Lösung wird in 500 ml kalter 2 N Salzsäure gegossen und kräftig gerührt. Die organische Phase wird abgetrennt, über Magnesiumsulfat getrocknet und zur Trockne eingeengt. Der Rückstand wird aus wenig Methanol umkristallisiert.
Ausbeute: 39,97 (95 % d. Th.) eines farblosen kristallinen Pulvers

| Elementaranalyse: | | | | |
|---|---|---|---|---|
| ber. | C 33,02 | H 1,79 | F 52,23 | S 5,19 |
| gef. | C 32,81 | H 1,93 | F 52,04 | S 5,05 |

### b) 10-[(1-Hydroxymethyl-1-carboxy)-methyl]-1,4,7-tris(carboxymethyl)-1,4,7,10-tetraazacyclododecan

Zu einer Lösung aus 20 g (57,78 mmol) 1,4,7-Tris(carboxymethyl)-1,4,7,10-tetraazacyclododecan (DO3A), 31,21 g (780 mmol) Natriumhydroxid und 2 g (12 mmol) Kaliumjodid in 100 ml Dimethylformamid gibt man 37,2 g (173,4 mmol) 2-Chlor-3-benzyloxy-propansaure und rührt 3 Tage bei 60°C. Man dampft zur Trockne ein und löst den Rückstand in 300 ml Wasser. Dann stellt man mit 3 N Salzsäure auf pH 3 und extrahiert 2 mal mit je 250 ml Dichlormethan. Zur Wasserphase gibt man 4 g Palladiumkatalysator (10 % Pd/C) und hydriert 5 Stunden bei 60°C. Der Katalysator wird abfiltriert und das Filtrat zur Trockne eingeengt. Der Rückstand wird durch RP-Chromatographie gereinigt (RP-18/Laufmittel= Gradient aus Wasser/2-Propanol/ Acetonitril).
Ausbeute: 5,92 g (21 % d. Th. bezogen auf DO3A) eines farblosen glasigen Feststoffes
Wassergehalt: 11,1 %

| Elementaranalyse (bezogen auf wasserfreie Substanz): | | | |
|---|---|---|---|
| ber. | C 47,00 | H 6,96 | N 12,90 |
| gef. | C 46,81 | H 6,78 | N 12,99 |

### c) 10-[1-Hydroxymethyl-1-(methoxycarbonyl)-methyl]-1,4,7-tris(methoxycarbonylmethyl)-1,4,7,10-tetraazacyclododecan

Zu 200 ml Methanol tropft man bei 0°C 9,53 g (80 mmol) Thionylchlorid. Dann gibt man 5,8 g (13,35 mmol) der Titelverbindung aus Beispiel 7b) zu und rührt 1 Stunde bei 0°C. Dann erwärmt man 6 Stunden auf 60°C. Man dampft zur Trockne ein, nimmt den Rückstand in 150 ml Methylenchlorid auf und extrahiert 3 mal mit je 200 ml 8 %iger aqu. Soda-Lösung. Die organische Phase wird über Magnesiumsulfat getrocknet und zur Trockne eingedampft. Man erhält 6,09 g (93 % d. Th.) der Titelverbindung als leicht gelblich gefärbtes Öl.

| Elementaranalyse: | | | |
|---|---|---|---|
| ber. | C 51,42 | H 7,81 | N 11,42 |
| gef. | C 51,20 | H 7,95 | N 11,28 |

### d) 10-[1-(Methoxycarbonyl)-3-oxa-1H,2H,2H,4H,4H,5H,5H-perfluortridecyl]-1,4,7-tris(methoxycarbonylmethyl)-1,4,7,10-tetraazacyclododecan

Zu 6 g (12,23 mmol) der Titelverbindung aus Beispiel 7c) in 40 ml Dimethylformamid gibt man 0,44 g (14,68 mmol) Natriumhydrid (80 %ige Suspension in Mineralöl) und rührt 30 min bei -10°C. Dann werden 8,32 g (13,45 mmol) der Titelverbindung aus Beispiel 7a) zugesetzt und 8 Stunden bei Raumtemperatur gerührt. Man setzt vorsichtig 400 ml Eiswasser zu und extrahiert 2 mal mit je 300 ml Essigsäureethylester. Die vereinigten Essigsäureethylester-phasen werden mit gesättigter aqu. Kochsalzlösung gewaschen und über Magnesiumsulfat getrocknet. Man dampft im Vakuum zur Trockne ein und chromatographiert den Rückstand an Kieselgel (Laufmittel: Dichlormethan/ Methanol= 20/1).
Ausbeute: 7,68 g (67 % d. Th.) eines zähen gelben Öls

| Elementaranalyse: | | | | |
|---|---|---|---|---|
| ber. | C 39,75 | H 4,41 | F 34,48 | N 5,98 |
| gef. | C 39,58 | H 4,60 | F 34,27 | N 5,75 |

### e) 10-[1-Carboxy-3-oxa-1H,2H,2H,4H,4H,5H,5H-perfluortridecyl]-1,4,7-tris(carboxymethyl)-1,4,7,10-tetraazacyclododecan

7,5 g (8,01mmol) der Titelverbindung aus Beispiel 7d) wird in einer Mischung aus 50 ml Wasser/30 ml Ethanol suspendiert und anschließend 3,84 g (96 mmol) Natriumhydroxid zugegeben. Man kocht über Nacht unter Rückfluß. Es wird auf Raumtemperatur abgekühlt und mit 3 N Salzsäure auf pH 3 gestellt. Man dampft im Vakuum zur Trockne ein und reinigt den Rückstand durch RP-Chromatographie (RP-18/Laufmittel= Gradient aus Wasser/n-Butanol/Acetonitril).
Ausbeute: 6,84 g (87 % d. Th.) eines glasigen Feststoffes
Wassergehalt: 10,3 %

| Elementaranalyse (bezogen auf wasserfreie Substanz): | | | | |
|---|---|---|---|---|
| ber.: | C 36,83 | H 3,78 | F 36,68 | N 6,36 |
| gef.: | C 36,67 | H 3,90 | F 36,49 | N 6,25 |

### f) Gadolinium-Komplex von 10-[1-Carboxy-3-oxa-1H,2H,2H,4H,4H,5H,5H-perfluortridecyl]1,4,7-tris(carboxymethyl)-1,4,7,10-tetraazacyclododecan (als Natriumsalz)

6 g (6,81 mmol) der Titelverbindung aus Beispiel 7e) werden in 80 ml Wasser suspendiert und 1,23 g (3,4 mmol) Gadoliniumoxid zugegeben. Man erhitzt 3 Stunden auf 90°C. Man läßt auf Raumtemperatur abkühlen und stellt mit 2 N Natronlauge auf pH 7,2 ein. Die Lösung wird filtriert und anschließend gefriergetrocknet.
Ausbeute: 7,83 g (quantitativ) eines farblosen, flockigen Pulvers
Wassergehalt: 8,1 %

| Elementaranalyse (bezogen auf wasserfreie Substanz): | | | | | | |
|---|---|---|---|---|---|---|
| ber. | C 30,69 | H 2,77 | F 30,56 | Gd 14,88 | N 5,30 | Na 2,18 |
| gef. | C 30,48 | H 2,85 | F 30,37 | Gd 14,69 | N 5,17 | Na 1,95 |

### Beispiel 8

### a) 2H,2H-Perfluoroctanal

30 g (82,4 mmol) 1H,1H,2H,2H-Perfluoroctan-1-ol werden in 500 ml Dichlormethan gelöst und 17,76 g (82,4 mmol) Pyridiniumchlorochromat zugegeben. Man rührt über Nacht bei Raumtemperatur. Die Lösung wird über eine kurze Säule, gefüllt mit Aluminiumoxid (neutral) filtriert, das Filtrat zur Trockne eingedampft und der Rückstand an Kieselgel chromatographiert (Laufmittel: Dichlormethan/ Hexan/Aceton=10/10/1)
Ausbeute: 26,55 g (89 % d. Th.) eines wachsartigen Feststoffes

| Elementaranalyse: | | | |
|---|---|---|---|
| ber. | C 26,54 | H 0,84 | F 68,21 |
| gef. | C 26,47 | H 1,05 | F 68,10 |

### b) 2-Amino-2H,3H,3H-perfluomonansäure (als Hydrochlorid)

7,04 g (143,6 mmol) Natriumcyanid und 8,45 g (158 mmol) Ammoniumchlorid werden in 30 ml Wasser gelöst. Zu dieser Lösung gibt man 40 ml Ethanol und 26 g (71,8 mmol) der Titelverbindung aus Beispiel 8a). Man erwärmt 2 Stunden auf 45°C. Es wird 300 ml Wasser zugesetzt und 3 mal mit je 200 ml Benzol extrahiert. Die vereinigten Benzolphasen werden 3 mal mit je 200 ml Wasser gewaschen und die organische Phase im Vakuum zur Trockne eingedampft. Der Rückstand wird in 100 ml 6 N aqu. Salzsäure/50 ml Methanol aufgenommen und 2 Stunden unter Rückfluß erhitzt. Man dampft im Vakuum zur Trockne ein. Der Rückstand wird aus wenig 2-Propanol/Methyl-tert.-butylether umkristallisiert.
Ausbeute: 11,15 g (35 % d. Th.) eines kristallinen Feststoffes

| Elementaranalyse: | | | | | |
|---|---|---|---|---|---|
| ber. | C 24,37 | H 1,59 | Cl 7,99 | F 55,68 | N 3,16 |
| gef. | C 24,15 | H 1,72 | Cl 7,65 | F 55,51 | N 3,05 |

### c) 2-[(N-Benzyloxycarbonyl)-triglycidyl]-amino-2H,3H,3H-perfluornonansäure

8,37 g (24,8 mmol) N-Benzyloxycarbonyl-Triglycin und 3,14 g (27,28 mmol) N-Hydroxysuccinimid werden in 80 ml Dimethylformamid gelöst und bei 0°C 5,63 g (27,28 mmol) Dicyclohexylcarbodiimid zugegeben. Man rührt 1 Stunde bei 0°C, dann 2 Stunden bei Raumtemperatur. Man kühlt auf 0°C ab, gibt 7,53 g (74,4 mmol) Triethylamin und 11 g (24,8 mmol) der Titelverbindung aus Beispiel 8b zu und rührt anschließend über Nacht bei Raumtemperatur. Man dampft im Vakuum zur Trockne ein, nimmt den Rückstand in 300 ml 5 %iger aqu. Zitronensäure auf und extrahiert 3 mal mit je 200 ml Essigsaureäthylester. Die vereinigten organischen Phasen werden über Magnesiumsulfat getrocknet und im Vakuum zur Trockne eingedampft. Der Rückstand wird an Kieselgel chromatographiert (Laufmittel: Dichlormethan/n-Propanol= 20/1).
Ausbeute: 11,83 g (67 % d. Th.) eines farblosen, schuppenartigen Feststoffes

| Elementaranalyse: | | | | |
|---|---|---|---|---|
| ber. | C 38,78 | H 2,97 | F 34,67 | N 7,86 |
| gef. | C 38,59 | H 2,85 | F 34,48 | N 7,91 |

### d) 2-[Triglycidyl]-amino-2H,3H,3H-perfluornonansäure

11,5, g (16,14 mmol) der Titelverbindung aus Beispiel 8c) werden in 200 ml 2-Propanol gelöst und 3 g Palladium-Katalysator (10 % Pd/C) zugegeben. Man hydriert über Nacht bei Raumtemperatur. Es wird vom Katalysator abfiltriert und das Filtrat zur Trockne eingedampft.
Ausbeute: 9,33 g (quantitativ) eines farblosen Feststoffes

| Elementaranalyse: | | | | |
|---|---|---|---|---|
| ber. | C 31,15 | H 2,61 | F 42,71 | N 9,69 |
| gef. | C 31,29 | H 2,80 | F 42,53 | N 9,48 |

### e) 2-(1H,1H-Perfluorheptyl)-1,4,7,10-tetraaza-3,6,9,12-tetraoxo-cyclododecan

9,2 g (15,91 mmol) der Titelverbindung aus Beispiel 8d) werden in 1000 ml Dimethylformamid gelöst und 3,93 g (15,91 mmol) 2-Ethoxy-1-ethoxycarbonyl-1,2-dihydroquinolin zugegeben. Man rührt 3 Tage bei Raumtemperatur. Es wird zur Trockne eingedampft und der Rückstand an Kieselgel chromatographiert (Laufmittel: Dichlormethan/2-Propanol= 20/1).
Ausbeute: 4,54 g (51 % d. Th.) eines wachsartigen Feststoffes

| Elementaranalyse: | | | | |
|---|---|---|---|---|
| ber. | C 32,16 | H 2,34 | F 44,08 | N 10,00 |
| gef. | C 32,05 | H 2,47 | F 43,87 | N 9,89 |

### f) 2-(1H,1H-Perfluorheptyl)-1,4,7,10-tetraazacyclododecan (als Tetrahydrochlorid)

Zu 4,4 g (7,85 mmol) der Titelverbindung aus Beispiel 8e) gibt man 200 ml 1 M-Boran-Tetrahydrofuran-Komplex-Lösung zu und kocht 2 Tage unter Rückfluß. Man dampft im Vakuum zur Trockne ein und nimmt den Rückstand in 50 ml konz. Salzsäure auf. Es werden 100 ml Ethanol zugegeben und 8 Stunden unter Rückfluß gekocht. Es wird im Vakuum zur Trockne eingedampft und der Rückstand aus Ethanol umkristallisiert.
Ausbeute: 4,75 g (93 % d. Th.) eines farblosen kristallinen Pulvers

| Elementaranalyse: | | | | | |
|---|---|---|---|---|---|
| ber. | C 27,71 | H 3,88 | Cl 21,81 | F 37,99 | N 8,62 |
| gef. | C 27,65 | H 3,95 | Cl 21,40 | F 37,69 | N 8,41 |

### g) 2-(1H,1H-perfluorheptyl)-1,4,7,10-tetra(carboxymethyl)-1,4,7,10-tetraazacyclododecan

4,6 g (7,07 mmol) der Titelverbindung aus Beispiel 8f) und 4,0 g (42,4 mmol) Chloressigsäure werden in 40 ml Wasser gelöst und der pH durch Zugabe von 30 %iger aqu. Kalilauge auf pH 10 gestellt. Man erwarmt 8 Stunden auf 70°C und hält dabei den pH-Wert zwischen 8 und 10 (durch Zugabe von 30 %iger aqu. Kalilauge). Die Lösung wird auf Raumtemperatur abgekühlt, mit konz. Salzsäure auf pH 2 gestellt und zur Trockne eingedampft. Der Rückstand wird in 150 ml Methanol aufgenommen, die Salze abfiltriert und das Filtrat im Vakuum zur Trockne eingedampft. Der Rückstand wird durch RP-18 Chromatographie gereinigt (RP-18/Laufmittel: Gradient aus Wasser/2-Propanol/Acetonitril). Ausbeute: 5,03 g (87 % d. Th.) eines glasigen Feststoffes
Wassergehalt: 10,1%

| Elementaranalyse (bezogen auf wasserfreie Substanz): | | | | |
|---|---|---|---|---|
| ber. | C 37,51 | H 3,97 | F 33,53 | N 7,61 |
| gef. | C 37,35 | H 4,12 | F 33,40 | N 7,45 |

### h) Gadolinium-Komplex von 2-(1H,1H-perfluorheptyl)-1,4,7,10-tetra(carboxymethyl)-1,4,7,10-tetraazacyclododecan (als Natriumsalz)

4,5 g (6,11 mmol) der Titelverbindung aus Beispiel 8g) werden in 100 ml Wasser suspendiert und 1,107 g (3,05 mmol) Gadoliniumoxid zugegeben. Man erhitzt 3 Stunden auf 90°C. Man läßt auf Raumtemperatur abkühlen und stellt mit 2 N Natronlauge auf pH 7,2 ein. Die Lösung wird filtriert und anschließend gefriergetrocknet.
Ausbeute: 6,03 g (quantitativ) eines farblosen Pulvers
Wassergehalt: 7,5 %

| Elementaranalyse (bezogen auf wasserfreie Substanz): | | | | | | |
|---|---|---|---|---|---|---|
| ber. | C 30,23 | H 2,87 | F 27,03 | Gd 17,21 | N 6,13 | Na 2,52 |
| gef. | C 30,10 | H 3,05 | F 26,81 | Gd 17,15 | N 5,95 | Na 2,30 |

### Beispiel 9

### a) 10-[2-Hydroxy-1H,1H,2H,3H,3H-perfluornonyl]-1,4,7-tris(carboxymethyl)-1,4,7,10-tetraazacyclododecan

Zu 15 g (43,3 mmol) l,4,7-Tris(carboxymethyl)-1,4,7,10-tetraazacyclododecan in 50 ml Wasser gibt man 13,85 g (346,4 mmol) Natriumhydroxid. Hierzu tropft man eine Lösung aus 27,68 g (64,95 mmol) 1,2-Epoxy-1H,1H,2H,3H,3H-Perfluornonan gelöst in 50 ml n-Butanol/50 ml 2-Propanol und erwärmt die Lösung über Nacht auf 80°C. Man dampft im Vakuum zur Trockne ein, nimmt den Rückstand in 200 ml Wasser auf und stellt mit 3 N Salzsäure auf pH 3. Dann wird 2 mal mit 200 ml n-Butanol extrahiert. Die vereinigten Butanolphasen werden im Vakuum zur Trockne eingeengt und der Rückstand durch RP-Chromatographie gereinigt (RP-18/Laufmittel: Gradient aus Wasser/ n-Butanol/Acetonitril).
Ausbeute: 30,34 g (78 % d. Th.) eines glasigen Feststoffes
Wassergehalt: 13,7 %

| Elementaranalyse (bezogen auf wasserfreie Substanz): | | | | |
|---|---|---|---|---|
| ber. | C 37,32 | H 4,04 | F 36,89 | N 7,25 |
| gef. | C 37,15 | H 4,21 | F 36,70 | N7,19 |

### b) Gadolinium-Komplex von 10-[2-Hydroxy-1H,1H,2H,3H,3H-perfluornonyl]-1,4,7-tris(carboxymethyl)-1,4,7,10-tetraazacyclododecan

10 g (12,94 mmol) der Titelverbindung aus Beispiel 9a) werden in 100 ml Wasser/50 ml Ethanol gelöst und 2,34 g (6,47 mmol) Gadoliniumoxid zugegeben. Man rührt 3 Stunden bei 80°C. Die Lösung wird filtriert und im Vakuum zur Trockne eingedampft.
Ausbeute: 13,16 g (quantitativ) eines farblosen glasigen Feststoffes
Wassergehalt: 9,1 %

| Elementaranalyse (bezogen auf wasserfreie Substanz): | | | | | |
|---|---|---|---|---|---|
| ber. | C 31,11 | H 3,05 | F 30,75 | Gd 16,97 | N 6,05 |
| gef. | C 31,01 | H 3,19 | F 30,55 | Gd 16,71 | N 5,88 |

### Beispiel 10

### a) 9H,9H,10H,11H,12H,12H-Perfluoreicos-10-en

24,77 g (52,26 mmol) 1H,1H,2H,2H-Perfluorodecyl-1-jodid und 13,71 g (52,26 mmol) Triphenylphosphin werden in 500 ml Aceton unter Rühren auf 70°C erhitzt. Die anfangs klare Lösung wird rasch milchig trübe und scheidet das farblose Phosphoniumsalz ab. Man filtriert das Phosphoniumsalz ab, trocknet im Vakuum bei 40°C.
Ausbeute: 38,9 g (89 % d. Th.)

Dieses Phosphoniumsalz wird ohne Reinigung direkt in der folgenden Reaktion verwendet: Zum oben hergestellten Phosphoniumsalz, 38,9 g (46,5 mmol) in 250 ml Dichlormethan gibt man 5,22 g (46,5 mmol) Kalium-tert.-butylat, 0,20 g (0,75 mmol) 18-Krone-6 und 19,54 g (42,28 mmol) 2H,2H-Perfluordecanal zu und rührt 10 Stunden bei Raumtemperatur. Man dampft zur Trockne ein und chromatographiert den Rückstand an Kieselgel (Laufmittel: Dichlormethan/n-Hexan/Diethylether= 10/20/1).
Ausbeute: 30,3 g (65 % d. Th. bezogen auf eingesetztes Jodid) eines farblosen, wachsarten Feststoffes

| Elementaranalyse: | | | |
|---|---|---|---|
| ber. | C 26,92 | H 0,68 | F 72,40 |
| gef. | C 26,81 | H 0,79 | F 72,20 |

### b) 10,11-Epoxy-9H,9H,10H,11H,12H,12H-perfluoreicosan

Zu 25 g (28,02 mmol) der Titelverbindung aus Beispiel 10a) gelöst in 250 ml Dichlormethan gibt man bei 0°C 10,47 g (36,42 mmol) 3-Chlorperoxybenzoesaure (ca. 60 %ig) und rührt über Nacht bei Raumtemperatur. Man setzt 300 ml 5 %ige aqu. Natriumcarbonat-Lösung zu und rührt gut durch. Die organische Phase wird abgetrennt, über Magnesiumsulfat getrocknet und im Vakuum zur Trockne eingedampft. Der Rückstand wird an Kieselgel chromatographiert (Laufmittel: n-Hexan/Dichlormethan/Diethylether= 10/10/1).
Ausbeute: 24,17 g (95 % d. Th.) eines farblosen Feststoffes

| Elementaranalyse: | | | |
|---|---|---|---|
| ber. | C 26,45 | H 0,67 | F 71,12 |
| gef. | C 26,25 | H 0,88 | F 71,35 |

### c) 10-[1-(1H,1H-Perfluornonyl)-2-hydroxy-1H,2H,3H,3H-perfluorundecyl]-1,4,7-tris(carboxymethyl)-1,4,7,10-tetraazacyclododecan

Zu 7,63 g (22,02 mmol) 1,4,7-Tris(carboxymethyl)-1,4,7,10-tetraazacyclododecan in 35 ml Wasser gibt man 7,04 g (0,176 mmol) Natriumhydroxid. Hierzu tropft man eine Lösung aus 20 g (22,02 mmol) der Titelverbindung aus Beispiel 10b) gelöst in 50 ml n-Butanol/40 ml 2-Propanol und erwärmt die Lösung über Nacht auf 120°C im Autoklaven. Man dampft im Vakuum zur Trockne ein, nimmt den Rückstand in 200 ml Wasser auf und stellt mit 3 N Salzsäure auf pH 3. Dann wird 2 mal mit 300 ml n-Butanol extrahiert. Die vereinigten Butanolphasen werden im Vakuum zur Trockne eingeengt und der Rückstand durch RP-Chromatographie gereinigt (RP-18/Laufmittel: Gradient aus Wasser/n-Butanol/Acetonitril).
Ausbeute: 9,79 g (31 % d.Th.) eines farblosen, glasigen Feststoffes
Wassergehalt: 12,5 %

| Elementaranalyse (bezogen auf wasserfreie Substanz): | | | | |
|---|---|---|---|---|
| ber. | C 32,55 | H 2,57 | F 51,49 | N 4,47 |
| gef. | C 32,38 | H 2,75 | F 51,29 | N 4,28 |

### d) Gadolinium-Komplex von 10-[1-(1H,1H-Perfluornonyl)-2-hydroxy-1H,2H,3H,3H-perfluorundecyl]-1,4,7-tris(carboxymethyl)-1,4,7,10-tetraazacyclododecan

8 g (6,38 mmol) der Titelverbindung aus Beispiel 10c) werden in 50 ml Wasser/40 ml Ethanol/20 ml Chloroform gelöst und 1,16 g (3,19 mmol) Gadoliniumoxid zugegeben. Man rührt 4 Stunden bei 90 °C im Autoklaven. Die Lösung wird filtriert und im Vakuum zur Trockne eingedampft.
Ausbeute: 9,47 g (quantitativ) eines glasigen Feststoffes
Wassergehalt: 5,2 %

| Elementaranalyse (bezogen auf wasserfreie Substanz): | | | | | |
|---|---|---|---|---|---|
| ber. | C 28,99 | H 2,07 | F 45,85 | Gd 11,16 | N 3,98 |
| gef. | C 28,81 | H 2,19 | F 45,71 | Gd 11,03 | N 4,12 |

### Beispiel 11

### a) 7H,7H,8H,9H,10H,10H-Perfluorhexadec-8-en

18,7 g (50 mmol) 1H,1H,2H,2H-Perfluoroctyl-1-jodid und 13,11 g (50 mmol) Triphenyl phosphin werden in 400 ml Aceton unter Rühren auf 70°C erhitzt. Die anfangs klare Lösung wird rasch milchig trübe und scheidet das farblose Phosphoniumsalz ab. Man filtriert das Phosphoniumsalz ab, trocknet im Vakuum bei 40°C.
Ausbeute: 28,95 g (91 % d. Th.)
Dieses Phosphoniumsalz wird ohne Reinigung direkt in der folgenden Reaktion verwendet: Zum oben hergestellten Phosphoniumsalz, 28,95 g (45,5 mmol) in 200 ml Dichlormethan gibt man 5,05 g (45,5 mmol) Kalium-tert. butylat, 0,20 g (0,75 mmol) 18-Krone-6 und 14,98 g (41,36 mmol) der Titelverbindung aus Beispiel 8a) zu und rührt 10 Stunden bei Raumtemperatur. Man dampft zur Trockne ein und chromatographiert den Rückstand an Kieselgel (Laufmittel: Dichlormethan/n-Hexan/Diethylether= 10/20/1).
Ausbeute: 19,65 g (61 % d. Th.) eines farblosen wachsartigen Feststoffes

| Elementaranalyse: | | | |
|---|---|---|---|
| ber. | C 22,38 | H 0,94 | F 76,69 |
| gef. | C 22,20 | H 0,99 | F 76,51 |

### b) 8,9-Epoxy-7H,7H,8H,9H,10H,10H-perfluorhexadecan

Zu 19 g (29,5 mmol) der Titelverbindung aus Beispiel lla) gelöst in 200 ml Dichlormethan gibt man bei 0°C 11,03 g (38,35 mmol) 3-Chlorperoxybenzoesäure (ca. 60 %ig) und rührt über Nacht bei Raumtemperatur. Man setzt 300 ml 5 %ige aqu. Natriumcarbonat-Lösung zu und rührt gut durch. Die organische Phase wird abgetrennt, über Magnesiumsulfat getrocknet und im Vakuum zur Trockne eingedampft. Der Rückstand wird an Kieselgel chromatographiert (Laufmittel: n-Hexan/Dichlormethan/Diethylether= 10/10/1).
Ausbeute: 19,43 g (93 % d. Th.) eines farblosen Feststoffes

| Elementaranalyse: | | | |
|---|---|---|---|
| ber. | C 27,14 | H 0,85 | F 69,75 |
| gef. | C 27,01 | H 0,97 | F 69,60 |

### c) 10-[1-(1H,1H-Perfluorheptyl)-2-hydroxy-1H,2H,3H,3H-perfluornonyl]-1,4,7-tris(carboxymethyl)-1,4,7,10-tetraazacyclododecan

Zu 9,3 g (26,83 mmol) 1,4,7-Tris(carboxymethyl)-1,4,7,10-tetraazacyclodo- decan in 50 ml Wasser gibt man 8,59 g (214,6 mmol) Natriumhydroxid. Hierzu tropft man eine Lösung aus 19 g (26,83 mmol) der Titelverbindung aus Beispiel 11b) gelöst in 70 ml n-Butanol/60 ml 2-Propanol und erwärmt die Lösung über Nacht auf 120°C im Autoklaven. Man dampft im Vakuum zur Trockne ein, nimmt den Rückstand in 200 ml Wasser auf und stellt mit 3 N Salzsäure auf pH 3. Dann wird 2 mal mit 300 ml n-Butanol extrahiert. Die vereinigten Butanolphasen werden im Vakuum zur Trockne eingeengt und der Rückstand durch RP-Chromatographie gereinigt (RP-18/Laufmittel: Gradient aus Wasser/n-Butanol/Acetonitril).
Ausbeute: 9,4 g (29 % d.Th.) eines glasigen Feststoffes
Wassergehalt: 12,7 %

| Elementaranalyse (bezogen auf wasserfreie Substanz): | | | | |
|---|---|---|---|---|
| ber. | C 34,17 | H 3,06 | F 46,84 | N 5,31 |
| gef. | C 33,98 | H 3,18 | F 46,65 | N 5,20 |

### d) Gadolinium-Komplex von 10-[1-(1H,1H-Perfluorheptyl)-2-hydroxy-1H,2H,3H,3H-perfluornonyl]-1,4,7-tris(carboxymethyl)-1,4,7,10-tetraazacyclododecan

9 g (8,53 mmol) der Titelverbindung aus Beispiel 11c) werden in 60 ml Wasser/40 ml Ethanol/30 ml Chloroform gelöst und 1,54 g (4,27 mmol) Gadoliniumoxid zugegeben. Man rührt 4 Stunden bei 90°C im Autoklaven. Die Lösung wird filtriert und im Vakuum zur Trockne eingedampft
Ausbeute: 11,45 g (quantitativ) eines farblosen, glasigen Feststoffes
Wassergehalt: 10,2 %

| Elementaranalyse (bezogen auf wasserfreie Substanz): | | | | | |
|---|---|---|---|---|---|
| ber. | C 29,81 | H 2,42 | F 40,86 | Gd 13,01 | N 4,63 |
| gef. | C 29,60 | H 2,60 | F 40,63 | Gd 12,84 | N 4,51 |

### Beispiel 12

### a) 7,12-Dioxa-5H,5H,6H,6H,8H,8H,9H,10H,11H,11H,13H,13H,14H,14H-perfluoroctadec-9-en

30 g (91,74 mmol) 1H,1H,2H,2H-Perfluorhexyl-1-bromid werden in 100 ml Toluol gelöst dann 3,23 g (36,7 mmol) cis-1,4-Buten-diol und 1 g (2,95 mmol) Tetrabutylammoniumhydrogensulfat zugegeben. Man kühlt auf 0°C ab und fügt 16 g (400 mmol) feingepulvertes Natriumhydroxid zu. Dann wird 1 Stunde bei 0°C und über Nacht bei Raumtemperatur gerührt. Man filtriert vom Feststoff ab, wäscht das Filtrat zweimal mit je 200 ml Wasser, trocknet die organische Phase über Magnesiumsulfat und dampft dann im Vakuum zur Trockne ein. Der Rückstand wird an Kieselgel chromatographiert (Laufmittel: Dichlormethan/n-Hexan/Aceton= 15/15/1).
Ausbeute: 11,71 g (55 % d. Th. bezogen aufDiol) eines wachsartigen Feststoffes

| Elementaranalyse: | | | |
|---|---|---|---|
| ber. | C 33,12 | H 2,43 | F 58,93 |
| gef. | C 33,05 | H 2,61 | F 58,73 |

### b) 9,10-Epoxy-7,12-dioxa-5H,5H,6H,6H,8H,8H,9H,10H,11H,11H,13H,13H,14H,14H-perfluoroctadecan

Zu 11 g (18,96 mmol) der Titelverbindung aus Beispiel 12a) gelöst in 100 ml Dichlormethan gibt man bei 0°C 7,08 g (24,64 mmol) 3-Chlorperoxybenzoesäure (ca. 60 %ig) und rührt über Nacht bei Raumtemperatur. Man setzt 150 ml 5 %ige aqu. Natriumcarbonat-Lösung zu und rührt gut durch. Die organische Phase wird abgetrennt, über Magnesiumsulfat getrocknet und im Vakuum zur Trockne eingedampft. Der Rückstand wird an Kieselgel chromatographiert (Laufmittel: n-Hexan/Dichlormethan/Diethylether= 10/10/1).
Ausbeute: 10,74 g (95 % d. Th.) eines farblosen Feststoffes

| Elementaranalyse: | | | |
|---|---|---|---|
| ber. | C 32,23 | H 2,37 | F 57,35 |
| gef. | C 32,13 | H 2,51 | F 57,20 |

### c) 10-[1-(2-Oxa-1H,1H,3H,3H,4H,4H-perfluoroctyl)-2-hydroxy-4-oxa-1H,2H,3H,3H,5H,5H,6H,6H-perfluordecyl]-1,4,7-tris(carboxymethyl)-1,4,7,10-tetraazacyclododecan

Zu 6,1 g (17,61 mmol) 1,4,7-Tris(carboxymethyl)-1,4,7,10-tetraazacyclodo- decan in 40 ml Wasser gibt man 5,63 g (141 mmol) Natriumhydroxid. Hierzu tropft man eine Lösung aus 10,5 g (17,61 mmol) der Titelverbindung aus Beispiel 12b) gelöst in 50 ml n-Butanol/40 ml 2-Propanol und erwärmt die Lösung über Nacht auf 120°C im Autoklaven. Man dampft im Vakuum zur Trockne ein, nimmt den Rückstand in 200 ml Wasser auf und stellt mit 3 N Salzsäure auf pH 3. Dann wird 2 mal mit 300 ml n-Butanol extrahiert. Die vereinigten Butanolphasen werden im Vakuum zur Trockne eingeengt und der Rückstand durch RP-Chromatographie gereinigt (RP-18/Laufmittel: Gradient aus Wasser/n-Butanol/Acetonitril).
Ausbeute: 4,96 g (27 % d.Th.) eines farblosen, glasigen Feststoffes
Wassergehalt: 9,7 %

| Elementaranalyse (bezogen auf wasserfreie Substanz): | | | | |
|---|---|---|---|---|
| ber. | C 38,27 | H 4,17 | F 36,32 | N 5,95 |
| gef. | C 38,12 | H 4,20 | F 36,20 | N 5,81 |

### d) Gadolinium-Komplex von 10-[1-(2-Oxa-1H,1H,3H,3H,4H,4H-perfluoroctyl)-2-hydroxy-4-oxa-1H,2H,3H,3H,5H,5H,6H,6H-perfluordecyl]-1,4,7-tris(carboxymethyl)-1,4,7,10-tetraazacyclododecan

4,7 g (5 mmol) der Titelverbindung aus Beispiel 12c) werden in 30 ml Wasser/ 30 ml Ethanol/20 ml Chloroform gelöst und 0,90 g (2,5 mmol) Gadoliniumoxid zugegeben. Man rührt 3,5 Stunden bei 90°C im Autoklaven. Die Lösung wird filtriert und im Vakuum zur Trockne eingedampft.
Ausbeute: 5,89 g (quantitativ) eines farblosen, glasigen Feststoffes
Wassergehalt: 7,1 %

| Elementaranalyse (bezogen auf wasserfreie Substanz): | | | | | |
|---|---|---|---|---|---|
| ber. | C 32,88 | H 3,31 | F 31,21 | Gd 14,35 | N 5,11 |
| gef. | C 32,67 | H 3,45 | F 31,04 | Gd 14,18 | N 5,02 |

### Beispiel 13

### a) 1-Phenyl-2,6-dioxa-1H,1H,3H,3H,4H,5H,5H,7H,7H,8H,8H-perfluorhexadecan-4-ol

Zu 7,14 g (39,2 mmol) Glycerin-1-monobenzylether und 25 g (43,55 mmol) 1H,1H,2H,2H-Perfluordecyl-1-jodid in 100 ml Toluol gibt man 1 g (2,94 mmol) Tetrabutylammoniumhydrogensulfat und 15,6 g (390 mmol) feingepulvertes Natriumhydroxid. Man rührt 24 Stunden bei Raumtemperatur. Man trennt die organische Phase vom Feststoff ab und wäscht 2 mal mit je 5 %iger aqu. Salzsäure.
Die organische Phase wird über Magnesiumsulfat getrocknet und im Vakuum zur Trockne eingedampft. Der Rückstand wird an Kieselgel chromatographiert (Laufmittel: n-Hexan/Aceton= 15/1).
Ausbeute: 19,95 g (81 % d. Th.) eines farblosen Öls

| Elementaranalyse: | | | |
|---|---|---|---|
| ber. | C 38,23 | H 2,73 | F 51,40 |
| gef. | C 38,10 | H 2,89 | F 51,25 |

### b) 1-Phenyl-4-(decyloxy)-2,6-dioxa-1H,1H,3H,3H,4H,5H,5H,7H,7H,8H,8H-perfluorhexadecan

Zu 19,5 g (31,03 mmol) der Titelverbindung aus Beispiel 13a) gelöst in 100 ml Dimethylformamid gibt man portionsweise 1,12 g (37,24 mmol) Natriumhydrid (80 %ige Suspension in Mineralöl) und rührt 2 Stunden bei Raumtemperatur. Anschließend werden 8,24 g (37,24 mmol) n-Decylbromid zugegeben und über Nacht bei 50°C gerührt. Man gibt 150 ml Eiswasser zu und extrahiert 2 mal mit je 150 ml Essigsäureethylester. Die vereinigten organischen Phasen werden 2 mal mit je 150 ml Wasser gewaschen, über Magnesiumsulfat getrocknet und zur Trockne eingedampft. Der Rückstand wird an Kieselgel chromatographiert (Laufmittel: n-Hexan/Aceton= 20:1).
Ausbeute: 22,66 g (95 % d. Th.) eines wachsartigen Feststoffes

| Elementaranalyse: | | | |
|---|---|---|---|
| ber. | C 46,88 | H 4,85 | F 42,02 |
| gef. | C 46,64 | H 4,97 | F 41,87 |

### c) 2-(Decyloxy)-4-oxa-1H,1H,2H,3H,3H,5H,5H,6H,6H-perfluortetradecan-1-ol

20 g (26,02 mmol) der Titelverbindung aus Beispiel 13b) werden in 200 ml Isopropanol gelöst und 3 g Palladium-Katalysator (10 % Pd/C) zugegeben. Man hydriert über Nacht bei Raumtemperatur. Der Katalysator wird abfiltriert und das Filtrat im Vakuum zur Trockne eingeengt.
Ausbeute: 17,65 g (quantitativ) eines farblosen Feststoffes

| Elementaranalyse: | | | |
|---|---|---|---|
| ber. | C 40,72 | H 4,61 | F 47,60 |
| gef. | C 40,55 | H 4,76 | F 47,43 |

### d) 1,2-Epoxy-4-oxa-6-(decyloxy)-8-oxa-1H,1H,2H,3H,3H,5H,5H,6H,7H,7H,9H,9H,10H,10H-perfluoroctadecan

Zu einer Mischung aus 17 g (25,06 mmol) der Titelverbindung aus Beispiel 13c) und 2 g (5,89 mmol) Tetrabutylammoniumhydrogensulfat in 300 ml 60%iger aqu. Kalilauge/ 100 ml Toluol tropft man unter starkem Rühren bei 10°C 9,25 g (100 mmol) Epichlorhydrin zu und achtet darauf, daß die Temperatur der Reaktionslösung nicht über 20°C steigt. Man läßt 2 Stunden bei 15°C rühren und tropft anschließend wie oben beschrieben 4,63 g (50 mmol) Epichlorhydrin zu. Dann wird über Nacht bei Raumtemperatur gerührt. Man gibt 100 ml Toluol und Methyl-tert.-butylether zu und trennt die wassrige Phase ab. Diese wird 2 mal mit je 100 ml Toluol nachextrahiert. Die organischen Phasen werden vereinigt, über Magnesiumsulfat getrocknet und im Vakuum eingedampft. Der Rückstand wird an Kieselgel chromatographiert (Laufmittel: Dichlormethan/Hexan/Aceton= 20/10/1).
Ausbeute: 14,91 g (81 % d. Th.) eines farblosen Feststoffes

| Elementaranalyse: | | | |
|---|---|---|---|
| ber. | C 42,51 | H 4,80 | F 43,97 |
| gef. | C 42,37 | H 4,96 | F 43,68 |

### e) 10-[2-Hydroxy-4,8-dioxa-6-(decyloxy)-1H,1H,2H,3H,3H,5H,5H,6H,7H,7H,9H,9H,10H,10H-perfluoroctadecyl]-1,4,7-tris(carboxymethyl)-1,4,7,10-tetraazacyclododecan

Zu 6,6 g (19,06 mmol) 1,4,7-Tris(carboxymethyl)-1,4,7,10-tetraazacyclodo- decan in 60 ml Wasser gibt man 6,11 g (152,8 mmol) Natriumhydroxid. Hierzu tropft man eine Lösung aus 14 g (19,06 mmol) der Titelverbindung aus Beispiel 13d) gelöst in 80 ml n-Butanol/40 ml 2-Propanol und erwärmt die Lösung über Nacht auf 80°C im Autoklaven. Man dampft im Vakuum zur Trockne ein, nimmt den Rückstand in 200 ml Wasser auf und stellt mit 3 N Salzsäure auf pH 3. Dann wird 2 mal mit 300 ml n-Butanol extrahiert. Die vereinigten Butanolphasen werden im Vakuum zur Trockne eingeengt und der Rückstand durch RP-Chromatographie gereinigt (RP-18/Laufmittel: Gradient aus Wassern-Butanol/Acetonitril).
Ausbeute: 17,88 g (76 % d.Th.) eines glasigen Feststoffes
Wassergehalt: 12,5 %

| Elementaranalyse (bezogen auf wasserfreie Substanz): | | | | |
|---|---|---|---|---|
| ber. | C 44,49 | H 5,60 | F 29,91 | N 5,19 |
| gef. | C 44,31 | H 5,75 | F 29,70 | N 5,03 |

### f) Gadoliniumkomplex von 10-[2-Hydroxy-4,8-dioxa-6-(decyloxy)-1H,1H,2H,3H,3H,5H,5H,6H,7H,7H,9H,9H,10H,10H-perfluoroctadecyl]-1,4,7-tris(carboxymethyl)-1,4,7,10-tetraazacyclododecan

10 g (9,26 mmol) der Titelverbindung aus Beispiel 13e) werden in 30 ml Wasser/100 ml Ethanol/30 ml Chloroform gelöst und 1,68 g (4,63 mmol) Gadoliniumoxid zugegeben. Man rührt 3,5 Stunden bei 90°C im Autoklaven. Die Lösung wird filtriert und im Vakuum zur Trockne eingedampft.
Ausbeute: 12,39 g (quantitativ) eines farblosen, glasigen Feststoffes
Wassergehalt: 7,8 %

| Elementaranalyse (bezogen auf wasserfreie Substanz): | | | | | |
|---|---|---|---|---|---|
| ber. | C 38,93 | H 4,66 | F 26,17 | Gd 12,74 | N 4,54 |
| gef. | C 38,71 | H 4,82 | F 26,01 | Gd 12,55 | N 4,38 |

### Beispiel 14

### a) 1-Phenyl-2-oxa-4,4,4-tris(2-oxa-1H,1H,3H,3H,4H,4H-perfluordecyl)-butan

Zu 4,24 g (18,74 mmol) Pentaerythrit-monobenzylether und 40 g (93,7 mmol) 1H,1H,2H,2H-Perfluoroctyl-l-bromid in 150 ml Toluol gibt man 2 g (5,89 mmol) Tetrabutylammoniumhydrogensulfat und 22,48 g (562 mmol) feingepulvertes Natriumhydroxid. Man rührt 24 Stunden bei Raumtemperatur. Man trennt die organische Phase vom Feststoff ab und wäscht 2 mal mit je 5 %iger aqu. Salzsäure.

Die organische Phase wird über Magnesiumsulfat getrocknet und im Vakuum zur Trockne eingedampft. Der Rückstand wird an Kieselgel chromatographiert (Laufmittel: n-Hexan/Aceton= 25/1).
Ausbeute: 14,45 g (61 % d. Th. bezogen auf den Benzylether) eines farblosen,
wachsarten Feststoffes

| Elementaranalyse: | | | |
|---|---|---|---|
| ber. | C 34,19 | H 2,15 | F 58,59 |
| gef. | C 34,02 | H 2,31 | F 58,41 |

### b) 2,2,2-Tris(2-oxa-1H,1H,3H,3H,4H,4H-perfluordecyl)-ethan-1-ol

14 g (11,07 mmoL) der Titelverbindung aus Beispiel 14a) werden in 100 ml Isopropanol/100 ml Tetrahydrofuran gelöst und 3 g Palladium-Katalysator (10 % Pd/C) zugegeben. Man hydriert über Nacht bei Raumtemperatur. Der Katalysator wird abfiltriert und das Filtrat im Vakuum zur Trockne eingeengt.
Ausbeute: 13 g (quantitativ) eines farblosen Feststoffes

| Elementaranalyse: | | | |
|---|---|---|---|
| ber. | C 29,66 | H 1,80 | F 63,09 |
| gef. | C 29,45 | H 1,97 | F 62,91 |

### c) 1,2-Epoxy-4-oxa-6,6,6-tris(2-oxa-1H,1H,3H,3H,4H,4H-perfluordecyl)-hexan

Zu einer Mischung aus 12,5 g (10,64 mmol) der Titelverbindung aus Beispiel 14b) und 1 g (2,95 mmol) Tetrabutylammoniumhydrogensulfat in 150 ml 60%iger aqu. Kalilauge/ 50 ml Toluol tropft man unter starkem Rühren bei 10°C 3,94 g (42,57 mmol) Epichlorhydrin zu und achtet darauf, daß die Temperatur der Reaktionslösung nicht über 20°C steigt. Man läßt 2 Stunden bei 15°C rühren und tropft anschließend wie oben beschrieben 1,97 g (21,29 mmol) Epichlorhydrin zu. Dann wird über Nacht bei Raumtemperatur gerührt. Man gibt 100 ml Toluol und 100 ml Methyl-tert.-butylether zu und trennt die wässrige Phase ab. Diese wird 2 mal mit je 50 ml Toluol nachextrahiert. Die organischen Phasen werden vereinigt, über Magnesiumsulfat getrocknet und im Vakuum eingedampft. Der Rückstand wird an Kieselgel chromatographiert (Laufmittel: Dichlormethan/Hexan/Aceton= 20/10/1).
Ausbeute: 8,12 g (62 % d. Th.) eines farblosen Feststoffes

| Elementaranalyse: | | | |
|---|---|---|---|
| ber. | C 31,24 | H 2,05 | F 60,22 |
| gef. | C 31,09 | H 2,19 | F 60,10 |

### d) 10-[2-Hydroxy-4-oxa-6,6,6-tris(2-oxa-1H,1H,3H,3H,4H,4H-perfluordecyl)-hexyl]-1,4,7-tris(carboxymethyl)-1,4,7,10-tetraazacyclododecan

Zu 2,25 g (6,50 mmol) 1,4,7-Tris(carboxymethyl)-1,4,7,10-tetraazacyclodo- decan in 30 ml Wasser gibt man 2,08 g (52 mmol) Natriumhydroxid. Hierzu tropft man eine Lösung aus 8,0 g (6,50 mmol) der Titelverbindung aus Beispiel 14c) gelöst in 50 ml n-Butanol/30 ml 2-Propanol und erwärmt die Lösung über Nacht auf 100°C im Autoklaven. Man dampft im Vakuum zur Trockne ein, nimmt den Rückstand in 200 ml Wasser auf und stellt mit 3 N Salzsäure auf pH 3. Dann wird 2 mal mit 100 ml n-Butanol extrahiert. Die vereinigten Butanolphasen werden im Vakuum zur Trockne eingeengt und der Rückstand durch RP-Chromatographie gereinigt (RP-18/Laufmittel: Gradient aus Wasser/n-Butanol/Acetonitril).
Ausbeute: 7,79 g (67 % d.Th.) eines farblosen, glasigen Feststoffes
Wassergehalt: 11,9 %

| Elementaranalyse (bezogen auf wasserfreie Substanz): | | | | |
|---|---|---|---|---|
| ber. | C 35,06 | H 3,20 | F 47,02 | N 3,56 |
| gef. | C 34,90 | H 3,38 | F 46,86 | N 3,47 |

### e) Gadolinium-Komplex von 10-[2-Hydroxy-4-oxa-6,6,6-tris(2-oxa-1H,1H,3H,3H,4H,4H-perfluordecyl)-hexyl]-1,4,7-tris(carboxymethyl)-1,4,7,10-tetraazacyclododecan

7 g (4,44 mmol) der Titelverbindung aus Beispiel 14d) werden in 30 ml Wasser/50 ml Ethano1/50 ml Chloroform gelöst und 0,80 g (2,22 mmol) Gadoliniumoxid zugegeben. Man rührt 5 Stunden bei 90°C im Autoklaven. Die Lösung wird filtriert und im Vakuum zur Trockne eingedampft.
Ausbeute: 8,34 g (quantitativ) eines farblosen, glasigen Feststoffes
Wassergehalt: 8,1 %

| Elementaranalyse (bezogen auf wasserfreie Substanz): | | | | | |
|---|---|---|---|---|---|
| ber. | C 31,94 | H 2,74 | F 42,83 | Gd 9,09 | N 3,24 |
| gef. | C 31,74 | H 2,91 | F 42,67 | Gd 8,85 | N 3,15 |

### Beispiel 15

### a) 1,7-Bis[acetyl-(2-(N-ethyl-N-perfluoroctylsulfonylamino)]-1,4,7-triazaheptan

20 g (34,17 mmol) der Titelverbindung aus Beispiel 1b) und 4,33 g (37,59 mmol) N-Hydroxysuccinimid werden in 150 ml Dimethylformamid gelöst. Bei 0°C gibt man 7,76 g (37,59 mmol) Dicyclohexylcarbodiimid hinzu und rührt 3 Stunden bei Raumtemperatur. Man filtriert vom Dicyclohexylharnstoff ab und tropft das Filtrat zu einer Lösung aus 1,76 g (17,09 mmol) Diethylentriamin und 13,83 g (136,7 mmol) Triethylamin in 200 ml Dimethylformamid bei Raumtemperatur. Man rührt über Nacht bei Raumtemperatur. Es wird im Vakuum zur Trockne eingedampft und der Rückstand in 200 ml 5 %iger aqu. Soda-Lösung aufgenommen. Man extrahiert 2 mal mit je 150 ml Dichlormethan, trocknet die vereinigten organischen Phasen über Magnesiumsulfat und dampft im Vakuum zur Trockne ein. Der Rückstand wird an Kieselgel chromatographiert (Laufmittel: Dichlormethan/2-Propanol= 20/1).
Ausbeute: 16,5 g (78 % d. Th.) eines wachsartigen Feststoffes

| Elementaranalyse: | | | | | |
|---|---|---|---|---|---|
| ber. | C 27,17 | H 2,04 | F 52,19 | N 5,66 | S 5,18 |
| gef. | C 27,03 | H 2,17 | F 52,04 | N 5,49 | S 5,07 |

### b) 4-(3-Carboxy-propanoyl)-1,7-bis-{acetyl-[2-(N-ethyl-N-perfluoroctylsulfonylamino)]}-1,4,7-triazaheptan

Zu 16 g (12,93 mmol) der Titelverbindung aus Beispiel 15a) in 100 ml Methylenchlorid gibt man 3,92 g (38,78 mmol) Triethylamin und kühlt die Lösung auf 0°C ab. Dann gibt man 2,59 g (25,86 mmol) Bernsteinsäureanhydrid zu und rührt 3 Stunden bei 0°C, über Nacht bei Raumtemperatur. Man gibt 200 ml 5 %ige aqu. Salzsäure zu und schüttelt gut durch. Die organische Phase wird abgetrennt und über Magnesiumsulfat getrocknet. Man dampft im Vakuum zur Trockne ein und chromatographiert der Rückstand an Kieselgel (Laufmittel: Dichlormethan/2-Propanol= 15/1).
Ausbeute: 15,74 g (91 % d. Th.) eines farblosen Feststoffes

| Elementaranalyse: | | | | | |
|---|---|---|---|---|---|
| ber. | C 28,73 | H 2,19 | F 48,29 | N 5,24 | S 4,79 |
| gef. | C 28,58 | H 2,40 | F 48,17 | N 5,17 | S 4,65 |

### c) 10-[7-Hydroxy-5-aza-4-oxo-octansäure-N,N-bis(3-aza-4-oxo-6-aza-6-(perfluoroctylsulfonyl)-octyl)-amid]-1,4,7-tris(carboxymethyl)-1,4,7,10-tetraazacyclododecan

15 g (11,21 mmol) der Titelverbindung aus beispiel 15b) und 1,42 g (12,33 mmol) N-Hydroxysuccinimid werden in einer Mischung aus 80 ml Dimethylformamid/30 ml Chloroform gelöst. Bei 0°C gibt man 2,54 g (12,33 mmol) Dicyclohexylcarbodiimid zu und rührt 1 Stunde bei 0°C, anschließend 3 Stunden bei Raumtemperatur. Man kühlt erneut auf 0°C und gibt 4,05 g (40 mol) Triethylamin/50 ml 2-Propanol zu. Anschliessend werden 7,07 g (12,33 mmol) Gadolinium-Komplex von 10-[2-Hydroxy-3-amino-propyl]-1,4,7-tris(carboxymethyl)-1,4,7,10-tetraazacyclododecan gelöst in 30 ml Wasser zugegeben und 3 Stunden bei Raumtemperatur gerührt. Man dampft zur Trockne ein, nimmt den Rückstand in einer Mischung aus 100 ml Methanol/ 50 ml Chloroform auf und filtriert vom Dicyclohexylharnstoff ab. Das Filtrat wird zur Trockne eingedampft und durch RP-Chromatographie gereinigt (RP-18/Laufmittel:
Gradient aus Wassern-Propanol/Acetonitril).
Ausbeute: 17,76 g (78 % d. Th.) eines farblosen, glasigen Feststoffes
Wassergehalt: 6,8 %

| Elementaranalyse (bezogen auf wasserfreie Substanz): | | | | | | |
|---|---|---|---|---|---|---|
| ber. | C 31,08 | H 3,03 | F 34,12 | Gd 8,31 | N 7,40 | S 3,39 |
| gef. | C 30,89 | H 3,15 | F 34,01 | Gd 8,14 | N 7,25 | S 3,24 |

### Beispiel 16

### Gadoliniumkomplex von 1,4,7-Tris(carboxylatomethyl)-10-(2-hydroxy-19,19,20,20,21,21,22,22,23,23,24,24,25,25,26,26,26-heptadecafluor-4,7,10,13,16-penta-oxa-hexacosan)-1,4,7,10-tetraazacyclododecan

### a) 16,16,17,17,18,18,19,19,20,20,21,21,22,22,22-Heptadecafluor-3,6,9,12-tetraoxa-docosan-1-ol

Eine Mischung aus 20 g (32,35 mmol) 1-p-Toluolsulfonyloxy-1H,1H,2H,2H-perfluordecan [s. Beispiel 7a], 1g Tetrabutylammoniumhydrogensulfat, 62,83 g (323,5 mmol) Tetraethylenglykol, 300 ml Dichlormethan und 100 ml 50 %ige Natronlauge wird 24 Stunden intensiv bei ca. 5 °C gerührt. Man verdünnt dann mit 200 ml Dichlormethan, trennt die Phasen und wäscht die Dichlormethanphase mit Wasser. Die organische Phase wird über Magnesiumsulfat getrocknet und im Vakuum eingedampft. Man erhält 18,5 g der gewünschten Titelverbindung als hellgelbes Öl.

### b) 1,2-Epoxy-19,19,20,20,21,21,22,22,23,23,24,24,25,25,26,26,26-heptadecafluor-4,7,10,13,16-penta-oxa-hexacosan

Eine Mischung aus 17 g (26,5 mmol) 16,16,17,17,18,18,19,19,20,20,21,21,22,22,22-Heptadecafluor-3,6,9,12-tetra-oxadocosan-1-ol, 0,5 g Tetrabutylammoniumhydrogensulfat, 2,94 g Epichlorhydrin, 200 ml Dichlormethan und 50 ml 50 %ige Natronlauge wird 8 Stunden intensiv bei Raumtemperatur gerührt. Man trennt die Phasen, schüttelt die wäßrige Phase mit 100 ml Dichlormethan, vereinigt die organischen Phasen, schüttelt mit 50 ml Wasser, trocknet über Magnesiumsulfat und dampft im Vakuum ein. Den Rückstand chromatographiert man an Kieselgel mit Hexan / 5 - 50 % Ethylacetat und erhält 12,92 g der Titelverbindung als Öl.

| Elementaranalyse: | | | |
|---|---|---|---|
| ber. | C 36,22 | H 3,62 | F 46,38 |
| gef. | C 36,00 | H 3,78 | F 46,20 |

### c) 1,4,7-Tris(carboxylatomethyl)-10-(2-hydroxy-19,19,20,20,21,21,22,22,23,23,24,24,25,25,26,26,26-heptadecafluor-4,7,10,13,16-penta-oxa-hexacosan)-1,4,7,10-tetraazacyclododecan

Zu einer Lösung von 6 g (17,3 mmol) 1,4,7-(Triscarboxylatomethyl)-1,4,7,10-tetraazacyclododecan und 4 g Natriumhydroxid in 30 ml Wasser gibt man eine Lösung von 12,05 g (17,3 mmol) 1,2-Epoxy-19,19,20,20,21,21,22,22,23,23,24,24,25,25,26,26,26-heptadecafluor-4,7,10,13,16-penta-oxa-hexacosan in 50 ml Tetrahydrofuran. Man rührt über Nacht bei 70 °C, dampft im Vakuum dann weitgehend ein, nimmt den Rückstand in 150 ml Wasser auf und stellt mit 6N Salzsäure auf pH3 und extrahiert mehrmals mit n-Butanol. Die vereinigten Extrakte werden im Vakuum eingedampft, und der Rückstand wird durch Chromatographie an RP-18 mit einem Gradienten aus Wasser/n-Butanol/Acetonitril gereinigt. Man erhält 13,71 g der Titelverbindung als gelbes viskoses Öl.

| Elementaranalyse | | | | |
|---|---|---|---|---|
| ber. | C 40,31 | H 4,93 | F 30,97 | N 5,37 |
| gef. | C 40,08 | H 5,21 | F 30,77 | N 5,29 |

### d) Gadoliniumkomplex von 1,4,7-Tris(carboxylatomethyl)-10-(2-hydroxy-19,19,20,20,21,21,22,22,23,23,24,24,25,25,26,26,26-heptadecafluor-4,7,10,13,16-penta-oxa-hexacosan)-1,4,7,10-tetraazacyclododecan

Man versetzt eine Mischung aus 5 g (4,79 mmol) 1,4,7-Tris(carboxylatomethyl)-10-(2-hydroxy-19,19,20,20,21,21,22,22,23,23,24,24,25,25,26,26,26-heptadecafluor-4,7,10,13,16-penta-oxa-hexacosan)-1,4,7,10-tetraazacyclododecan, 50 ml Wasser und 30 ml Ethanol mit 869 mg (2,397 mmol) Gadoliniumoxid und erhitzt 5 Stunden unter Rückfluß. Man filtriert die heiße Lösung und dampft im Vakuum ein. Man erhält 5,60 g der Titelverbindung als glasigen festen Stoff mit einem Wassergehalt von 4,1 %

| Elementaranalyse (bezogen auf wasserfreie Substanz): | | | | | |
|---|---|---|---|---|---|
| ber. | C 35,12 | H 4,04 | F 26,98 | Gd 13,14 | N 4,68 |
| gef. | C 34,90 | H4,38 | F 26,70 | Gd 13,10 | N4,62 |

### Beispiel 17

### Gadoliniumkomplex von 1,4,7-Tris(carboxylatomethyl)-10-(4-aza-2-hydroxy-26,26,26,25,25,24,24,23,23,22,22,21,21,20,20,19,19-heptadecafluor-5-oxo-16-thia-hexacosyl)-1,4,7,10-tetraazacyclododecan

### a) 22,22,22,21,21,20,20,19,19,18,18,17,17,16,16,15,15,-Heptadecafluor-12-thiadocosansäure

Man versetzt eine Lösung von 10 g (37,71 mmol) 11-Bromundecansäure in 150 ml Dichlormethan mit 11,43 g Triethylamin und 18,11 g (37,71 mmol) 1H,1H,2H,2H-Perfluordecylmercaptan und rührt über Nacht bei Raumtemperatur. Man extrahiert die Lösung mehrmals mit 2N Salzsäure, wäscht mit Kochsalzlösung, trocknet über Magnesiumsulfat und dampft im Vakuum ein. Man erhält 21,5 g der Titelverbindung als gelbes Öl.

| Elementaranalyse: | | | | |
|---|---|---|---|---|
| ber. | C 37,96 | H 3,79 | F 48,61 | S 4,83 |
| gef. | C 38,30 | H 4,01 | F 48,40 | S 5,20 |

### b) Gadoliniumkomplex von 1,4,7-Tris(carboxylatomethyl)-10-(4-aza-2-hydroxy-26,26,26,25,25,24,24,23,23,22,22,21,21,20,20,19,19-heptadecafluor-5-oxo-16-thia-hexacosyl)-1,4,7,10-tetraazacyclododecan

5 g (7,52 mmol) der Titelverbindung aus Beispiel 17a) und 0,95 g N-Hydroxysuccinimid werden in einer Mischung aus 25 ml Dimethylformamid und 15 ml Chloroform gelöst. Bei 0 °C gibt man 1,71 g Dicyclohexylcarbodiimid zu und rührt 1 Stunde bei 0 °C, dann 3 Stunden bei Raumtemperatur. Man kühlt dann wieder auf 0 °C und versetzt mit 3 ml Triethylamin und 20 ml n-Propano]. Anschließend werden 4,75 g (8,27 mmol) des Gadolinium-Komplexes von 10-(3-Amino-2-hydroxy-propyl)-1,4,7-tris(carboxylatomethyl)-1,4,7,10-tetraazacyclododecan gelöst in 25 ml Wasser zugegeben und 3 Stunden bei 20 °C gerührt. Man dampft zur Trockne ein, nimmt den Rückstand in einer Mischung aus 55 ml Methanol und 20 ml Chloroform auf und filtriert vom Dicyclohexylharnstoff ab. Das Filtrat dampft man zur Trockne ein und reinigt durch Chromatographie an RP-18 mit einem Gradienten aus Wasser/n-Propanol/Acetonitril. Man erhält 6,15 g der Titelverbindung als glasigen Feststoff, mit einem Wassergehalt von 2,3 %.

| Elementaranalyse (berechnet auf wasserfreie Substanz): | | | | | | |
|---|---|---|---|---|---|---|
| ber. | C 37,41 | H 4,38 | F 26,47 | Gd 12,89 | N 5,74 | S 2,63 |
| gef. | C 37,08 | H 4,60 | F 26,30 | Gd 12,68 | N 5,91 | S 2,49 |

### Beispiel 18

### Gadolinium-Komplex von 1,4,7-Tris(carboxylatomethyl)-10-[1-(1,2-dihydroxyethyl)3-oxa-6,6,7,7,8,8,9,9,10,10,11,11,11-tridecafluor]undecan-1,4,7,10-tetraazacyclodo decan

### a) 1-p-Toluolsulfonyloxy-1H,1H,2H,2H-perfluoroctan

Zu einer Lösung von 25 g (68,7 mmol) 1H,1H,2H,2H-Perfluoroctan-1-ol in 300 ml Dichlormethan gibt man bei 0 °C 20 ml Pyridin und trägt unter Rühren in Portionen 13,49 g (70,76 mmol) p-Toluolsulfonsäurechlorid ein. Man rührt noch 3 Stunden bei 0 °C, zieht bei Raumtemperatur im Vakuum das Dichlormethan ab.

Die zurückbleibende Pyridinlösung versetzt man mit Eiswasser, wobei das gewünschte Produkt ausfällt. Mandekantiert und löst den Rückstand in Dichlormethan, wäscht die Lösung mit Wasser, trocknet über Magnesiumsulfat und dampft im Vakuum ein. Der Rückstand wird durch Chromatographie an Kieselgel mit Hexan / 5 - 40 % Ethylacetat gereinigt. Man erhält 29,2 g der Titelverbindung als zähen Schaum.

| Elementaranalyse: | | | | |
|---|---|---|---|---|
| ber. | C 34,76 | H 2,14 | F 47,65 | S 6,19 |
| gef. | C 34,98 | H 2,38 | F 47,39 | S 6,42 |

### b) 1,4,7-Tris(Benzyloxycarbonyl)-10-[1-(2,2-dimethyl-1,3-dioxolan-4-yl)-6,6,7,7,8,8,9,9,10,10,11,11,11-tridecafluor-3-oxa]-undecan-1,4,7,10-tetraazacyclododecan

Zu 7,33 g (10mmol) 1,4,7-Tris(Benzyloxycarbonyl)-10-[2-hydroxy-1-(2,2-dimethyl-1,3-dioxolan-4-yl)]-ethyl-1,4,7,10-tetraazacyclododecan [J. Mag. Res. Imag. 5: 7-10, (1955)] gelöst in 100 ml Dichlormethan gibt man nacheinander 20 ml 50 %ige Natronlauge, 0,5 g Tetrabutylammoniumhydrogensulfat und 5,18 g (10 mmol) 1-p-Toluol-sulfonyloxy-1H,1H,2H,2H-perfluoroctan [s. Beispiel 18a)] und rührt die Mischung intensiv über Nacht bei Raumtemperatur. Man trennt die Phasen, wäscht die organische Phase mehrmals mit Wasser, trocknet über Magnesiumsulfat und dampft im Vakuum ein. Den Rückstand reinigt man durch Chromatographie an Kieselgel mit Dichlormethan / 1 - 10 % Ethanol. Man erhält 8,02 g der Titelverbindung als zähes Öl.

| Elementaranalyse: | | | | |
|---|---|---|---|---|
| ber. | C 53,01 | H 5,02 | F 23,19 | N 5,26 |
| gef. | C 53,30 | H 5,39 | F 23,01 | N 5,40 |

### c) 1-[1-(2,2-dimethyl-1,3-dioxolan-4-yl)-6,6,7,7,8,8,9,9,10,10,11,11,11-tridecafluor-3-oxa]-undecan-1,4,7,10-tetraazacyclododecan

Eine Lösung von 7 g (6,57 mmol) 1,4,7-Tris(Benzyloxycarbonyl)-10-[1-(2,2-dimethyl-1,3-dioxolan-4-yl)-6,6,7,7,8,8,9,9,10,10,11,11,11-tridecafluor-3-oxa]-undecan-1,4,7,10-tetraazacyclododecan in 100 ml Isopropylalkohol versetzt man mit 0,7 g Palladium auf Kohle (10 %ig) und schüttelt 3 Stunden unter einer Wasserstoffatmosphäre. Man filtriert vom Katalysator und dampft die Lösung im Vakuum ein. Man erhält 4,20 g der Titelverbindung als glasigen Schaum.

| Elementaranalyse: | | | | |
|---|---|---|---|---|
| ber. | C 41,70 | H 5,32 | F 37,28 | N 8,46 |
| gef. | C 41,61 | H 5,57 | F 37,10 | N 8,59 |

### d) 1,4,7-Tris(carboxylatomethyl)-10-[1-(1,2-dihydroxy-ethyl)-3-oxa-6,6,7,7,8,8,9,9,10,10,11,11,11-tridecafluor]undecan-1,4,7,10-tetraazacyclododecan

Man löst 3,36 g (24,15 mmol) Bromessigsäure in 50 ml Wasser und versetzt mit 6N Natronlauge bis pH7. Bei 40 °C tropft man unter Rühren gleichzeitig eine Lösung von 4 g (6,04 mmol) 1-[1-(2,2-dimethyl-1,3-dioxolan-4-yl)-6,6,7,7,8,8,9,9,10,10,11,11,11-tridecafluor-3-oxa]-undecan-1,4,7,10-tetraazacyclododecan, gelöst in 20 ml Isopropylalkohol, und soviel 6N Natronlauge zu, daß der pH bei 9-10 gehalten wird. Anschließend versetzt man mit halbkonzentrierter Salzsäure bis zu einem pH 1 und rührt weitere 3 Stunden bei 60 °C. Man kühlt auf Raumtemperatur ab und extrahiert die Lösung mehrmals mit n-Butanol. Der organische Extrakt wird eingedampft und der Rückstand durch Chromatographie an RP-18 mit einem Gradienten aus Wasser / n-Butanol / Acetonitril gereinigt. Man erhält 3,85 g der Titelverbindung als gelbes Öl mit einem Wassergehalt von 3,9 %.

| Elementaranalyse (bezogen auf wasserfreie Substanz): | | | | |
|---|---|---|---|---|
| ber. | C 39,20 | H 4,68 | F 31,00 | N 7,03 |
| gef. | C 39,08 | H 4,98 | F 30,72 | N 7,29 |

### e) Gadolinium-Komplex von 1,4,7-Tris(carboxylatomethyl)-10-[1-(1,2-dihydroxy-ethyl)-3-oxa-6,6,7,7,8,8,9,9,10,10,11,11,11-tridecafluor]undecan-1,4,7,10-tetraazacyclododecan

Man versetzt eine Mischung aus 1,59 g (2 mmol) 1,4,7-Tris(carboxylatomethyl)-10-[ 1-(1,2-dihydroxy-ethyl)-3-oxa-6,6,7,7,8,8,9,9,10,10,11,11,11-tridecafluor] undecan-1,4,7,10-tetraazacyclododecan, 25 ml Wasser und 15 ml Ethanol mit 363 mg (1 mmol) Gadoliniumoxid und erhitzt 5 Stunden unter Rückfluß. Man filtriert die heiße Lösung, dampft im Vakuum ein und erhält 1,85 g der Titelverbindung als glasigen festen Stoff mit einem Wassergehalt von 4,2%.

| Elementaranalyse (bezogen auf wasserfreie Substanz): | | | | | |
|---|---|---|---|---|---|
| ber. | C 32,84 | H 3,60 | F 25,98 | Gd 16,54 | N 5,89 |
| gef. | C 32,53 | H 3,71 | F 25,72 | Gd 16,39 | N 5,93 |

### Beispiel 19

### Gadolinium-Komplex von 1,4,7-Tris(carboxylatomethyl)-10-{2-hydroxy-4-oxa-4-[4-(2H,2H,3H,3H-1-oxa-perfluorundec-1-yl)]-phenyl}-but-1-yl-1,4,7,10-tetraazacyclododecan

### a) 1-Hydroxy-4-(2H,2H,3H,3H-1-oxa-perfluorundec-1-yl)-benzol

5 g (45,41 mmol) Hydrochinon werden mit 100 ml Aceton versetzt und unter Rühren nacheinander mit 13,8 g Kaliumcarbonat und 14,04 g (22,7 mmol) 1-p-Toluolsulfonyloxy-1H,1H,2H,2H-perfluordecan [s. Beispiel 7a)] versetzt. Man erhitzt 6 Stunden unter Rückfluß, engt dann im Vakuum weitgehend ein, verdünnt mit 200 ml Wasser, stellt mit Zitronensäure auf pH3 und extrahiert mehrmals mit Dichlormethan. Der organische Extrakt wird über Magnesiumsulfat getrocknet und im Vakuum eingedampft. Der Rückstand wird durch Chromatographie an Kieselgel mit Hexan / 5 - 30 % Ethylacetat gereinigt. Man erhält 8,20 g der gewünschten Titelverbindung als zähes Öl.

| Elementaranalyse: | | | |
|---|---|---|---|
| ber. | C 34,55 | H 1,63 | F 58,07 |
| gef. | C 34,31 | H 1,79 | F 58,01 |

### b) 1-(3,4-Epoxy-1-oxa-but-1-yl)-4-(2H,2H,3H,3H-1-oxa-perfluorundec-1-yl)-benzol

Eine Mischung aus 8 g (14,38 mmol) 1-Hydroxy-4-(2H,2H,3H,3H-l-oxa-perfluorundec-1-yl)-benzol, 0,4 g Tetrabutylammoniumhydrogensulfat, 1,60 g (17,26 mmol) Epichlorhydrin, 150 ml Dichlormethan und 30 ml 50 %ige Natronlauge wird 30 Minuten im Eisbad, dann 5 Stunden bei Raumtemperatur intensiv gerührt. Man trennt die Phasen, wäscht die organische Phase mit Wasser, trocknet über Magnesiumsulfat und dampft im Vakuum ein. Den Rückstand reinigt man durch Chromatographie an Kieselgel mit Hexan / 5 - 30 % Ethylacetat und erhält 6,60 g der Titelverbindung als zähes Öl.

| Elementaranalyse: | | | |
|---|---|---|---|
| ber. | C 37,27 | H 2,41 | F 52,75 |
| gef. | C 37,10 | H 2,66 | F 52,80 |

### c) 1,4,7-Tris(carboxylatomethyl)-10-{2-hydroxy-4-oxa-4-[4-(2H,2H,3H,3H-1-oxa-perfluorundec-1-yl)]-phenyl}-but-1-yl-1,4,7,10-tetraazacyclododecan

Zu einer Lösung von 3,46 g (10 mmol) 1,4,7-Tris(carboxylatomethyl)-1,4,7,10-tetraazacyclododecan und 2,5 g Natriumhydroxid in 25 ml Wasser gibt man eine Lösung von 6,12 g (10 mmol) 1-(3,4-Epoxy-1-oxa-but-1-yl)-4-(2H,2H,3H,3H-1-oxa-perfluorundec-1-yl)-benzol in 25 ml Tetrahydrofuran und erhitzt 24 Stunden unter Rückfluß, dampft dann im Vakuum weitgehend ein, löst den Rückstand in 100 ml Wasser, stellt mit 6N Salzsäure auf pH 3 und extrahiert mehrmals mit n-Butanol. Die vereinigten Extrakte werden im Vakuum eingedampft. Der Rückstand wird durch Chromatographie an RP-18 mit einem Gradienten aus Wasser/ n-Butanol/ Acetonitril gereinigt. Man erhält 6,71 g der Titelverbindung als viskoses Öl.

| Elementaranalyse: | | | | |
|---|---|---|---|---|
| ber. | C 41,35 | H 4,10 | F 33,69 | N 5,84 |
| gef. | C 41,58 | H 4,38 | F 33,50 | N 5,91 |

### d) Gadolinium-Komplex von 1,4,7-Tris(carboxylatomethyl)-10-{2-hydroxy-4-oxa-4-[4-(2H,2H,3H,3H-1-oxa-perfluorundec-1-yl)]-phenyl}-but-1-yl-1,4,7,10-tetraazacyclododecan

Man versetzt eine Mischung von 4,79 g (5 mmol) 1,4,7-Tris(carboxylatomethyl)-10-{2-hydroxy-4-oxa-4-[4-(2H,2H,3H,3H-1-oxa-perfluorundec-1-yl)]-phenyl}-but-1-yl-1,4,7,10-tetraazacyclododecan, 50 ml Wasser und 30 ml Ethanol mit 906 mg (2,5 mmol) Gadoliniumoxid und erhitzt 5 Stunden unter Rückfluß. Man filtriert die heiße Lösung und dampft im Vakuum ein. Man erhält 5,50 g der Titelverbindung als glasigen festen Stoff mit einem Wassergehalt von 4,9 %.

| Elementaranalyse (bezogen auf wasserfreie Substanz): | | | | | |
|---|---|---|---|---|---|
| ber. | C 35,62 | H 3,26 | F 29.02 | Gd 14,13 | N 5,03 |
| gef. | C 35,40 | H 3,50 | F 28,81 | Gd 14,01 | N 5,18 |

### Beispiel 20

### Gadoliniumkomplex, Dinatriumsalz von 3,9-Bis(carboxymethyl)-6-[(1-carboxy)-1H,2H,2H,4H,4H,5H,5H-3-oxa-perfluortridecyl]-3,6,9-triazaundecandisäure

### a) N-t-Butoxycarbonyl-serin-( 1H,1H,2H,2H-perfluordecyl)-ether-benzylester

In eine Lösung von 2,953 g (10 mmol) N-t-Butyloxycarbonyl-serinbenzylester (Kaufware Bachem) in 30 ml trockenem Dimethylformamid werden 300 mg (10 mmol) Natriumhydrid (80 % in Öl) anteilweise gegeben. Nach erfolgter Auflösung versetzt man mit 6,072 g (10 mmol) des unter 7a) hergestellten Tosylats. Man rührt 12 Stunden bei Raumtemperatur. Dann gießt man in 500 ml Eiswasser, nimmt das Produkt in Dichlormethan auf, wäscht die organische Lösung mit Wasser, trocknet über Natriumsulfat und engt zur Trockne ein. Der Rückstand wird durch Chromatographie an Kieselgel gereinigt. Als Elutionsmittel dient ein Gemisch aus Dichlormethan mit steigendem Methanolzusatz.
Die Titelverbindung wird als Sirup erhalten.
Ausbeute: 5,902 g (79,6 % d. Th.)

| Elementaranalyse: | | | | |
|---|---|---|---|---|
| ber. | C 40,50 | H 3,26 | F 43,56 | N 1,89 |
| gef. | C 40,64 | H 3,37 | F 43,49 | N 1,83 |

### b) Serin-(1H,1H,2H,2H-perfluordecyl)-ether-benzylester (als Salz der Trifluoressigsäure)

In 50 ml eines Gemisches aus Trifluoressigsäure und Dichlormethan im Verhältnis 2:1 werden 7,414 g (10 mmol) des unter 20a) hergestellten N-geschützten Verbindung gelöst und über Nacht bei Raumtemperatur gerührt. Man engt zur Trockne ein und entfernt Reste von Trifluoressigsäure durch Kodestillation mit Ethanol. Die Titelverbindung wird als Salz der Trifluoressigsäure isoliert.
Ausbeute: 7,418 g (98,2 % d. Th.)

| Elementaranalyse: | | | | |
|---|---|---|---|---|
| ber.: | C 34,98 | H 2,27 | F 50,30 | N 1,85 |
| gef.: | C 34,89 | H 2,31 | F 50,39 | N 1,80 |

### c) 3,9-Bis(t-butoxycarbonylmethyl)-6-[(1-benzyloxycarbonyl)-1H,2H,2H,4H,4H,5H,5H-3-oxa-perfluortridecyl)-3,6,9-triazaundecandisäure-di(t-butyl)-ester

In ein Gemisch aus 10 ml Acetonitril und 20 ml Phosphatpuffer von pH-Wert 8,0 werden 3,777 g (5 mmol) des unter 20b) hergestellten Amin-Trifluoracetates und 3,523 g (10 mmol) N,N-Bis(t-butyloxycarbonylmethyl)-2-(bromethyl)-amin gegeben und 2 Stunden intensiv bei Raumtemperatur gerührt. Dann trennt man die Pufferphase ab, extrahiert sie mit 10 ml Acetonitril und gibt dieses zur organischen Phase. Nach Zugabe von 20 ml frischem Puffer rührt man noch 20 Stunden bei Raumtemperatur. Man trennt die organische Phase ab, engt sie ein und verteilt den Rückstand zwischen 100 ml Phosphatpuffer (pH 8,0) und 100 ml Essigester. Die organische Phase wird mit gesättigter Kochsalzlösung gewaschen, über Natriumsulfat getrocknet und eingeengt. Die Titelverbindung wird durch Chromatographie an Kieselgel gereinigt. Als Elutionsmittel dient Dichlormethan mit steigendem Zusatz von Methanol. Die Titelverbindung wird als glasartiger Feststoff erhalten.
Ausbeute: 3,162 g (53,4 % d. Th.)

| Elementaranalyse: | | | | |
|---|---|---|---|---|
| ber.: | C 48,69 | H 5,62 | F 27,28 | N 3,55 |
| gef.: | C 48,82 | H 5,72 | F 27,37 | N 3,50 |

### d) 3,9-Bis(carboxymethyl)-6-[(1-carboxy)-1H,2H,2H,4H,4H,5H,5H-3-oxa-perfluor-tridecyl]-3,6,9-triazaundecandisäure

In eine Mischung von 25 ml Trifluoressigsäure/Dichlormethan im Verhältnis 2:1 werden 5,920 g (5 mmol) der unter 20c) hergestellten Verbindung gegeben. Man läßt über Nacht bei Raumtemperatur rühren, engt dann zur Trockne ein, nimmt den Rückstand in 100 ml 3 N Salzsäure auf, erhitzt 3 Stunden unter Rückfluß, engt dann im Vakuum zur Trockne ein und nimmt in 160 ml eines Gemisches aus Wasser, Ethanol und Chloroform (10:5:1) auf. Die Lösung wird durch Zugabe von Ionenaustauscher IRA 67 (OH⁻-Form) auf einen konstanten pH-Wert (etwa 3) eingestellt. Man saugt rasch ab, engt ein und erhält die Titelverbindung als glasigen Feststoff.
Ausbeute: 3,080 g (71,3 % d. Th.)
Wassergehalt: 11,3 %

| Elementaranalyse (auf wasserfreie Substanz bezogen): | | | | |
|---|---|---|---|---|
| ber. | C 34,53 | H 3,25 | F 37,15 | N 4,83 |
| gef. | C 34,41 | H 3,32 | F 37,29 | N 4,90 |

### e) Gadoliniumkomplex, Dinatriumsalz von 3,9-Bis(carboxymethyl)-6-[(1-carboxy)-1H,2H,2H,4H,4H,5H,5H-3-oxa-perfluortridecyl]-3,6,9-triazaundecandisäure

In einem Gemisch aus 60 ml destilliertem Wasser und 30 ml Ethanol werden 2,941 g (3,0 mmol, berechnet auf 11,3 % Wassergehalt) der unter 20d) hergestellten Säure gegeben. Unter Rühren und Erwärmen auf 50 °C werden anteilweise 543,8 mg (1,5 mmol) Gadoliniumoxid zugegeben. Nach beendeter Zugabe wird bis zur Lösung gerührt. Man stellt dann den pH-Wert der Lösung durch Zugabe von Natrionlauge auf 7,2 ein. Die Lösung wird dann eingeengt, wobei starkes Schäumen zu beobachten ist. Der Rückstand wird mit destilliertem Wasser kodestilliert. Die Titelverbindung wird als glasiger Feststoff erhalten.
Ausbeute: 3,489 g (quantitativ)
Wassergehalt: 8,2 %.

| Elementaranalyse (auf wasserfreie Substanz bezogen): | | | | | | |
|---|---|---|---|---|---|---|
| ber. | C 28,12 | H 2,17 | F 30,25 | Gd 14,73 | N3,94 | Na 4,31 |
| gef. | C 28,25 | H 2,26 | F 30,40 | Gd 14,85 | N 3,99 | Na 4,38 |

### Beispiel 21

### Gadoliniumkomplex, Mononatriumsalz von 3,6,9-Tris(carboxymethyl)-3,6,9-triazaundecandisäure-mono-N-{ethyl-2-amino-[carbonylmethyl-amino-(N-ethyl-N-perfluoroctylsulfonyl)] }-amid

### a) 3,6,9-Tris(carboxylatomethyl)-3,6,9-triazaundecandisäure-mono-N-{ethyl-2-amino-[carbonylmethyl-amino-(N-ethyl-N-perfluoroctylsulfonyl)]}-amid

In 200 ml eines Gemisches aus Dimethylformamid und Dichlormethan im Verhältnis 4:1 werden 17,87g (50 mmol) Diethylentriaminpentaessigsaurebisanhydrid suspeniert und anteilweise unter starkem Rühren mit dem Gemisch aus 3,137 g (5 mmol) [N-(2-Aminoethyl)-N-perfluoroctylsulfonyl]-aminoessigsäure-N-(2-aminoethyl)-amid und 6,50 g (64,2 mmol) Triethylamin versetzt. Man läßt 5 Stunden nachrühren, engt zur Trockne ein, versetzt mit 300 ml Eiswasser und stellt den pH-Wert des Ansatzes mit 3 N Salzsäure auf etwa 3 ein. Man extrahiert zweimal mit je 200 ml n Butanol, vereinigt die organischen Lösungen und engt sie ein. Das Produkt wird durch Chromatographie an Kieselgel RP-18 gereinigt. Als Elutionsmittel dienen Wasser und Tetrahydrofuran. Die Titelverbindung wird als glasiger Feststoff erhalten.
Ausbeute: 2,722 g (54,3 % d. Th.)
Wassergehalt: 9,7 %

| Elementaranalyse (bezogen auf wasserfreie Substanz): | | | | | |
|---|---|---|---|---|---|
| ber. | C 33,54 | H 3,52 | F 32,21 | N 8,38 | S 3,20 |
| gef. | C 33,65 | H 3,60 | F 32,14 | N 8,51 | S 3,29 |

### b) Gadoliniumkomplex, Mononatriumsalz von 3,6,9-Tris(carboxymethyl)-3,6,9-triazaundecandisäure-mono-N-{ethyl-2-amino-[carbonylmethyl-amino-(N-ethyl-N-perfluoroctylsulfonyl)] }-amid

In 90 ml eines Gemisches aus destilliertem Wasser und Ethanol (2:1) werden 3,259 g (3 mmol, berechnet auf 9,7% Wasser) der unter 21a) hergestellten Verbindung gegeben. Unter Rühren werden anteilweise 543,8 mg (1,5 mmol) Gadoliniumoxid zugefügt. Man rührt bis zur Lösung, stellt dann durch Zugabe von Natronlauge den pH-Wert der Lösung auf 7,2 ein und engt ein, wobei starkes Schäumen auftritt. Der Rückstand wird mit destilliertem Wasser kodestilliert. Die Titelverbindung wird als glasartiger Feststoff erhalten.
Ausbeute: 3,861 g (quantitativ)
Wassergehalt: 8,4 %

| Die Elementaranalyse ist auf wasserfreie Substanz berechnet. | | | | | | | |
|---|---|---|---|---|---|---|---|
| ber. | C 28,53 | H 2,65 | F 27,40 | Gd 13,34 | N 7,13 | Na 1,95 | S 2,72 |
| gef. | C 28,61 | H 2,68 | F 27,48 | Gd 13,40 | N 7,08 | Na 1,99 | S 2,76 |

### Beispiel 22

### Gadoliniumkomplex, Mononatriumsaiz von 3,9-Bis(carboxymethyl)-6-1H,1H,4H,4H,5H,5H,8H,8H,10H,10H,11H,11H-2,7-dioxo-3,6-diaza-9-oxa-perfluormonodecyl)-3,6,9-triazaundecandisäure

### a) Glycolsäure-(1H,1H,2H,2H-perfluordecyl)-ether-N-(2-aminoethyl)-amid

In 80 ml Dichlormethan werden 10,44 g (20 mmol) der Verbindung 2b) gelöst und mit 2,30 g (20 mmol) N-Hydroxysuccinimid sowie 4,13 g (20 mmol) Dicyclohexylcarbodiimid versetzt. Man läßt über Nacht rühren, filtriert vom Dicyclohexylharnstoff ab und rührt das Filtrat in eine Lösung von 60,1 g (1000 mmol) Ethylendiamin in 100 ml Dichlormethan. Man läßt über Nacht rühren, versetzt mit 1,5 l Wasser und trennt die organische Phase ab. Man wäscht die Dichlormethanlösung mit Wasser, trocknet über Natriumsulfat, engt zur Trockne ein und reinigt den Rückstand durch Chromatographie an Kieselgel. Als Elutionsmittel dient ein Gemisch aus Dichlormethan mit steigendem Isopropanolzusatz.
Ausbeute: 9,615 g (85,2 % d. Th.)

| Elementaranalyse: | | | | |
|---|---|---|---|---|
| ber. | C 29,80 | H 2,32 | F 57,24 | N 4,96 |
| gef. | C 29,96 | H 2,37 | F 57,12 | N 5,01 |

### b) Glycolsäure-(1H,1H,2H,2H-perfluordecyl)-ether-N-[ethyl-2-(benzyloxycarbonylaminomethylcarbonylamino)]-amid

In 15 ml Dichlormethan werden 2,092 g (10 mmol) Benzyloxycarbonylglycin gelöst und mit 1,151 g (10 mmol) N-Hydroxysuccinimid sowie 2,063 g (10 mmol) Dicyclohexylcarbodiimid versetzt. Man läßt über Nacht rühren, filtriert vom Dicyclohexylharnstoff ab und engt zur Trockne ein. Der Rückstand wird an Kieselgel durch Säulenchromatographie gereinigt. Als Elutionsmittel dient ein Gemisch aus Dichlormethan und Ethanol. Die Titelverbindung wird als glasiger Feststoff erhalten.
Ausbeute: 6,905 g (91,4 % d. Th.)

| Elementaranalyse: | | | | |
|---|---|---|---|---|
| ber. | C 38,16 | H 2,94 | F 42,75 | N 5,56 |
| gef. | C 38,28 | H 2,98 | F 42,52 | N 5,50 |

### c) Glycolsaure-(1H,1H,2H,2H-perfluordecyl)-ether-N-[ethyl-(2-aminomethylcarboxylamino)-amid

In 100 ml eines Gemisches aus Tetrahydrofuran und Ethanol im Verhältnis 2:1 werden 3,777 g (5 mmol) der unter 22b) hergestellten Verbindung in Gegenwart von 0,2 g Pearlman-Katalysator (Pd 20 % /C) bis zur Aufnahme von 112 ml Wasserstoff hydriert. Man saugt vom Katalysator ab, wäscht mit Ethanol gut nach und engt zur Trockne ein. Die Titelverbindung wird als glasartiger Feststoff erhalten.
Ausbeute: 3,097 g (99,7 % d. Th.)

| Elementaranalyse: | | | | |
|---|---|---|---|---|
| ber. | C 30,93 | H 2,60 | F 51,98 | N 6,76 |
| gef. | C 30,87 | H 2,64 | F 52,11 | N 6,82 |

### d) 3,9-Bis(t-butyloxycarbonylmethyl)-6-(1H,1H,4H,4H,5H,5H,8H,8H,10H,10H,-11H,11H,-2,7-dioxo-3,6-diaza-9-oxa-perfluornonadecyl)3,6,9-triazaundecandisäure-bis(t-butylester)

In ein Gemisch aus 10 ml Acetonitril und 20 ml Phosphatpuffer vom pH-Wert 8 werden 3,107 g (5 mmol) des unter 22c) hergestellten Amins und 3,523 g (10 mmol) N,N-Bis(t-butyloxycarbonylmethyl)-2-(bromethyl)-amin gegeben und 2 Stunden intensiv bei Raumtemperatur gerührt. Dann trennt man die Pufferphase ab, extrahiert sie mit 10 ml Acetonitril und gibt dieses zur organischen Phase. Nach Zugabe von 20 ml frischem Puffer rührt man noch 20 Stunden bei Raumtemperatur. Man trennt die organische Phase ab, engt sie ein und verteilt den Rückstand zwischen 100 ml Phosphatpuffer (pH 8,0) und 100 ml Essigester. Die organische Phase wird mit gesättigter Kochsalzlösung gewaschen, über Natriumsulfat getrocknet und eingeengt. Die Verbindung wird durch Chromatographie an Kieselgel gereinigt. Als Elutionsmittel dient Dichlormethan mit steigendem Zusatz von Methanol. Die Titelverbindung wird als glasartiger Feststoff erhalten.
Ausbeute: 3,044 g (52,3 % d. Th.)

| Elementaranalyse: | | | | |
|---|---|---|---|---|
| ber. | C 45,40 | H 5,71 | F 27,75 | N 6,02 |
| gef. | C 45,47 | H 5,78 | F 27,68 | N 6,10 |

### e) 3,9-Bis(carboxymethyl)-6-(1H,1H,4H,4H,5H,5H,8H,8H,10H,10H,11H,11H-2,7-dioxo-3,6-diaza-9-oxa-perfluormonodecyl)-3,6,9-triazaundecan-disäure

In eine Mischung von 120 ml Trifluoressigsäure / Dichlormethan im Verhältnis 2:1 werden 5,820 g (5 mmol) der unter 22d) hergestellten Verbindung gegeben. Man läßt über Nacht bei Raumtemperatur rühren, engt zur Trockne ein, entfernt Reste von Trifluoressigsäure durch Kodestillation mit Ethanol und nimmt in 240 ml eines Gemisches aus Wasser, Ethanol und Chloroform auf. Die Lösung wird durch Zugabe von Innenaustauscher IRA-67 (OH⁻Form) auf einen konstant bleibenden pH-Wert (etwa 3) eingestellt. Man saugt rasch ab, engt ein und erhält die Titelverbindung als glasigen Feststoff.
Ausbeute: 3,214 g (68,4 % d. Th.)
Wassergehalt: 10,3 %

| Elementaranalyse (auf wasserfreie Substanz bezogen): | | | | |
|---|---|---|---|---|
| ber. | C 35,79 | H 3,65 | F 34,37 | N 7,45 |
| gef. | C 35,90 | H 3,72 | F 34,31 | N 7,51 |

### f) Gadoliniumkomplex, Mononatriumsalz von 3,9-Bis(carboxymethyl)-6-(1H,1H,4H,4H,5H,5H,8H,8H,10H,10H,11H,11H-2,7-dioxo-3,6-diaza-9-oxa-perfluornonadecyl)-3,6,9-triaza-undecandisaure

In einem Gemisch aus 60 ml destilliertem Wasser und 30 ml Ethanol werden 3,143 g (3,0 mmol, berechnet auf 10,3 % Wassergehalt9 der unter 22e) hergestellten Säure gegeben. Unter Rühren und Erwärmen auf 50 °C werden anteilweise 543,8 mg (1,5 mmol) Gadoliniumoxid zugegeben. Nach beendeter Zugabe wird bis zur Lösung gerührt. Man stellt dann den pH-Wert der Lösung durch Zugabe von Natronlauge auf 7,2 ein, engt die Lösung ein, wobei starkes Schäumen zu beobachten ist. Der Rückstand wird mit destilliertem Wasser kodestilliert. Die Titelverbindung wird als glasiger Feststoff erhalten.
Ausbeute: 3,635 g (quantitativ)
Wassergehalt: 7,9 %

| Elementaranalyse (auf wasserfreie Substanz bezogen): | | | | | | |
|---|---|---|---|---|---|---|
| ber. | C 30,14 | H 2,71 | F 28,95 | Gd 14,09 | N 6,28 | Na 2,06 |
| gef. | C 30,21 | H 2,78 | F 29,03 | Gd 14,16 | N 6,22 | Na 2,11 |

### Beispiel 23

### Gadoliniumkomplex von 3,6,9-Tris(carboxymethyl)-3,6,9-triazaundecandisäure-bis{N-[2-aminoethyl-(N-ethyl-N-perfluoroctylsulfonyl]-amid}

### a) N-Ethyl-(2-benzyloxycarbonylamino-ethyl)-perfluoroctylsulfonsäureamid

In 30 ml Dimethylformamid werden 5,272 g (10 mmol) Perfluoroctylsulfonsäure-N-ethylamid gelöst. Unter Feuchtigkeitsausschluß versetzt man mit 330 mg (11 mmol) Natriumhydrid (80 % in Öl). Nach beendeter Gasentwicklung tropft man die Lösung von 2,093 g (10 mmol) N-Benzyloxycarbonylaziridin dazu und. Man gießt in 300 ml Eiswasser, extrahiert mit Dichlormethan, wäscht die organische Lösung mit Wasser, trocknet sie über Natriumsulfat und engt zur Trockne ein. Der Rückstand wird an Kieselgel mit Dichlormethan / Methanol chromatographiert Die Titelverbindung ist ein glasartiger Feststoff.
Ausbeute: 6,149 g (87,3 % d. Th.)

| Elementaranalyse: | | | | | |
|---|---|---|---|---|---|
| ber. | C 34,10 | H 2,43 | F 45,85 | N 3,98 | S 4,55 |
| gef. | C 34,00 | H 2,49 | F 45,97 | N 4,06 | S 4,49 |

### b) N-Ethyl-N-2-(aminoethyl)-perfluoroctylsulfonamid

In 100 ml eines Gemisches aus Tetrahydrofuran und Ethanol im Verhältnis 2:1 werden 3,522 g (5 mmol) der unter 23a) hergestellten Verbindung in Gegenwart von 0,2 g Pearlman-Katalysator (Pd 20%/C) bis zur Aufnahme von 112 ml Wasserstoff hydriert. Man saugt vom Katalysator ab, wäscht mit Ethanol gut nach und engt zur Trockne ein. Die Titelverbindung wird als amorpher Feststoff erhalten.
Ausbeute: 2,814 g (98,7 % d. Th.)

| Elementaranalyse: | | | | | |
|---|---|---|---|---|---|
| ber. | C 25,27 | H 1,94 | F 56,64 | N 4,91 | S 5,62 |
| gef. | C 25,39 | H 1,99 | F 56,57 | N 4,96 | S 5,53 |

### c) 3,6,9-Tris(carboxymethyl)-3,6,9-triazaundecandisäure-bis{N-[2-aminoethyl-(N-ethyl-N-perfluoroctylsulfonyl)]-amid}

In 30 ml trockenem Dimethylformamid werden 5,703 g (10 mmol) der unter 23b) hergestellten Verbindung sowie 1,518 g (15 mmol) Triethylamin gelöst und anteilweise unter Rühren und Feuchtigkeitsausschluß mit 1,787 g (5 mmol) Diethylentriaminpenta-essigsäure-Bisanhydrid versetzt. Man läßt über Nacht rühren, engt dann ein, versetzt mit Wasser, stellt den pH-Wert mit 3N Salzsäure auf etwa 3 ein und extrahiert zweimal mit je 100 ml n Butanol. Die organischen Lösungen werden vereinigt, eingeengt und einer Chromatographie an Kieselgel RP-18 unterworfen. Als Elutionsmittel dienen Wasser und Tetrahydrofuran. Die Titelverbindung wird als glasartiger Feststoff erhalten.
Ausbeute: 6,172 g (82,4 % d. Th.)
Wassergehalt: 9,8 %

| Elementaranalyse (auf wasserfreie Substanz bezogen): | | | | | |
|---|---|---|---|---|---|
| ber. | C 30,47 | H 2,76 | F 43,12 | N 6,55 | S 4,28 |
| gef. | C 30,59 | H 2,81 | F 43,00 | N 6,61 | S 4,33 |

### d) Gadoliniumkomplex von 3,6,9-Tris(carboxymethyl)-3,6,9-triazaundecandisäure-bis{N-[2-aminoethyl-(N-ethyl-N-perfluoroctylsulfonyl)]-amid}

In einem Gemisch aus 120 ml destilliertem Wasser, 60 ml Ethanol und 20 ml Chloroform werden 6,570 g (4 mmol, berechnet auf 9,8 % Wassergehalt) der unter 23c) hergestellten Verbindung gegeben. Unter rühren und Erwärmen auf 50 °C werden anteilweise 725 mg (82,0 mmol) Gadoliniumoxid zugegeben. Man rührt bis zur Lösung, engt dann ein, wobei starkes Schäumen auftritt und unterwirft den Rückstand der Kodestillation mit destilliertem Wasser. Die Kodestillation wird zweimal wiederholt. Die Titelverbindung wird als glasartiger Feststoff erhalten.
Ausbeute: 7,191 g (quantitativ)
Wassergehalt: 8,1 %

| Elementaranalyse (auf wasserfreie Substanz bezogen) | | | | | | |
|---|---|---|---|---|---|---|
| ber. | C 27,63 | H 2,32 | F 39,10 | Gd 9,52 | N 5,93 | S 3,88 |
| gef. | C 27,50 | H 2,37 | F 39,22 | Gd 9,61 | N 5,85 | S 3,95 |

### Beispiel 24

### Gadoliniumkomplex von 3,6,9-Tris(carboxymethyl)-3,6,9-triazaundecandisäure-bis{N-<2-aminoethyl-[glycolsäure-(1H,1H,2H,2H-perfluordecyl-ether)-amid]>-amid}

### a) 3,6,9-Tris(carboxymethyl)-3,6,9-triazaundecandisäure-bis{N-<2-aminoethylglycolsäure-(1H,1H,2H,2H-perfluordecyl-ether)-amid]>-amid}

In 40 ml trockenem Dimethylformamid werden 6,771 g (12 mmol) der unter Beispiel 22a) hergestellten Verbindung sowie 1,821 g (18 mmol) Triethylamin gelöst und anteilweise unter Rühren und Feuchtigkeitsausschluß mit 2,144 g (6 mmol) Diethylentriamin-pentaessigsaure-Bisanhydrid versetzt. Man läßt über Nacht rühren, engt dann ein, versetzt mit 20 ml Wasser, stellt den pH-Wert auf etwa 3 ein und extrahiert mit 3N Salzsäure zweimal mit je 150 ml Butanol. Die organischen Lösungen werden vereinigt, eingeengt und man unterwirft den Rückstand einer Chromatographie an Kieselgel RP-18. Als Elutionsmittel dienen Wasser und Tetrahydrofuran. Die Titelverbindung wird als glasartiger Feststoff erhalten.
Ausbeute: 6,989 g (78,4 % d. Th.)
Wassergehalt: 7,1 %

| Elementaranalyse (auf wasserfreie Substanz bezogen). | | | | |
|---|---|---|---|---|
| ber. | C 33,95 | H 3,05 | F 43,47 | N 6,60 |
| gef. | C 34,06 | H 3,11 | F 43,40 | N 6,67 |

### b) Gadoliniumkomplex von 3,6,9-Tris(carboxymethyl)-3,6,9-triazaundecandisäure-bis {N-<2-aminoethyl-[glycolsäure-(1H,1H,2H,2H-perfluordecyl-ether)-amid]>-amid}

In einem Gemisch aus 100 ml destilliertem Wasser, 50 ml Ethanol und 20 ml Chloroform werden 4,798 g (3 mmol, berechnet auf 7,1 % Wasser) der unter 24a) hergestellten Verbindung gegeben. Unter Rühren und Erwärmen auf 50 °C werden anteilweise 543,8 mg (1,5 mmol) Gadoliniumoxid zugefügt. Man rührt bis zur Lösung, engt dann ein, wobei starkes Schäumen auftritt. Der Rückstand wird mehrfach mit destilliertem Wasser kodestilliert. Die Titelverbindung wird als glasartiger Feststoff erhalten.
Ausbeute: 5,285 g (quantitativ)
Wassergehalt: 6,9 %

| Die Elementaranalyse ist auf wasserfreie Substanz berechnet. | | | | | |
|---|---|---|---|---|---|
| ber. | C 30,76 | H 2,58 | F 39,39 | Gd 9,59 | N 5,98 |
| gef. | C 30,87 | H 2,65 | F 39,51 | Gd 9,69 | N 6,11 |

### Beispiel 25

### Gadoliniumkomplex, Natriumsalz von 3,9-Bis(carboxymethyl)-6-[N-(1H,1H,2H,2H-perfluordecyl)-aminocarbonylmethyl-3,6,9-triazaundecandisäure

### a) N-Benzyloxycarbonylglycin-N-(1H,1H,2H,2H-perfluordecyl)-amid

In 70 ml Dichlormethan werden 7,877g (15 mmol) 1H,1H,2H,2H-Perfluordecylamin (J. Fluor. Chem. 55, 85 (1991)) gelöst und mit 1,726g (15 mmol) N-Hydroxysuccinimid, 3,095 g (15 mmol) Dicyclohexylcarbodiimid und 3,138 g (15 mmol) N-Benzyloxycarbonylglycin (Kaufware, Bachem) versetzt. Man läßt über Nacht rühren, filtriert den Dicyclohexylharnstoff ab, engt ein und unterwirft den Rückstand einer Säulenchromatographie an Kieselgel. Als Elutionsmittel dienen Gemische aus Dichlormethan und Ethanol Die Titelverbindung wird als Feststoff erhalten.
Ausbeute: 8,951 g (91,2 % d. Th.)

| Elementaranalyse: | | | | |
|---|---|---|---|---|
| ber. | C 36,71 | H 2,31 | F 49,36 | N 4,28 |
| gef. | C 36,87 | H 2,39 | F 49,51 | N 4,37 |

### b) Glycin-N-(1H,1H,2H,2H-perfluordecyl)-amid

In 150 ml eines Gemisches aus Tetrahydrofuran und Ethanol im Verhältnis 2:1 werden 7,594 g (10 mmol) der unter 28a) hergestellten Verbindung gelöst und in Gegenwart von 0,25 g Pearlman-Katalysator (Pd 20 % / C) bis zur Aufnahme von 224 ml Wasserstoff hydriert. Man saugt vom Katalysator ab, wäscht gut mit Ethanol nach und engt zur Trockne ein. Die Titelverbindung wird als amorpher Feststoff erhalten.
Ausbeute: 6,21 g (99,3 % d. Th.)

| Elementaranalyse: | | | | |
|---|---|---|---|---|
| ber. | C 25,37 | H 1,60 | F 56,84 | N 4,93 |
| gef. | C 25,28 | H 1,65 | F 56,92 | N 4,99 |

### c) 3,9-Bis(t-butyloxycarbonylmethyl)-6-N-[1H,1H,2H,2H-perfluordecyl)-amonocarbonylmethyl-3,6,9-triazaundecandisäure-di(t-butylester)

In ein Gemisch aus 10 ml Acetonitril und 20 ml Phosphatpuffer vom pH-Wert 8,0 werden 2,841 g (5 mmol) des unter 25b) hergestellten Amins und 3,875 g (11 mmol) N,N-Bis(t-butyloxycarbonylmethyl)-2-(bromethyl)-amin gegeben und 2 Stunden intensiv bei Raumtemperatur gerührt. Dann trennt man die Pufferphase ab, extrahiert sie mit 10 ml Acetonitril und gibt dieses zur organischen Phase. Nach Zugabe von 20 ml frischem Puffer rührt man noch 20 Stunden bei Raumtemperatur. Man trennt die organische Phase ab, engt sie ein und verteilt den Rückstand zwischen 100 ml Phosphatpuffer (pH 8.0) und 100 ml Essigester. Die organische Phase wird mit gesättigter Kochsalzlösung gewaschen, über Natriumsulfat getrocknet und eingeengt. Die Titelverbindung wird durch Chromatographie an Kieselgel gereinigt. Als Elutionsmittel dient Dichlormethan mit steigendem Zusatz von Methanol. Die Titelverbindung wird als glasartiger Feststoff erhalten.
Ausbeute: 4,161 g (78,3 % d. Th.)

| Elementaranalyse: | | | | |
|---|---|---|---|---|
| ber. | C 45,20 | H 5,59 | F 30,39 | N 5,27 |
| gef. | C 45,35 | H 5,67 | F 30,47 | N 5,34 |

### d) 3,9-Bis(carboxymethyl)-6-N-(1H,1H,2H,2H-perfluordecyl)-aminocarbonylmethyl-3,6,9-triazaundecandisäure

In eine Mischung von 100 ml Trifluoressigsäure / Dichlormethan im Verhältnis 2:1 werden 4,783 g (4,5 mmol) der unter 25c) hergestellten Verbindung gegeben. Man läßt über Nacht bei Raumtemperatur rühren, engt dann zur Trockne ein, entfernt Reste von Trifluoressigsäure durch Kodestillation mit Ethanol und nimmt in 160 ml eines Gemisches aus Wasser, Ethanol und Chloroform (10:5:1) auf. Durch Zugabe von Ionenaustauscher IR A-67 (OH⁻-Form) wird ein pH-Wert von etwa 3 (pH-Konstanz) eingestellt. Man saugt rasch ab, engt ein und erhält die Titelverbindung als glasigen Feststoff.
Ausbeute: 3,007 g (79,7 % d. Th )
Wassergehalt: 10,9 %

| Elementaranalyse (auf wasserfreie Substanz bezogen): | | | | |
|---|---|---|---|---|
| ber. | C 34,38 | H 3,25 | F 38,52 | N6,68 |
| gef. | C 34,29 | H 3,33 | F 38,65 | N 6,77 |

### e) Gadoliniumkomplex, Mononatriumsalz von 3,9-Bis(carboxymethyl)-6-N-(1H,1H,2H,2H-perfluordecyl)-aminocarbonylmethyl)-3,6,9-triazaundecandisäure

In ein Gemisch aus 60 ml destilliertem Wasser und 30 ml Ethanol werden 2,823 g (3,0 mmol, berechnet auf 10,9 % Wassergehalt) der unter Beispiel 25d) hergestellten Säure gegeben. Unter Rühren und Erwärmen auf 50 °C werden anteilweise 543,8 mg (1,5 mmol) Gadoliniumoxid zugegeben. Nach beendeter Zugabe wird bis zur Lösung gerührt. Dann stellt man den pH-Wert der Lösung durch Zugabe von Natronlauge auf 7,2 ein. Die Lösung wird eingeengt. Es tritt dabei starkes Schäumen auf. Der Rückstand wird zweimal mit destilliertem Wasser kodestilliert. Die Titelverbindung wird als glasiger Feststoff erhalten.
Ausbeute: 3,353 g (quant)
Wassergehalt: 9,2 %

| Die Elementaranalyse wird auf wasserfreie Substanz berechnet. | | | | | | |
|---|---|---|---|---|---|---|
| ber. | C 28,41 | H 2,28 | F 31,83 | Gd 15,50 | N 5,52 | Na 2,27 |
| gef. | C 28,51 | H 2,33 | F 31,76 | Gd 15,57 | N 5,46 | Na 2,35 |

### Beispiel 26

### Gadoliniumkomplex, Dinatriumsalz von 3,6,9-Tris(carboxymethyl)-4-[N-1H,1H,2H,2H-perfluordecyloxy)-benzyl]-3,6,9-triaza-undecandisäure

### a) 3,6,9-Tris-(t-butyloxycarbonylmethyl)-4-[4-(1H,1H,2H,2H-perfluordecyloxy)-benzyl]-3,6,9-triazaundecandisäure-di(t-butylester)

In 50 ml trockenes Dimethylformamid werden 6,131 g (5 mmol) 3,6,9-Tris (t-butyloxycarbonylmethyl)-4-(4-hydroxybenzyl)-3,6,9-triazaundecandisäure-di(t-butylester), dargestellt nach PCT WO 88/07521 gegeben und anteilweise unter Rühren und Feuchtigkeitsausschluß mit 150 g (5 mmol) Natriumhydrid (80 % in Öl) versetzt. Nach erfolgter Auflösung versetzt man mit 3,092 g (5 mmol) des unter Beispiel 7a) hergestellten Tosylats. Man rührt 12 Stunden bei 40°C. Dann gießt man in 500 ml Eiswasser, nimmt das Produkt in Dichlormethan auf, wäscht die organische Lösung mit Wasser, trocknet über Natriumsulfat und engt zur Trockne ein. Der Rückstand wird durch Chromatographie an Kieselgel gereinigt. Als Elutionsmittel dient ein Gemisch aus Dichlormethan, Isopropanol, Hexan im Verhältnis 20:1:5.
Die Titelverbindung wird als amorpher Feststoff erhalten.
Ausbeute: 5,015 g (81,8 % d. Th.)

| Elementaranalyse: | | | | |
|---|---|---|---|---|
| ber. | C 49,96 | H 5,92 | F 26,34 | N 3,43 |
| gef. | C 50,11 | H 6,00 | F 26,43 | N 3,38 |

### b) 3,6,9-Tris(carboxymethyl)-4-[4-(1H,1H,2H,2H-perfluordecyloxy)-benzyl]-3,6,9-triazaundecandisäure

In 100 ml eines Gemisches aus Trifluoressigsäure und Dichlormethan im Verhältnis 2:1 werden 3,678 g (3 mmol) der unter Beispiel 26a) hergestellten Verbindung gelöst und über Nacht bei Raumtemperatur gerührt. Man engt zur Trockne ein und entfernt Reste von Trifluoressigsäure durch Kodestillation mit Ethanol. Man nimmt den Rückstand in 160 ml eines Gemisches aus Wasser, Ethanol und Chloroform (10:5:1) auf. Durch Zugabe von Ionenaustauscher IRA-67 (OH⁻-Form) wird ein PH-Wert von etwa 3 (pH-Konstanz) eingestellt. Man saugt rasch ab, engt ein und erhält die Titelverbindung als glasigen Feststoff. Ausbeute: 2,357 g (83,1 % d. Th.)
Wassergehalt: 11,3 %

| Die Elementaranalyse ist auf wasserfreie Substanz berechnet. | | | | |
|---|---|---|---|---|
| ber. | C 39,38 | H 3,41 | F 34,16 | N 4,44 |
| gef. | C 39,52 | H 3,47 | F 34,32 | N 4,36 |

### c) Gadoliniumkomplex, Dinatriumsalz von 3,6,9-Tris(carboxymethyl)-4-[N-(1H,1H,2H,2H-perfluordecyloxy)-benzyl]-3,6,9-triaza-undecandisäure

In ein Gemisch aus 60 ml destilliertem Wasser und 30 ml Ethanol werden 3,145 g (3,0 mmol, berechnet auf 11,3 % Wassergehalt) der unter Beispiel 26b) hergestellten Säure gegeben. Unter Rühren und Erwärmen auf 50 °C werden anteilweise 543,8 mg (1,5 mmol) Gadoliniumoxid zugegeben. Nach beendeter Zugabe wird bis zur Lösung gerührt. Dann stellt man den pH-Wert der Lösung durch Zugabe von Natronlauge auf 7,2 ein und engt ein. Dabei tritt starkes Schäumen auf. Der Rückstand wird zweimal mit destilliertem Wasser kodestilliert.
Die Titelverbindung wird als glasiger Feststoff erhalten.
Ausbeute: 3,804 g (quantitativ)
Wassergehalt: 9,8 %

| Elementaranalyse (auf wasserfreie Substanz bezogen): | | | | | | |
|---|---|---|---|---|---|---|
| ber. | C 32,55 | H 2,38 | F 28,24 | Gd 13,75 | N 3,67 | Na 4,02 |
| gef. | C 32,44 | H 2,43 | F 28,30 | Gd 13,66 | N 3,71 | Na 4,10 |

### Beispiel 27

### Gadoliniumkomplex von 10-[(-perfluoroctyl-sulfonyl)-piperazin-1-yl-carbonylmethyl]-1,4,7-tris(carboxymethyl)-1,4,7,10-tetraazacyclododecan

### a) 1-Perfluoroctylsulfonyl-piperazin

34,39 g (398,3 mmol) Piperazin, 50 g (99,6 mmol) Perfluoroctylsulfonylfluorid und 10,12 g (100 mmol) Triethylamin werden 24 Stunden auf 85°C erhitzt. Man gibt 500 ml Wasser zu und extrahiert zweimal mit je 200 ml Dichlormethan. Die organische Phase wird über Magnesiumsulfat getrocknet und im Vakuum zur Trockne eingedampft. Der Rückstand wird an Kieselgel chromatographiert (Laufmittel: Dichlormethan/2-Propanol= 25:1).
Ausbeute: 17,55 g (31 % d. Th.) eines farblosen amorphen Feststoffes

| Elementaranalyse: | | | | | |
|---|---|---|---|---|---|
| ber. | C 25,36 | H 1,60 | F 56,84 | N 4,93 | S 5,64 |
| gef. | C 25,15 | H 1,80 | F 56,65 | N 4,81 | S 5,70 |

### b) 1-(2-Bromacetyl)-4-perfluoroctylsulfonyl-piperazin

17 g (29,9 mmol) der Titelverbindung aus Beispiel 27a) und 5,1 g (50 mmol) Triethylamin werden in 100 ml Dichlormethan gelöst. Bei -10°C tropft man innerhalb von 30 Minuten 9,1 g (44,9 mmol) Bromacetylbromid zu und rührt 2 Stunden bei 0°C. Man gießt die Lösung in 200 ml 2 N Salzsäure und rührt gut durch. Die organische Phase wird abgetrennt, über Magnesiumsulfat getrocknet und im Vakuum eingeengt. Der Rückstand wird an Kieselgel chromatographiert (Laufmittel: Dichlormethan/Aceton= 20/1).
Ausbeute: 18,55 g (90 % d. Th.) eines leicht gelbgefärbten wachsartigen Feststoffes

| Elementaranalyse: | | | | | | |
|---|---|---|---|---|---|---|
| ber. | C 24,40 | H 1,46 | F 46,86 | N 4,06 | S 4,65 | Br 11,59 |
| gef. | C 24,22 | H 1,60 | F 46,75 | N 3,97 | S 4,48 | Br 11,41 |

### c) 10-[(-perfluoroctyl-sulfonyl)-piperazin-1-yl-carbonylmethyl]-1,4,7-tris(carboxymethyl)-1,4,7,10-tetraazacyclododecan

Zu 17,78 g (20 mmol) der Titelverbindung aus Beispiel 27b) in 180 ml Methanol gibt man 4,63 g (13,36 mmol) 1,4,7-Tris(carboxymethyl)-1,4,7,10-tetraazacyclododecan (≅ DO3A) und 18,5 g (133,6 mmol) Kaliumkarbonat. Man kocht 12 Stunden unter Rückfluss. Die anorganischen Salze werden abfiltriert und das Filtrat zur Trockne eingedampft. Der Rückstand wird in 100 ml Wasser aufgenommen und mit 5 N Salzsäure auf pH 3 gestellt. Man extrahiert 2 mal mit 150 ml n-Butanol. Die vereinigten organischen Phasen werden im Vakuum zur Trockne eingeengt und der Rückstand durch RP-Chromatographie gereinigt (RP-18/Laufmittel= Gradient aus Wasser/n-Butanol/Acetonitril).
Ausbeute: 12,79 g (67 % d. Th.) eines farblosen Feststoffes
Wassergehalt: 8,5 %

| Elementaranalyse (bezogen auf wasserfreie Substanz): | | | | | |
|---|---|---|---|---|---|
| ber. | C 35,23 | H 3,70 | F 33,83 | N 8,80 | S 3,36 |
| gef. | C 35,17 | H 3,81 | F 33,67 | N 8,65 | S 3,18 |

### d) Gadoliniumkomplex von 10-[(-perfluoroctyl-sulfonyl)-piperazin-1-yl-carbonylmethyl]-1,4,7-tris(carboxymethyl)-1,4,7,10-tetraazacyclododecan

10 g (10,47 mmol) der Titelverbindung aus Beispiel 27c) werden in einer Mischung aus 50 ml Wasser/20 ml Ethanol gelöst und 1,90 g (5,23 mmol) Gadoliniumoxid zugegeben. Man rührt 4 Stunden bei 80°C. Die Lösung wird filtriert und im Vakuum zur Trockne eingedampft.
Ausbeute: 12,2 g (quantitativ)
Wassergehalt: 5,1 %

| Elementaranalyse (bezogen auf wasserfreie Substanz): | | | | | |
|---|---|---|---|---|---|
| ber. | C 30,33 | H 2,91 | F 29,13 | Gd 14,18 | S 2,89 |
| gef. | C 30,39 | H 2,81 | F 29,02 | Gd 14,01 | S 2,78 |

### Beispiel 28

### Gadoliniumkomplex, Mononatriumsalz von 3,9-Bis(carboxymethyl)-6-[(4-perfluoroctylsulfonyl)-piperazin-1-carbonylmethyl]-3,6,9-triazaundecandisäure

### a) 1-(2-Benzyloxycarbonylamino)-methyl-carbonyl-4-(perfluoroctylsulfonyl)-piperazin

In 80 ml Dichlormethan werden 8,524 g (15 mmol) des unter 27a) hergestellten Piperazinderivates gelöst und mit 1,726 g (15 mmol) N-Hydroxysuccinimid, 3,095 g (15 mmol) Dicyclohexylcarbodiimid und 3,138 g (15 mmol) N-Benzyloxycarbonylglycin (Kaufware, Bachem) versetzt. Man läßt über Nacht rühren, filtriert den Dicyclohexylharnstoff ab, engt ein und unterwirft den Rückstand einer Säulenchromatographie an Kieselgel. Als Elutionsmittel dienen Gemische aus Dichlormethan und Ethanol Die Titelverbindung wird als Feststoff erhalten.
Ausbeute: 10,16 g (89,2 % d. Th.)

| Elementaranalyse. | | | | | |
|---|---|---|---|---|---|
| ber. | C 34,79 | H 2,39 | F 42,53 | N 5,53 | S 4,22 |
| gef. | C 34,60 | H 2,43 | F 42,65 | N 5,66 | S 4,17 |

### b) 1-(2-Amino)-acetyl-4-(perfluoroctyl)-sulfonyl-piperazin

In 150 ml eines Gemisches aus Tetrahydrofuran und Ethanol im Verhältnis 2:1 werden 7,594 g (10 mmol) der unter 28a) hergestellten Verbindung gelöst und in Gegenwart von 0,25 g Pearlman-Katalysator (Pd 20 % / C) bis zur Aufnahme von 224 ml Wasserstoff hydriert. Man saugt vom Katalysator ab, wäscht gut mit Ethanol nach und engt zur Trockne ein. Die Titelverbindung wird als amorpher Feststoff erhalten.
Ausbeute: 6,21 g (99,3 % d. Th.)

| Elementaranalyse: | | | | | |
|---|---|---|---|---|---|
| ber. | C 26,89 | H 1,93 | F 51,65 | N 6,72 | S 5,13 |
| gef. | C 27,03 | H 1,97 | F 51,77 | N 6,58 | S 5,20 |

### c) 3,9-Bis(t-butyloxycarbonylmethyl)-6-[(4-perfluoroctylsulfonyl)-piperazin-1-carbonylmethyl]-3,6,9-triazaundecandicarbonsäure-di(t-butylester)

In ein Gemisch aus 10 ml Acetonitril und 20 ml Phosphatpuffer vom pH-Wert 8,0 werden 3,127 g (5 mmol) des unter 28b) hergestellten Amins und 3,875 g (11 mmol) N,N-Bis(t-butyloxycarbonylmethyl)-2-(bromethyl)-amin gegeben und 2 Stunden intensiv bei Raumtemperatur gerührt. Dann trennt man den Puffer ab, extrahiert mit 10 ml Acetonitril und gibt dieses zur organischen Phase. Nach Zugabe von 20 ml frischem Puffer rührt man noch 20 Stunden bei Raumtemperatur. Man trennt die organische Phase ab, engt sie ein und verteilt den Rückstand zwischen 100 ml Phosphatpuffer (pH 8.0) und 100 ml Essigester. Die organische Phase wird mit gesättigter Kochsalzlösung gewaschen, über Natriumsulfat getrocknet und eingeengt. Die Titelverbindung wird durch Chromatographie an Kieselgel gereinigt. Als Elutionsmittel dient Dichlormethan mit steigendem Zusatz von Methanol. Die Titelverbindung wird als glasartiger Feststoff erhalten.
Ausbeute: 4,481 g (76,3 % d. Th.)

| Elementaranalyse: | | | | | |
|---|---|---|---|---|---|
| ber. | C 43,71 | H 5,42 | F 27,99 | N 4,85 | S 2,78 |
| gef. | C 43,84 | H 5,47 | F 28,10 | N 5,00 | S 2,69 |

### d) 3,9-Bis(carboxymethyl)-6-[(4-perfluoroctyl-sulfonyl)-piperazin-1-yl-carbonylmethyl]-3,6,9-triazaundecandisäure

In eine Mischung von 100 ml Trifluoressigsäure / Dichlormethan im Verhältnis 2:1 werden 5,193 g (4,5 mmol) der unter 28c) hergestellten Verbindung gegeben. Man läßt über Nacht bei Raumtemperatur rühren, engt dann zur Trockne ein, entfernt Reste von Trifluoressigsäure durch Kodestillation mit Ethanol und nimmt in 160 ml eines Gemisches aus Wasser, Ethanol und Chloroform (10:5:1) auf. Durch Zugabe von Ionenaustauscher IRA-67 (OH⁻-Form) wird ein pH-Wert von etwa 3 (pH-Konstanz) eingestellt. Man saugt rasch ab, engt ein und erhält die Titelverbindung als glasigen Feststoff.
Ausbeute: 3,718 g (79,2 % d. Th.)
Wassergehalt: 10,9 %

| Elementaranalyse (bezogen auf wasserfreie Substanz): | | | | | |
|---|---|---|---|---|---|
| ber. | C 33,59 | H 3,25 | F 34,74 | N 6,03 | S 3,45 |
| gef. | C 33,69 | H 3,36 | F 34,82 | N 6,10 | S 3,38 |

### e) Gadoliniumkomplex, Mononatriumsalz von 3,9-Bis(carboxymethyl)-6-[(4-perfluoroctylsulfonyl)-piperazin-1-carbonylmethyl]-3,6,9-triazaundecandisäure

In ein Gemisch aus 60 ml destilliertem Wasser und 30 ml Ethanol werden 3,13 g (3,0 mmol, berechnet auf 10,9 % Wassergehalt) der unter Beispiel 28d) hergestellten Säure gegeben. Unter Rühren und Erwärmen auf 50 °C werden anteilweise 543,8 mg (1,5 mmol) Gadoliniumoxid zugegeben. Nach beendeter Zugabe wird bis zur Lösung gerührt. Dann stellt man den pH-Wert der Lösung durch Zugabe von Natronlauge auf 7,2 ein und engt ein. Dabei tritt starkes Schäumen auf. Der Rückstand wird zweimal mit destilliertem Wasser kodestilliert.
Die Titelverbindung wird als glasiger Feststoff erhalten.
Ausbeute: 3,678 g (quantitativ)
Wassergehalt. 9,2%

| Elementaranalyse (bezogen auf wasserfreie Substanz): | | | | | | | |
|---|---|---|---|---|---|---|---|
| ber. | C 28,24 | H 2,37 | F 29,21 | Gd 14,22 | N 5,07 | Na 2,08 | S 2,90 |
| gef. | C 28,36 | H 2,41 | F 29,14 | Gd 14,30 | N 5,15 | Na 2,12 | S 2,83 |

### Beispiel 29

### Gadoliniumkomplex von 3,6,9-Tris(carboxymethyl)-3,6,9-triazaundecandisäure-bis[(4-perfluoroctylsulfonyl)-piperazin]-amid

### a) 3,6,9-Tris(carboxymethyl)-3,6,9-triazaundecandisäure-bis[(4-perfluoroctylsulfonyl)-piperazin]-amid

In 30 ml trockenem Dimethylformamid werden 5,683 g (10 mmol) der unter 27a) hergestellten Verbindung sowie 1,518 g (15 mmol) Triethylamin gelöst und anteilweise unter Rühren und Feuchtigkeitsausschluß mit 1,787 g (5 mmol) Diethylentriaminpenta-essigsäure-Bisanhydrid versetzt. Man läßt über Nacht rühren, engt dann ein, versetzt mit Wasser, stellt den pH-Wert mit 3N Salzsäure auf etwa 3 ein und extrahiert zweimal mit je 100 ml n Butanol. Die organischen Lösungen werden vereinigt, eingeengt und einer Chromatographie an Kieselgel RP-18 unterworfen. Als Elutionsmittel dienen Wasser und Tetrahydrofuran. Die Titelverbindung wird als glasartiger Feststoff erhalten.
Ausbeute: 6,741 g (81,4 % d. Th.)
Wassergehalt: 9,8 %

| Elementaranalyse (bezogen auf wasserfreie Substanz): | | | | | |
|---|---|---|---|---|---|
| ber. | C 30,55 | H 2,50 | F 43,24 | N 6,56 | S 4,29 |
| gef. | C 30,67 | H 2,55 | F 43,33 | N 6,49 | S 4,21 |

### b) Gadoliniumkomplex von 3,6,9-Tris(carboxymethyl)-3,6,9-triazaundecandisäure-bis[(4-perfluoroctylsulfonyl)-piperazin]-amid

In einem Gemisch aus 120 ml destilliertem Wasser, 60 ml Ethanol und 20 ml Chloroform werden 6,570 g (4 mmol, berechnet auf 9,8 % Wassergehalt) der unter 23c) hergestellten Verbindung gegeben. Unter Rühren und Erwärmen auf 50 °C werden anteilweise 725 mg (82,0 mmol) Gadoliniumoxid zugegeben. Man rührt bis zur Lösung, engt dann ein, wobei starkes Schäumen auftritt und unterwirft den Rückstand der Kodestillation mit destilliertem Wasser. Die Kodestillation wird zweimal wiederholt. Die Titelverbindung wird als glasartiger Feststoff erhalten.
Ausbeute: 7,191 g (quantitativ)
Wassergehalt: 8,1 %

| Elementaranalyse (bezogen auf wasserfreie Substanz): | | | | | | |
|---|---|---|---|---|---|---|
| ber. | C 27,69 | H 2,08 | F 39,19 | Gd 9,54 | N 5,95 | S 3,89 |
| gef. | C 27,83 | H 2,15 | F 39,10 | Gd 6,91 | N 6,03 | S 3,88 |

### Beispiel 30

### a) 11 -[N-Ethyl-N-(perfluoroctylsulfonyl)-amino]undecansäurebenzylester

20 g (37,94 mmol) N-Ethyl-N-perfluoroctylsulfonamid und 15,73 g {113,8 mmol) Kaliumcarbonat werden in 200 ml Aceton suspendiert und bei 60 °C 26,96 g (75,87 mmol) 11-Bromundecansäurebenzylester zugetropft. Man rührt 3 Stunden bei 60 °C. Man filtriert von den Salzen ab und dampft das Filtrat im Vakuum zur Trockne ein. Der Rückstand wird an Kieselgel chromatographiert (Laufmittel: Hexan/Dichlormethan/Aceton = 10/10/1). Nach Eindampfen der produkthaltigen Fraktionen kristallisiert man den Rückstand aus Methanol/Ether um.
Ausbeute: 26,46 g (87 % der Theorie) eines farblosen, kristallinen Pulvers.

| Elementaranalyse: | | | | | |
|---|---|---|---|---|---|
| ber. | C 41,95 | H 4,02 | N 1,75 | F 40,29 | S 4,00 |
| gef. | C 41,78 | H 4,17 | N 1,68 | F 40,12 | S 3,88 |

### b) 11-[N-Ethyl-N-(perfluoroctylsulfonyl)-aminoundecansäure

20 g (24,95 mmol) der Titelverbindung aus Beispiel 30 a) werden in 300 ml Isopronanol / 200 ml Dichlormethan gelöst und 3 g Palladiumkatalysator (10 % Pd/C) zugegeben. Man hydriert über Nacht bei Raumtemperatur. Es wird vom Katalysator abfiltriert und das Filtrat im Vakuum zur Trockne eingedampft. Der Rückstand wird aus Ether/Hexan umkristallisiert.
Ausbeute: 16,69 g (94 % der Theorie) eines farblosen, kristallinen Feststoffs.

| Elementaranalyse: | | | | | |
|---|---|---|---|---|---|
| ber. | C 35,45 | H 3,68 | N 1,97 | F 45,39 | S 4,51 |
| gef. | C 35,31 | H 3,81 | N 1,85 | F 45,25 | S 4,42 |

### c) Gadolinium-Komplex von 10-[2-Hydroxy-4-aza-5-oxo-16-aza-16-(perfluoroctylsulfonyl-octadecyl]-1,4,7-tris(carboxymethyl)-1,4,7,10-tetraazacyclododecan

12,16 g (17,09 mmol) der Titelverbindung aus Beispiel 30b) und 1,97 g (18,79 mmol) N-Hydroxysuccinimid werden in einer Mischung aus 50 ml Dimethylformamid/50 ml Chloroform gelöst. Bei 0 °C gibt man 3,88 g (18,79 mmol) Dicyclohexylcarbodiimid zu und rührt 1 Stunde bei 0 °C, anschließend 3 Stunden bei Raumtemperatur. Man kühlt erneut auf 0 °C und gibt 5,19 g (51,27 mmol) Triethylamin/50 ml 2-Propanol zu. Anschließend werden 10,78 g (18,79 mmol) Gadolinium-Komplex von 10-(3-Amino-2-hydroxypropyl). 1,4,7-tris(carboxymethyl)-1,4,7,10-tetraazacyclododecan (WO 95/17451) gelöst in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylharnstoff ab. Das Filtrat wird zur Trockne eingedampft und durch RP-Chromatographie gereinigt (RP-18 / Laufmittel: Gradient aus Wasser/N-Propanol/Acetonitril).
Ausbeute: 16,82 g (71 % der Theorie) eines farblosen, glasigen Feststoffs.
Wassergehalt: 8,6 %

| Elementaranalyse (bezogen auf wasserfreie Substanz): | | | | | | |
|---|---|---|---|---|---|---|
| ber. | C 36,02 | H 4,30 | F 25,49 | Gd 12,41 | N 6,63 | S 2,53 |
| gef. | C 35,87 | H 4,45 | F 25,28 | Gd 12,29 | N 6,50 | S 2,41 |

### d) 10-[2-Hydroxy-4-aza-5-oxo-16-aza-16-(perfluoroctylsulfonyl-octadecyl]-1,4,7-tris(carboxymethyl)-1,4,7,10-tetraazacyclododecan

11,1 g (8,76 mmol) der Titelverbindung aus Beispiel 30 c) werden in einer Mischung aus 100 ml Wasser/100 ml Ethanol gelöst und 1,73 g (13,71 mmol) Oxalsäure-Dihydrat zugesetzt. Man erhitzt 8 Stunden auf 80 °C. Es wird auf 0 °C abgekühlt und vom ausgefallenen Gadoliniumoxalat abfiltriert. Das Filtrat wird zur Trockne eingedampft und der Rückstand an RP-18 gereinigt (RP-18 / Laufmittel: Gradient aus Wasser/i-Propanol/Acetonitril).
Ausbeute: 9,80 g (92 % der Theorie) eines glasigen Feststoffs.
Wassergehalt: 8,5 %

| Elementaranalyse (bezogen auf wasserfreie Substanz): | | | | | |
|---|---|---|---|---|---|
| ber. | C 41,01 | H 5,16 | F 29,02 | N 7,55 | S 2,88 |
| gef. | C 40,87 | H 5,31 | F 28,85 | N 7,40 | S 2,73 |

### e) Ytterbium-Komplex von 10-[2-Hydroxy-4-aza-5-oxo-16-aza-16-(perfluoroctylsulfonyl-octadecyl]-1,4,7-tris(carboxymethyl)-1,4,7,10-tetraazacyclododecan

Zu 5,64 g (5,07 mmol) der Titelverbindung aus Beispiel 30 d) in 100 ml Wasser/50 ml Ethanol gibt man 1,33 g (2,53 mmol) Ytterbiumcarbonat und rührt 3 Stunden bei 80 °C.

Die Lösung wird filtriert und das Filtrat im Vakuum zur Trockne eingedampft.
Ausbeute: 7,08 g (quantitativ) eines glasigen Feststoffs.
Wassergehalt: 8,1 %

| Elementaranalyse (bezogen auf wasserfreie Substanz): | | | | | | |
|---|---|---|---|---|---|---|
| ber. | C 35,58 | H 4,24 | F 25,17 | N 6,55 | S 2,50 | Yb 13,49 |
| gef. | C 35,43 | H 4,37 | F 25,05 | N 6,48 | S 2,39 | Yb 13,35 |

### f) Dysprosium-Komplex von 10-[2-Hydroxy-4-aza-5-oxo-16-aza-16-(perfluoroctylsulfonyl-octadecyl]-1,4,7-tris(carboxymethyl)-1,4,7,10-tetraazacyclododecan

Zu 5,64 g (5,07 mmol) der Titelverbindung aus Beispiel 30 d) in 100 ml Wasser/50 ml Ethanol gibt man 0,95 g (2,53 mmol) Dysprosiumoxid und rührt 3 Stunden bei 80 °C. Die Lösung wird filtriert und das Filtrat im Vakuum zur Trockne eingedampft.
Ausbeute: 7,10 g (quantitativ) eines farblosen glasigen Feststoffs.
Wassergehalt: 9,1 %

| Elementaranalyse (bezogen auf wasserfreie Substanz): | | | | | | |
|---|---|---|---|---|---|---|
| ber. | C 35,87 | H 4,28 | F 25,38 | N 6,60 | S 2,52 | Dy 12,77 |
| gef. | C 35,69 | H 4,39 | F 25,18 | N 6,49 | S 2,43 | Dy 12,70 |

### Beispiel 31

### a) 11,11,11,10,10,9,9,8,8,7,7-Tridecafluor-3-oxaundecansäure-tert.-butylester

Zu einer Mischung aus 27,57 g (75.73 mmol) 1H, 1H, 2H, 2H-Perfluoroctan-1-ol und 2,57 g (7.57 mmol) Tetrabutylammoniumhydrogensulfat in 300 ml 60 % wäss. Kalilauge/200 ml Toluol tropft man unter starkem Rühren bei 0 °C 19.51 g (100.0 mmol)Bromessigsaure-tert.-butylester zu. Man rührt eine Stunde bei 0 °C, trennt die organische Phase ab und extrahiert die wässrige Phase 2 mal mit 50 ml Toluol. Die vereinigten organischen Extrakte werden über Natriumsulfat getrocknet und im Vakuum eingedampft. Der Rückstand wird an Kieselgel chromatographiert (Laufmittel: Dichlormethan).
Ausbeute 28.97 g (80 % der Theorie) eines farblosen Öls.

| Elementaranalyse: | | | |
|---|---|---|---|
| ber. | C 35.16 | H 3.16 | F 51.64 |
| gef. | C 35.08 | H 3.20 | F 51.70 |

### b) 11,11,11,10,10,9,9,8,8,7,7-Tridecafluor-3-oxaundecansäure

25.29 g (52.88 mmol) der Titelverbindung aus Beispiel 1a) werden in 300 ml Trifluoressigsaure gelöst und über Nacht bei Raumtemperatur gerührt. Man dampft im Vakuum zur Trockne ein und kristallisiert den Rückstand aus Hexan/Diethylether um.
Ausbeute 20.54 g (92 % der Theorie) eines farblosen kristallinen Feststoffs.

| Elementaranalyse: | | | |
|---|---|---|---|
| ber. | C 28.45 | H 1.67 | F 58.51 |
| gef. | C 28.36 | H 1.60 | F 58.62 |

### c) Gadolinium-Komplex von 10-[2-Hydroxy-4-aza-5-oxo-7-oxa-10,10,11,11, 12,12,13,13,14,14,15,15,15-tridecafluor-pentadecyl]-1,4,7-tris(carboxymethyl)-1,4,7,10-tetraazacyclododecan

7.21 g (17.09 mmol) der Titelverbindung aus Beispiel 31b und 1.97 g (18.79 mmol) N-Hydroxysuccinimid werden in einer Mischung aus 50 ml Dimethylformamid/50 ml Chloroform gelöst. Bei 0 °C gibt man 3.88 g (18.79 mmol) Dicyclohexylcarbodiimid zu und rührt 1 Stunde bei 0 °C, anschließend 3 Stunden bei Raumtemperatur. Man kühlt erneut auf 0 °C und gibt 5.19 g (51.27 mmol) Triethylamin/50 ml 2-Propanol zu. Anschließend werden 10.78 g (18.79 mmol) Gadolinium-Komplex von 10-(3-Amino-2-hydroxypropyl)-1.4.7-tris(carboxymethyl)-1.4.7.10-tetraazacyclododecan (WO 95/17451) gelöst in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylharnstoff ab. Das Filtrat wird zur Trockne eingedampft und durch RP-Chromatograpie gereinigt (RP-18/Laufmittel: Gradient aus Wassern-Propanol/Acetonitril).
Ausbeute 12,68 g (71 % der Theorie) eines farblosen glasigen Feststoffes.
Wassergehalt: 6,4 %

| Elementaranalyse (bezogen auf wasserfreie Substanz): | | | | | |
|---|---|---|---|---|---|
| ber. | C 33.16 | H 3.61 | F 25.26 | Gd 16.08 | N 7,16 |
| gef. | C 32.85 | H 3.84 | F 25.01 | Gd 15.87 | N 7.03 |

### Beispiel 32

### a) 15,15,15,14,14,13,13,12,12,11,11,10,10,9,9,8,8-7,7-Henicosafluor-3-oxapentadecansäure-tert.-butylester

Zu einer Mischung aus 42.72 g (75.73 mmol) 1H, 1H, 2H, 2H-Perfluoroctan-1-ol und 2,57 g (7.57 mmol) Tetrabutylammoniumhydrogensulfat in 300 ml 60 % wäss. Kalilauge/200 ml Toluol tropft man unter starkem Rühren bei 0 °C 19.51 g (100.0 mmol)Bromessigsäure-tert.-butylester zu. Man rührt eine Stunde bei 0 °C, trennt die organische Phase ab und extrahiert die wässrige Phase 2 mal mit 50 ml Toluol. Die vereinigten organischen Extrakte werden über Natriumsulfat getrocknet und im Vakuum eingedampft. Der Rückstand wird an Kieselgel chromatographiert (Laufmittel: Dichlormethan).
Ausbeute 42.12 g (82 % der Theorie) eines farblosen Öls.

| Elementaranalyse: | | | |
|---|---|---|---|
| ber. | C 31.87 | H 2.23 | F 58.82 |
| gef. | C 31.73 | H 2.20 | F 58.90 |

### b) 15,15,15,14,14,13,13,12,12,11,11,10,10,9,9,8,8,7,7-Henicosafluor-3-oxapentadecansäure-tert.-butylester

35.87 g (52.88 mmol) der Titelverbindung aus Beispiel 1a) werden in 300 ml Trifluoressigsäure gelöst und über Nacht bei Raumtemperatur gerührt. Man dampft im Vakuum zur Trockne ein und kristallisiert den Rückstand aus Hexan/Diethylether um.
Ausbeute 30.60 g (93 % der Theorie) eines farblosen kristallinen Feststoffs.

| Elementaranalyse: | | | |
|---|---|---|---|
| ber. | C 27.03 | H 1.13 | F 64.12 |
| gef. | C 26.91 | H 1.20 | F 64.02 |

### c) Gadolinium-Komplex von 10-[2-Hydroxy-4-aza-5-oxo-7-oxa-10,10,11,11, 12,12,13,13,14,14,15,15,16,16,17,17,18,18,19,19,19-henicosafluornonadecyl]-1,4,7-tris(carboxymethyl)-1,4,7-tris(carboxymethyl)-1,4,7,10-tetraazacyclododecan

10,63 g (17.09 mmol) der Titelverbindung aus Beispiel 32b und 1.97 g (18.79 mmol) N-Hydroxysuccinimid werden in einer Mischung aus 50 ml Dimethylformamid/50 ml Chloroform gelöst. Bei 0 °C gibt man 3.88 g (18.79 mmol) Dicyclohexylcarbodiimid zu und rührt 1 Stunde bei 0 °C, anschließend 3 Stunden bei Raumtemperatur. Man kühlt erneut auf 0 °C und gibt 5.19 g (51.27 mmol) Triethylamin/50 ml 2-Propanol zu. Anschließend werden 10.78 g (18.79 mmol) Gadolinium-Komplex von 10-(3-Amino-2-hydroxypropyl)-1.4.7-tris(carboxymethyl)-1.4.7.10-tetraazacyclododecan (WO 95/17451) gelöst in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylharnstoff ab. Das Filtrat wird zur Trockne eingedampft und durch RP-Chromatograpie gereinigt (RP-18/Laufmittel: Gradient aus Wasser/n-Propanol/Acetonitril).
Ausbeute 14,73 g (69 % der Theorie) eines farblosen glasigen Feststoffes.
Wassergehalt: 5,7 %

| Elementaranalyse (bezogen auf wasserfreie Substanz): | | | | | |
|---|---|---|---|---|---|
| ber. | C 31.61 | H 2.99 | F 33.87 | Gd 13.35 | N 5.95 |
| gef. | C 31.49 | H 3.15 | F 33.68 | Gd 13.21 | N 6.01 |

### Beispiel 33

### a) N-(2-Brompropionyl)glycin-benzylester

Zu 100 g (296,4 mmol) Glycinbenzylester-p-Toluolsulfonsäuresalz und 33,0 g (326,1 mmol) Triethylamin in 400 ml Methylenchlorid tropft man bei 0 °C 55,9 g (326,1 mmol) 2-Brompropionsäurechlorid zu. Man läßt die Temperatur nicht über 5 °C kommen. Nach beendeter Zugabe wird eine Stunde bei 0 °C gerührt, anschließend
2 Stunden bei Raumtemperatur. Man setzt 500 ml Eiswasser zu und stellt die Wasserphase mit 10 % aqu. Salzsäure auf pH 2. Die organische Phase wird abgetrennt, je einmal mit
300 ml 5 % aqu. Sodalösung und 400 ml Wasser gewaschen. Man trocknet die organische Phase über Magnesiumsulfat und dampft im Vakuum zur Trockne ein.
Der Rückstand wird aus Diisopropylether umkristallisiert.
Ausbeute: 68,51 g (75 % d. Th.) eines farblosen kristallinen Pulvers
Schmelzpunkt: 69-70°C

| Elementaranalyse: | | | | |
|---|---|---|---|---|
| ber. | C 48,02 | H 4,70 | N 4,67 | Br 26,62 |
| gef. | C 47,91 | H 4,82 | N 4,51 | Br 26,47 |

### b) 1-[4-(Benzyloxycarbonyl)-1-methyl-2-oxo-3-azabutyl]-1,4,7,10-tetraazacyclododecan

Zu 55,8 g (324,4 mmol) 1,4,7,10-Tetraazacyclododecan, gelöst in 600 ml Chloroform, gibt man 50 g (162,2 mmol) der Titelverbindung aus Beispiel 1a) und rührt über Nacht bei Raumtemperatur. Man gibt 500 ml Wasser zu, trennt die organische Phase ab und wäscht sie noch jeweils 2 mal mit 400 ml Wasser. Man trocknet die organische Phase über Magnesiumsulfat und dampft im Vakuum zur Trockne ein. Der Rückstand wird an Kieselgel chromatographiert (Laufmittel: Chloroform/Methanol/aqu. 25 % Ammoniak = 10/5/1).
Ausbeute. 40,0 g [63 % d. Th.bezogen auf eingesetztes 1a)] eines leicht gelblichen zähen Öls.

| Elementaranalyse: | | | |
|---|---|---|---|
| ber.: | C 61,36 | H 8,50 | N 17,89 |
| gef.: | C 61,54 | H 8,68 | N 17,68 |

### c) 10-[4-(Benzyloxycarbonyl)-1-methyl-2-oxo-3-azabutyl]-1,4,7-tris(tert.-butoxycarbonylmethyl)-1,4,7,10-tetraazacyclododecan (Natriumbromid-Komplex)

Zu 20 g (51,08 mmol) der Titelverbindung aus Beispiel 1b) und 17,91 (169 mmol) Natriumcarbonat in 300 ml Acetonitril gibt man 33 g (169 mmol) Bromessigsäure-tert.-butylester zu und rührt 24 Stunden bei 60 °C. Man kühlt auf 0 °C ab, filtriert von den Salzen ab und dampft das Filtrat zur Trockne ein. Der Rückstand wird an Kieselgel chromatographiert (Laufmittel: Essigsäureethylester/Ethanol: 15/1) Die das Produkt enthaltenden Fraktionen werden eingedampft und der Rückstand aus Diisopropylether umkristallisiert.
Ausbeute: 34,62 g (81 % d. Th.) eines farblosen kristallinen Pulvers
Schmelzpunkt: 116 - 117 °C

| Elementaranalyse: | | | | | |
|---|---|---|---|---|---|
| ber. | C 54,54 | H 7,59 | N 8,37 | Na 2,74 | Br 9,56 |
| gef. | C 54,70 | H 7,65 | N 8,24 | Na 2,60 | Br 9,37 |

### d) 10-(4-Carboxy-1-methyl-2-oxo-3-azabutyl)-1,4,7-tris(tert.-butoxy carbonylmethyl)-1,4,7,10-tetraazacyclododecan (Natriumbromid-Komplex)

30 g (35,85 mmol) der Titelverbindung aus Beispiel 1c werden in 500 ml Isopropanol gelöst und 3 g Palladiumkatalysator (10 % Pd/C) hinzugegeben. Man hydriert über Nacht bei Raumtemperatur. Es wird vom Katalysator abfiltriert, das Filtrat im Vakuum zur Trockne eingedampft und aus Aceton umkristallisiert.
Ausbeute: 22,75 g (85 % d. Th.) eines farblosen kristallinen Pulvers
Schmelzpunkt: 225 °C (Zers.)

| Elementaranalyse: | | | | | |
|---|---|---|---|---|---|
| ber. | C 49,86 | H 7,69 | N 9,38 | Na 3,07 | Br 10,71 |
| gef. | C 49,75 | H 7,81 | N 9,25 | Na 2,94 | Br 10,58 |

### e) 10-[1-Methyl-2-oxo-3 -aza-5-oxo-5- {4-perfluorooctylsulfonyl-piperazin-1-yl}-pentyl]-1,4,7-tris(carboxymethyl)-1,4,7,10-tetraazacyclododecan

10 g (13,39 mmol) der Titelverbindung aus Beispiel 33 d und 7,61 g (13.39 mmol) der Titelverbindung aus Beispiel 27a werden in 150 ml Tetrahydrofuran gelöst. Bei 0 °C gibt man 3,97 g (16.07 mmol) N-Ethoxycarbonyl-2-ethoxy-1,2-dihydrochinolin (EEDQ) zu, rührt 3 Stunden bei 0 °C, anschließend 12 Stunden bei Raumtemperatur. Man dampft im Vakuum zur Trockne ein. Der Rückstand wird in 150 ml Trifluoressigsäure aufgenommen und 12 Stunden bei Raumtemperatur gerührt. Es wird zur Trockne eingedampft, der Rückstand in Wasser gelöst und mit 10 %iger wässriger Natronlauge auf pH 3.2 eingestellt. Zur Reinigung wird an RP-18 chromatographiert (Gradient aus Wasser/Acetonitril/Tetrahydrofuran).
Ausbeute 9,67 g (63 % der Theorie) eines hygroskopischen Feststoffes.
Wassergehalt: 10,5 %

| Elementaranalyse (bezogen auf wasserfreie Substanz): | | | | | |
|---|---|---|---|---|---|
| ber. | C 36.30 | H 3.93 | N 9,56 | F 31.49 | S 3.13 |
| gef. | C 36.14 | H 3.98 | N 9.40 | F 31.67 | S 3.02 |

### f) Gadolinium-Komplex von 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan

5 g (4.87 mmol) der Titelverbindung aus Beispiel 33 e werden in 60 ml Wasser gelöst und 0.883 g (2.44 mmol) Gadolinium-oxid zugegeben. Man rührt 3 Stunden bei 90 °C. Die Lösung wird filtriert und das Filtrat gefriergetrocknet.
Ausbeute 6,47 g (quantitativ) eines voluminösen amorphen Pulvers..
Wassergehalt: 11,3 %

| Elementaranalyse (bezogen auf wasserfreie Substanz): | | | | | | |
|---|---|---|---|---|---|---|
| ber. | C 31.56 | H 3.16 | N 8.31 | F 27.37 | S 2,72 | Gd 13.33 |
| gef. | C 31.37 | H 3.35 | N 8.18 | F 27.19 | S 2.92 | Gd 13.05 |

### Beispiel 34

### a) 4-Perfluoroctansulfonylpiperazin-1-ylpentandiamsäure

Zu einer Suspension von 11 41 g (100.0 mmol) Glutarsäureanhydrid in 100 ml Tetrahydrofuran tropft man unter kräftigem Rühren bei 0 °C eine Lösung von 10.62 g (105.0 mmol) Triethylamin und 59.67 g (105.0 mmol) der Titelverbindung aus Beispiel 27a) in 50 ml Tetrahydrofuran und läßt über Nacht auf Raumtemperatur kommen. Man säuert das Reaktionsgemisch mit 100 ml 2N HCL an und extrahiert 3 mal mit 100 ml Tetrahydrofuran. Die vereinigten organischen Extrakte werden mit Natriumsulfat getrocknet, filtriert und eingeengt. Der Rückstand wird aus 2-Propanol/Ethylacetat umkristallisiert.
Ausbeute 52.30 g (73 % der Theorie) eines farblosen kristallinen Feststoffes.

| Elementaranalyse: | | | | | |
|---|---|---|---|---|---|
| ber. | C 29.92 | H 2.22 | N 4.11 | F 47.33 | S 4.70 |
| gef. | C 29.90 | H 2.18 | N 4.07 | F 47.42 | S 4.79 |

### b) Gadolinium-Komplex von 10-[2-Hydroxy-4-aza-5,9-dioxo-9-{4-perfluoroctyl)-piperazin-1-yl}-nonyl]-1,4,7-tris(carboxymethyl)-1,4,7-tris(carboxymethyl)-1,4,7,10-tetraazacyclododecan

11.66 g (17.09 mmol) der Titelverbindung aus Beispiel 34a und 1.97 g (18.79 mmol) N-Hydroxysuccinimid werden in einer Mischung aus 50 ml Dimethylformamid/50 ml Chloroform gelöst. Bei 0 °C gibt man 3.88 g (18.79 mmol) Dicyclohexylcarbodiimid zu und rührt 1 Stunde bei 0 °C, anschließend 3 Stunden bei Raumtemperatur. Man kühlt erneut auf 0°C und gibt 5.19 g (51.27 mmol) Triethylamin/50 ml 2-Propanol zu. Anschließend werden 10.78 g (18.79 mmol) Gadolinium-Komplex von 10-(3-Amino-2-hydroxypropyl)-1.4.7-tris(carboxymethyl)-1.4.7,10-tetraazacyclododecan (WO 95/17451) gelöst in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylharnstoff ab. Das Filtrat wird zur Trockne eingedampft und durch RP-Chromatograpie gereinigt (RP-18/Laufmittel: Gradient aus Wasser/n-Propanol/Acetonitril).
Ausbeute 16,7 g (73 % der Theorie) eines farblosen glasigen Feststoffes.
Wassergehalt: 7,5 %

| Elementaranalyse (bezogen auf wasserfreie Substanz): | | | | | | |
|---|---|---|---|---|---|---|
| ber. | C 32.99 | H 3.50 | F 26.09 | Gd 12.70 | N 7,92 | S 2.59 |
| gef. | C 32.75 | H 3.68 | F 25.88 | Gd 12.55 | N 7.84 | S 2.63 |

### Beispiel 35

### a) N-Benzylperfluoroctansulfonamid

Zu einer Mischung von 10.62 g (105.0 mmol) Triethylamin und 10.72 g (100.0 mmol) Benzylamin tropft man bei 80°C unter kräftigem Rühren 50.21 g (100.0 mmol) Perfluoroctansulfonylfluorid. Man rührt 2 Tage bei 80°C, versetzt das Rektionsgemisch mit 300 ml Wasser und extrahiert 3 mal mit Ethylacetat. Die vereinigten organischen Extrakte werden über Natriumsulfat getrocknet, filtriert und eingeengt. Der Rückstand wird an Kieselgel chromatographiert (Laufmittel: Dichlormethan/Methanol = 4/1).
Ausbeute 45.96 g (78% d.Th.) einer farblosen Flüssigkeit

| Elementaranalyse: | | | | | |
|---|---|---|---|---|---|
| ber. | C 30.57, | H 1.37, | N 2.38, | S 5.44, | F 54.81 |
| gef. | C 30.49 | H 1.30, | N 2,42, | S 5.50, | F 54.90 |

### b) N-Benzyl-N-(perfluoroctylsulfonyl)-aminoessigsäure-t.-butylester

22,4 g (37,94 mmol) der Titelverbindung aus Beispiel 35 a und 15.73 g (113,8 mmol) Kaliumcarbonat werden in 200 ml Aceton suspendiert und bei 60°C 14,80 g (75,87 mmol) Bromessigsäure-tert.-butylester zugetropft. Man rührt 3 Stunden bei 60°C. Man filtriert von den Salzen ab und dampft das Filtrat im Vakuum zur Trockne ein. Der Rückstand wird an Kieselgel chromatographiert (Laufmittel: Hexan/Dichlormethan/Aceton = 10/10/1). Nach Eindampfen der produkthaltigen Fraktionen kristallisiert man den Rückstand aus Methanol/Ether um.
Ausbeute: 24,02 g (90% d. Th.) eines wachsartigen farblosen Feststoffes

| Elementaranalyse: | | | | | |
|---|---|---|---|---|---|
| ber. | C 35.86, | H 2.58, | N 1.99, | S 4.56, | F 45.91 |
| gef. | C 35.67 | H 2.71, | N 2.13, | S 4.45, | F 45.83 |

### c) N-Benzyl-N-(perfluoroctylsulfonyl)-aminoessigsäure

20 g (28,43 mmol) der Titelverbindung aus Beispiel 35 b werden in 200 ml Trifluoressigsäure gelöst und über Nacht bei Raumtemperatur gerührt. Man dampft im Vakuum zur Trockne ein. Der Rückstand wird aus Methanol/Ether umkristallisiert.
Ausbeute: 17,48 g (95% d. Th.) eines farblosen kristallinen Feststoffes

| Elementaranalyse: | | | | | |
|---|---|---|---|---|---|
| ber. | C 31,54, | H 1.56, | N 2.16, | S 4.95, | F 49.89 |
| gef. | C 31.38 | H 1.70, | N 2,05, | S 4.87, | F 49.71 |

### d) Gadolinium-Komplex von 10-[2-Hydroxy-4-aza-5-oxo-7-aza-7-(perfluoroctylsulfonyl)-8-phenyl-octyl]-1,4,7-tris(carboxymethyl)-1,4,7,10-tetraazacyclododecan

11,06 g (17,09 mmol) der Titelverbindung aus Beispiel 35 c und 1,97 g (18.79 mmol) N-Hydroxysuccinimid werden in einer Mischung aus 50 ml Dimethylformamid/50 ml Chloroform gelöst. Bei 0°C gibt man 3,88 g (18,79 mmol) Dicyclohexylcarbodiimid zu und rührt 1 Stunde bei 0°C, anschließend 3 Stunden bei Raumtemperatur. Man kühlt erneut auf 0°C ab und gibt 5.19 g(51,27 mmol) Triethylamin/50 ml 2-Propanol zu. Anschließend werden 10,78 g (18.79 mmol) Gadolinium-Komplex von 10-(3-Amino-2-hydroxy-propyl)-1,4,7-tris(carboxymethyl)-1,4,7,10-tetraazacyclododecan (WO 95/17451) gelost in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylharnstoff ab. Das Filtrat wird zur Trockne eingedampft und durch RP-Chromatographie gereinigt (RP-18 Laufmittel: Gradient aus Wasser/n-Propanol/Acetonitril).
Ausbeute: 16,49 g (75% d. Th.) eines farblosen glasigen Feststoffes
Wassergehalt: 6,5%

| Elementaranalyse: | | | | | | |
|---|---|---|---|---|---|---|
| ber. | C 33,95, | H 3,18, | N 6.99, | S 2.67, | F 26,85 | Gd 13.07 |
| gef. | C 33,81 | H 3,24, | N 6.82, | S 2.54, | F 26,64 | Gd 12.91 |

### Beispiel 36

### a) N-Decylperfluoroctansulfonamid

Zu einer Mischung von 10.62 g (105.0 mmol) Triethylamin und 15.73 g (100.0 mmol) Decylamin tropft man bei 80°C unter kräftigem Rühren 50.21 g (100.0 mmol) Perfluoroctansulfonylfluorid. Man rührt 2 Tage bei 80°C, versetzt das Reaktionsgemisch mit 300 ml Wasser und extrahiert 3 mal mit Ethylacetat. Die vereinigten organischen Extrakte werden über Natriumsulfat getrocknet, filtriert und eingeengt. Der Rückstand wird an Kieselgel chromatographiert (Laufmittel: Dichlormethan/Methanol = 4/1).
Ausbeute: 43,48 g (68% d. Th.) einer farblosen viskosen Flüssigkeit

| Elementaranalyse: | | | | | |
|---|---|---|---|---|---|
| ber. | C 33.81, | H 3.47, | N 2.19, | S 5.02, | F 50.51 |
| gef. | C 33,71 | H 3.39, | N 2.15, | S 4.93, | F 50.31 |

### b) N-Decyl-N-(perfluoroctylsulfonyl)-aminoessigsäure-t.-butylester

24,26 g (37,94 mmol) der Titelverbindung aus Beispiel 36 a und 15,73 g (113,8 mmol) Kaliumcarbonat werden in 200 ml Aceton suspendiert und bei 60°C 14,80 g (75,87 mmol) Bromessigsäure-tert.-butylester zugetropft.Man ruhrt 3 Stunden bei 60°C. Man filtriert von den Salzen ab und dampft das Filtrat im Vakuum zur Trockne ein. Der Rückstand wird an Kieselgel chromatographiert (Laufmittel: Hexan/Dichlormethan/Aceton = 10/10/1). Nach Eindampfen der produkthaltigen Fraktionen kristallisiert man den Rückstand aus Methanol/Ether um.
Ausbeute: 24,87 g (87% d. Th.) eines wachsartigen farblosen Feststoffes

| Elementaranalyse: | | | | | |
|---|---|---|---|---|---|
| ber. | C 38,25, | H 4,28, | N 1,86, | S 4.26, | F 42,86 |
| gef. | C 38,09 | H 4,41, | N 1,74, | S 4.10, | F 42,67 |

### c) N-Decyl-N-(perfluoroctylsulfonyl)-aminoessigsäure

20 g (26,54 mmol) der Titelverbindung aus Beispiel 36 b werden in 200 ml Trifluoressigsäure gelöst und über Nacht bei Raumtemperatur gerührt. Man dampft im Vakuum zur Trockne ein. Der Rückstand wird aus Methanol/Ether umkristallisiert.
Ausbeute: 17,22 g (93% d. Th.) eines farblosen cristallinen Feststoffes

| Elementaranalyse: | | | | | |
|---|---|---|---|---|---|
| ber. | C 34,44, | H 3,47, | N 2,01, | S 4.61 | F 46,31 |
| gef. | C 34,28 | H 3,30, | N 1,95, | S 4.65 | F 46,28 |

### d) Gadolinium-Komplex von 10-[2-Hydroxy-aza-5-oxo-7-aza-7-(perfluoroctylsulfonyl)-heptadecyl]-1,4,7-triscarboxymethyl)-1,4,7,10-tetraazacyclododecan

11,92 g (17,09 mmol) der Titelverbindung aus Beispiel 36 c und 1,97 g (18.79 mmol) N-Hydroxysuccinimid werden in einer Mischung aus 50 ml Dimethylformamid/50 ml Chloroform gelöst. Bei 0°C gibt man 3,88 g (18,79 mmol) Dicyclohexylcarbodiimid zu und rührt 1 Stunde bei 0°C, anschließend 3 Stunden bei Raumtemperatur. Man kuhlt erneut auf 0°C to und gibt 5.19 g(51,27 mmol) Triethylamin/50 ml 2-Propanol zu. Anschließend werden 10,78 g (18.79 mmol) Gadolinium-Komplex von 10-(3-Amino-2-hydroxy-propyl)-1,4,7-tris(carboxymethyl)-1,4,7,10-tetraazacyclododecan (WO 95/17451) gelöst in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylharnstoff ab. Das Filtrat wird zur Trockne eingedampft und durch RP-Chromatographie gereinigt (RP-18 Laufmittel: Gradient aus Wasser/n-Propanol/Acetonitril).
Ausbeute: 16,76 g (71% d. Th.) eines farblosen glasigen Feststoffes
Wassergehalt: 6,5%

| Elementaranalyse: | | | | | | |
|---|---|---|---|---|---|---|
| ber. | C 35,46, | H 4,18, | N 6.71, | S 2.5, | F 25,77 | Gd 12,55 |
| gef. | C 35,28 | H 4,33, | N 6.80, | S 2.6, | F 25,65 | Gd 12.41 |

### Beispiel 37

### a) N-Hexylperfluoroctansulfonamid

Zu einer Mischung von 10,62 g (105.0 mmol) Triethylamin und 10,12 g (100.0 mmol) Benzylamin tropft man bei 80°C unter kräftigem Rühren 50.21 g (100.0 mmol) Perfluoroctansulfonylfluorid. Man rührt 2 Tage bei 80°C, versetzt das Rektionsgemisch mit 300 ml Wasser und extrahiert 3 mal mit Ethylacetat. Die vereinigten organischen Extrakte werden über Natriumsulfat getrocknet, filtriert und eingeengt. Der Rückstand wird an Kieselgel chromatographiert (Laufmittel: Dichlormethan/Methanol = 4/1).
Ausbeute: 45.50 g (78% d.Th.) einer farblosen Flüssigkeit

| Elementaranalyse: | | | | | |
|---|---|---|---|---|---|
| ber. | C 28,83, | H 2,42, | N 2.40, | S 5.50, | F 55,37 |
| gef. | C 28,29 | H 2,39, | N 2.44, | S 5.55, | F 55,50 |

### b) N-Hexyl-N-(perfluoroctylsulfonyl)-aminoessigsaure-t.-butylester

22,13 g (37,94 mmol) der Titelverbindung aus Beispiel 37 a und 15.73 g (113,8 mmol) Kaliumcarbonat werden in 200 ml Aceton suspendiert und bei 60°C 14,80 g (75,87 mmol) Bromessigsäure-tert.-butylester zugetropft. Man ruhrt 3 Stunden bei 60°C. Man filtriert von den Salzen ab und dampft das Filtrat im Vakuum zur Trockne ein. Der Rückstand wird an Kieselgel chromatographiert (Laufmittel: Hexan/Dichlormethan/Aceton = 10/10/1). Nach Eindampfen der produkthaltigen Fraktionen, kristallisiert man den Rückstand aus Methanol/Ether um.
Ausbeute: 23,02 g (87% d. Th.) eines wachsartigen farblosen Feststoffes

| Elementaranalyse: | | | | | |
|---|---|---|---|---|---|
| ber. | C 34,44, | H 3,47, | N 2,01, | S 4.60, | F 46,31 |
| gef. | C 34,31 | H 3,61, | N 1,97, | S 4.65, | F 46,25 |

### c) N-Hexyl-N-(perfluoroctylsulfonyl)-aminoessigsäure

20 g (28,43 mmol) der Titelverbindung aus Beispiel 37 b werden in 200 ml Trifluoressigsäure gelöst und über Nacht bei Raumtemperatur gerührt. Man dampft im Vakuum zur Trockne ein. Der Rückstand wird aus Methanol/Ether umkristallisiert.
Ausbeute: 16,74 g (91% d. Th.) eines farblosen kristallinen Feststoffes

| Elementaranalyse: | | | | | |
|---|---|---|---|---|---|
| ber. | C 29,96, | H 2,51, | N 2.18, | S 5,00, | F 50,36 |
| gef. | C 29,87 | H 2.70, | N 2.05, | S 4.84, | F 50,17 |

### d) Gadolinium-Komplex von 10-[2-Hydroxy-4-aza-5-oxo-7-aza-7-(perfluoroctylsulfonyl)-tridecyl]-1,4,7-tris(carboxymethyl)-1,4,7,10-tetraazacyclododecan

10,96 g (17,09 mmol) der Titelverbindung aus Beispiel 37 c und 1,97 g (18.79 mmol) N-Hydroxysuccinimid werden in einer Mischung aus 50 ml Dimethylformamid/50 ml Chloroform gelöst. Bei 0°C gibt man 3,88 g (18,79 mmol) Dicyclohexylcarbodiimid zu und rührt 1 Stunde bei 0°C, anschließend 3 Stunden bei Raumtemperatur. Man kühlt erneut auf 0°C ab und gibt 5.19 g(51,27 mmol) Triethylamin/50 ml 2-Propanol zu. Anschließend werden 10,78 g (18.79 mmol) Gadolinium-Komplex von 10-(3-Amino-2-hydroxy-propyl)-1,4,7-tris(carboxymethyl)-1,4,7,10-tetraazacyclododecan (WO 95/17451) gelöst in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylharnstoff ab. Das Filtrat wird zur Trockne eingedampft und durch RP-Chromatographie gereinigt (RP-18 Laufmittel: Gradient aus Wasser/n-Propanol/Acetonitril).
Ausbeute: 16,46 g (75% d. Th.) eines farblosen glasigen Feststoffes
Wassergehalt: 6,8%

| Elementaranalyse: | | | | | | |
|---|---|---|---|---|---|---|
| ber. | C 33,11, | H 3,70, | N 7,02, | S 2.68, | F 26,98 | Gd 13.14 |
| gef. | C 33,01 | H 3,84, | N 6.95, | S 2.57, | F 26,85 | Gd 13,03 |

### Beispiel 38

### a) 11-[N-Ethyl-N-(perfluoroctylsulfonyl)-amino]-hexansäurebenzylester

20 g (37,94 mmol) N-Ethyl-N-perfluoroctylsulfonylamid und 15.73 g (113,8 mmol) Kaliumcarbonat werden in 200 ml Aceton suspendiert und bei 60°C 21,64 g (75,87 mmol) 6-Bromhexansäurebenzylester zugetropft. Man rührt 3 Stunden bei 60°C. Man filtriert von den Salzen ab und dampft das Filtrat im Vakuum zur Trockne ein. Der Rückstand wird an Kieselgel chromatographiert (Laufmittel: Hexan/Dichlormethan/Aceton = 10/10/1). Nach Eindampfen der produkthaltigen Fraktionen, kristallisiert man den Rückstand aus Methanol/Ether um.
Ausbeute: 25,26 g (91% d. Th.) eines farblosen kristallinen Pulvers

| Elementaranalyse: | | | | | |
|---|---|---|---|---|---|
| ber. | C 37,77, | H 3,03, | N 1.91, | S 4.38, | F 44,15 |
| gef. | C 37,61 | H 3,18, | N 1,84, | S 4.27, | F 44,01 |

### b) 11-[N-Ethyl-N-(perfluoroctylsulfonyl)-amino]-hexansäure

20 g (27,34 mmol) der Titelverbindung aus Beispiel 38 b werden in 300 ml Isopropanol/200 ml Dichlormethan gelöst und 3 g Palladiumkatalysator (10% Pd/C) zugegeben. Man hydriert über Nacht bei Raumtemperatur. Es wird vom Katalysator abfiltriert und das Filtrat im Vakuum zur Trockne eingedampft. Der Rückstand wird aus Ether/Hexan umkristallisiert.
Ausbeute: 16,13 g (92% d. Th.) eines farblosen kristallinen Feststoffes

| Elementaranalyse: | | | | | |
|---|---|---|---|---|---|
| ber. | C 29,96, | H 2.51, | N 2,18, | S 5,00, | F 50,36 |
| gef. | C 29,81 | H 2.70, | N 2.09, | S 4.93, | F 50,14 |

### d) Gadolinium-Komplex von 10-[2-Hydroxy-4-aza-5-oxo-11-aza-11-(perfluoroctylsulfonyl)-tridecyl]-1,4,7-tris(carboxymethyl)-1,4,7,10-tetraazacyclododecan

10,96 g (17,09 mmol) der Titelverbindung aus Beispiel 38 b und 1,97 g (18.79 mmol) N-Hydroxysuccinimid werden in einer Mischung aus 50 ml Dimethylformamid/50 ml Chloroform gelöst. Bei 0°C gibt man 3,88 g (18,79 mmol) Dicyclohexylcarbodiimid zu und rührt 1 Stunde bei 0°C, anschließend 3 Stunden bei Raumtemperatur. Man kühlt erneut auf 0°C ab und gibt 5.19 g(51,27 mmol) Triethylamin/50 ml 2-Propanol zu. Anschließend werden 10,78 g (18.79 mmol) Gadolinium-Komplex von 10-(3-Amino-2-hydroxy-propyl)-1,4,7-tris(carboxymethyl)-1,4,7,10-tetraazacyclododecan (WO 95/17451) gelöst in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylharnstoff ab. Das Filtrat wird zur Trockne eingedampft und durch RP-Chromatographie gereinigt (RP-18 Laufmittel: Gradient aus Wasser/n-Propanol/Acetonitril).
Ausbeute: 15,0 g (69% d. Th.) eines farblosen glasigen Feststoffes
Wassergehalt: 5,9%

| Elementaranalyse: | | | | | | |
|---|---|---|---|---|---|---|
| ber. | C 33,11, | H 3,70, | N 7,02, | S 2.68, | F 26,98 | Gd 13.14 |
| gef. | C 33,01 | H 3,83, | N 6.91, | S 2.49, | F 26,83 | Gd 13,05 |

### Beispiel 39

### Bluteliminationskinetik von Kontrastmitteln

Die Blut-Eliminationskinetik von Kontrastmitteln wurde an Ratten (Han. Wistar, Schering SPF, ≈ 250 g Körpergewicht) untersucht. Hierfür wurde nach einmaliger intravenöser Applikation (via einer Caudalvene) der Substanzen (Dosis: 50 - 100 µmol Me pro kg Körpergewicht) die Substanzkonzentration im Blut (basierend auf dem Gd- bzw. Dy-Gehalt) über einen Zeitraum bis zu 300 Minuten p. i. mittels ICP-AES bestimmt. Die pharmakokinetischen Parameter: Verteilungsvolumen (Vss), Gesamtclearance (CLtot) und Eliminationshalbwertszeit (tβ) wurden mit einem speziellen Computerprogramm (TOPFIT 2.0; Thomae, Schering, Gödecke) berechnet, wobei ein Ein- bzw. Zwei-Kompartment-Distributionsmodell zu Grunde gelegt wurde.

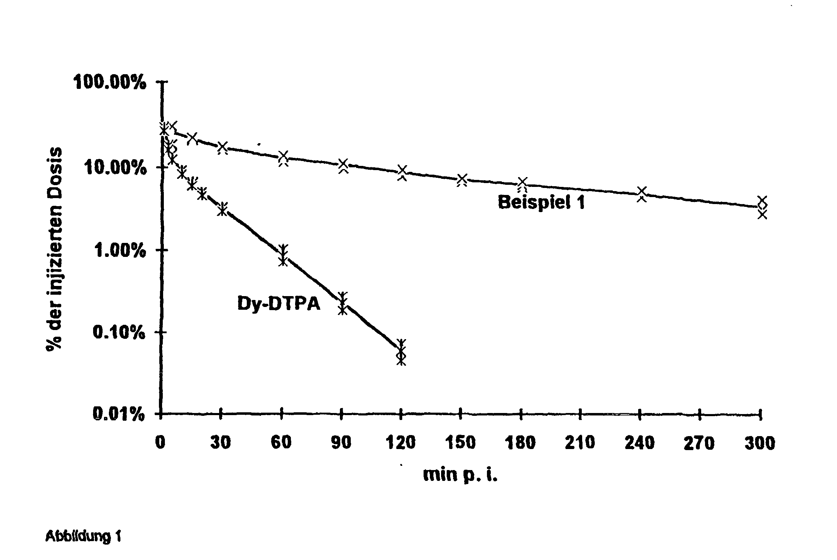

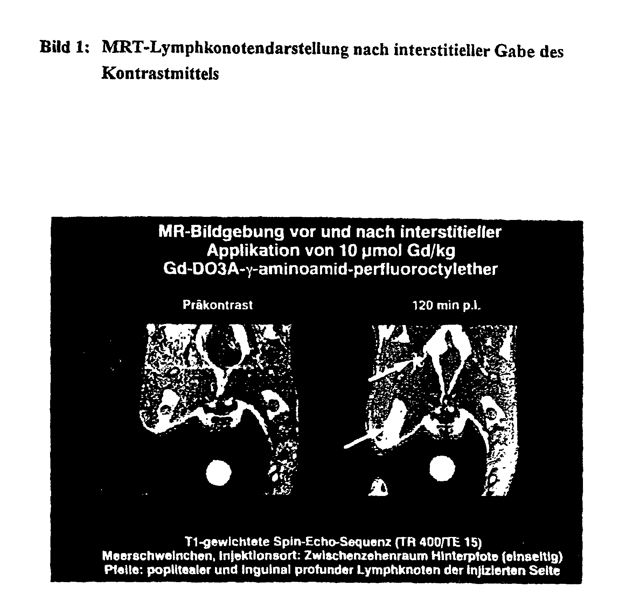

Im Vergleich zu Dy-DTPA (dem Dysprosium-Analogon von Magnevist® ) zeigten die erfindungsgemäßen Fluorverbindungen (z. B. Beispiel 1c) eine deutlich langsamere Elimination aus dem Blut und außerdem ein kleineres Verteilungsvolumen (siehe auch Abbildung 1 und Tabelle 1)

Es ist festzustellen, daß diese Verbindungen überraschenderweise eine verlängerte Retention im Blutraum haben und daher als "blood pool Kontrastmittel" - z. B. zur Darstellung von Blutgefäßen mit geeigneten Techniken - auch in relativ kleinen Dosierungen von < 50 µmol Gd pro kg Körpergewicht geeignet sind.

### Abbildung 1:

Elimination aus dem Blut (in % der injizierten Dosis) von Dy-DTPA (Dosis: 100 µmol Dy pro kg Körpergewicht, n=3) und von der erfindungsgemäßen Fluorverbindung des Beispiels 1c (Dosis: 50 µmol Gd pro kg Körpergewicht, n=2) nach einmaliger intravenöser Applikation der Substanzen bei Ratten (Han Wistar, Schering SPF, ≈ 250 g Körpergewicht).

Die Gd- und Dy-Gehalte im Blut wurden mittels ICP-AES bestimmt.

**Tabelle 1:**

| Pharmakokinetikparameter: Verteilungsvolumen (Vss), Gesamtclearance (CLtot) und Eliminationshalbwertszeit (tβ) von Dy-DTPA und der erfindungsgemäßen Fluorverbindung des Beispiels 1c. (Berechnet mit TOPFIT 2.0; Ein- bzw. Zwei-Kompartmentmodell). | | | | | | |
|---|---|---|---|---|---|---|
| | **Vss (l/kg)** | | **CL tot** | **(ml/(min*kg))** | **t b (min)** | |
| | **MEAN** | **SD** | **MEAN** | **SD** | **MEAN** | **SD** |
| **Dy-DTPA** | 0.17 | 0.00 | 9.27 | 0.60 | 14.98 | 0.73 |
| **Beispiel 1c** | 0.14 | 0.02 | 1.07 | 0.09 | 95.01 | 10.37 |

Weitere Einzelheiten siehe Text zur Abbildung 1

### Beispiel 40

### Lymphknotenanreicherung am Meerschweinchen

Verschiedene fluorhaltige Gadolinium- und Mangan-Komplexe wurden 90 Minuten bis 24 Stunden nach subkutaner Gabe (2,5-10 µmol Gesamtgadolinium/kg Körpergewicht, Hinterpfote s.c.) an stimulierten Meerschweinchen (komplettes Freund-Adjuvant; jeweils 0,1 ml i.m. in den rechten und linken Ober- und Unterschenkel; 2 Wochen vor Gabe der Prüfsubstanzen) hinsichtlich ihrer Lymphknotenanreicherung in drei aufeinanderfolgenden Lymphknotenstationen (popliteal, inguinal, iliakal) untersucht. Hierbei wurden die nachfolgend in Tabelle 2 aufgelisteten Ergebnisse (Ermittlung der Gadolinium-Konzentration mittels ICP-AES) erhalten:

**Tabelle 2**

| **Substanz Beispiel Nr.** | **Zeitpunkt der Lymphknote n-entnahme (Dosis)** | **Gadolinium- bzw. Mangan-Konzentration in drei aufeinanderfolgenden Lymphknotenstationen [µmol/l] [% Dosis/g Gewebe]** | | | |
|---|---|---|---|---|---|
| | | **Popliteal** | **Inguinal** | **Iliakal** | **Verhältnis** |
| 1c) | 4 h (2,5 µmol/kg) | 120 µmol/l 17,2 % | 29 µmol/l 4,2 % | 40 µmol/l 5,6 % | 10 : 2,4 : 3,3 |
| 2c) | 4 h (10 µmol/kg) | 435 µmol/l 10,5 % | 84 µmol/l 2,0 % | 150 µmol/l 3,6 % | 10 : 2,0 : 3,5 |
| 1e) | 90 min (10 µmol/kg | 559 µmol/l 15,0 % | 224 µmol/l 6,0 % | 290 µmol/l 7,8 % | 10 : 4,0 : 5,2 |
| 3c) | 90 min (10 µmol/kg) | 880 µmol/l 21,4 % | 277 µmol/l 6,7 % | 339 µmol/l 8,3 % | 10 : 3,1 : 3,9 |

Die Tabelle 2 zeigt, daß eine hohe Kontrastmittelanreicherung über drei aufeinanderfolgende Lymphknotenstationen zu verzeichnen ist.

### Beispiel 41

### Lymphknotendarstellung (MRT) nach interstitieller Gabe des Kontrastmittels

Das Bild 1 zeigt MR-Aufnahmen von poplitealen und inguinalen Lymphknoten sowohl vor (linke Seite: Präkontrast), als auch 120 Minuten nach (rechte Seite) subkutaner Applikation (Meerschweinchen, Hinterpfote, Zwischenzehenraum) vom Gd-Komplex aus Beispiel 2c (in der Abbildung als Gd-DO3A-g-aminoamidperfluoroctylether bezeichnet) (10 µmol Gd/kg Körpergewicht). Die T¹-gewichteten Spin-Echo-Aufnahmen (TR 400 ms, TE 15 ms) verdeutlichen den starken Signalanstieg in den poplitealen und inguinalen Lymphknoten der injizierten (gerader Pfeil) im Vergleich zur nicht-injizierten (gebogener Pfeil) Körperseite, bzw. zum Präkontrast-Bild.

### Beispiel 42

### Ausscheidung des Kontrastmittels nach i.p. Gabe

Nach Gabe eines erfindungsgemäßen perfluorierten Gadoliniumkomplexes (100 µmol Gesamtgadolinium/kg Körpergewicht) in den

Intraperitonealraum der Ratte wurde 14 Tage nach Applikation die Retention des Metalls in der Leber sowie im Restkörper untersucht. Bei diesem Versuch wurde die fluorhaltige Verbindung 2c) verwendet. Nach 14 d p.i. betrug die Gadoliniumkonzentration in der Leber 0,22% und im Restkörper 1,1% der applizierten Dosis.

Im Vergleich dazu wird Gd-DPTA-Polylysin als polymeres Material nicht vollständig ausgeschieden. Nach 14 d sind noch 7 % der Anfangsdosis im Körper enthalten.

### Beispiel 43

### Bestimmung der T¹-Relaxivity ausgewählter Verbindungen

Die Relaxivity der folgenden Verbindungen wurde mit einem Minispec pc 20 (20 MHz, 0,47T) bei 37 °C in Wasser und Humanplasma bestimmt und mit der von Gd-DTPA-Polylysin und Magnevist® als Vergleichssubstanzen verglichen.

**Tabelle 3**

| | **R**^{**1**} **[L/mmol*sec] bei 0.47 T und 37° C** | |
|---|---|---|
| **Substanz Beispiel Nr.** | **Wasser** | **Plasma** |
| 1c) | 41 | 49 |
| 2c) | 19 | 33 |
| 3c) | 15,2 | 27,5 |
| 22f) | 6,9 | 20,5 |
| 30c) | 21,1 | 26,9 |
| 31c) | 5,2 | 29,1 |
| 32c) | 19,4 | 24,8 |
| 33f) | 31,5 | 35,7 |
| 34b) | 25,9 | 24,9 |
| 35d) | 23,1 | 34,0 |
| 37d) | 19,9 | n.b. |
| 38c) | 23,3 | 30,5 |

| **Vergleichssubstanzen:** | | |
|---|---|---|
| Magnevist® | 3,8 | 4,8 |
| Gd-DTPA-Polylysin ¹⁾ | 13,1 | 16,8 |

| | | |
|---|---|---|
| n.b. = nicht bestimmt 1) aus Invest. Radiol. 1992, 346 | | |

## Patentansprüche

1. Perfluoralkylhaltige Verbindungen der allgemeinen Formel I
R^{F}-L-A I
worin
R^{F} eine perfluorierte, geradkettige oder verzweigte Kohlenstoffkette mit der Formel -CₙF₂ₙX ist, in der
X ein endständiges Fluor-, Chlor-, Brom-, Jod- oder Wasserstoffatom darstellt und n für die Zahlen 4 - 30 steht,
L eine direkte Bindung, eine Methylengruppe, eine -NHCO-Gruppe, eine Gruppe 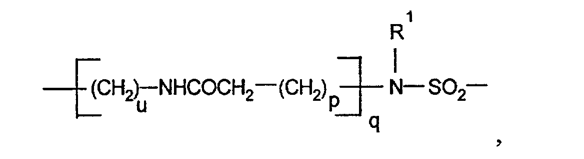 wobei p die Zahlen 0 bis 10, q und u unabhängig voneinander die Zahlen 0 oder 1 und
R¹ ein Wasserstoffatom, eine Methylgruppe, eine -CH₂-OH-Gruppe, eine -CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen, eine Gruppe -SO₃H- bedeuten,
oder eine geradkettige, verzweigte, gesättigte oder ungesättigte C₂-C₃₀-Kohlenstoffkette ist, die gegebenenfalls 1 bis 10 Sauerstoffatome, 1 bis 3 - NR¹-Gruppen, 1 bis 2 Schwefelatome, ein Piperazin, eine -CONR¹-Gruppe, eine -NR¹CO-Gruppe, eine -SO₂-Gruppe, eine -NR¹-CO₂-Gruppe, 1 bis 2 - CO-Gruppen, eine Gruppe 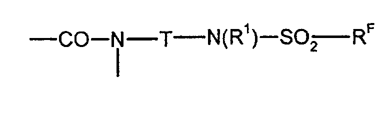 oder 1 bis 2 gegebenenfalls substituierte Aryle enthält und/oder durch diese Gruppen unterbrochen ist, und/oder gegebenenfalls substituiert ist mit 1 bis 3 -OR¹-Gruppen, 1 bis 2 Oxogruppen, 1 bis 2 -NH-COR¹-Gruppen, 1 bis 2 -CONHR¹-Gruppen, 1 bis 2 -(CH₂)ₚ-CO₂H-Gruppen, 1 bis 2 Gruppen -(CH₂)ₚ-(O)_{q}-CH₂CH₂-R^{F},
wobei
R¹, R^{F} und p und q die oben angegebenen Bedeutungen haben und
T eine C₂-C₁₀-Kette bedeutet, die gegebenenfalls durch 1 bis 2 Sauerstoffatome oder 1 bis 2 -NHCO-Gruppen unterbrochen ist,
A für einen Komplexbildner oder Metallkomplex oder deren Salze organischer und/oder anorganischer Basen oder Aminosäuren oder Aminosäureamide steht, und zwar für einen Komplexbildner oder Komplex der allgemeinen Formel II 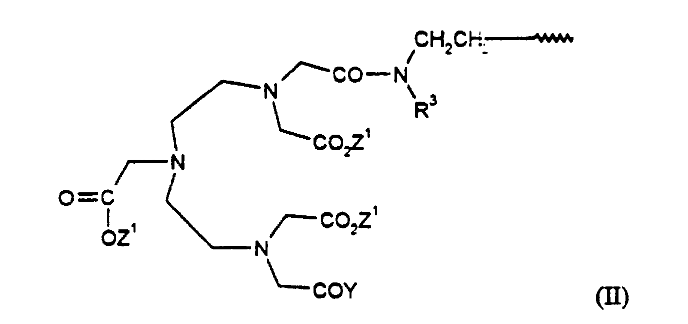 in der R³, Z¹ und Y unabhängig voneinander sind und
R³ die Bedeutung von R¹ hat oder -(CH₂ₘ-L-R^{F} bedeutet, wobei m 0, 1 oder 2 ist und L und R^{F} die o.g. Bedeutung haben,
Z¹ unabhängig voneinander ein Wasserstoffatom oder ein Metallionenäquivalent der Ordnungszahlen 21-29, 39, 42, 44 oder 57 - 83 bedeutet,
Y -OZ¹ oder 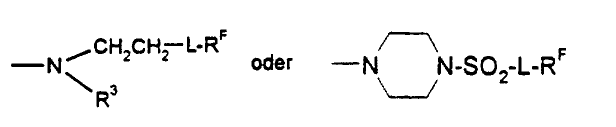 bedeutet, wobei Z^{1,} L, R^{F} und R³ die o. g. Bedeutungen haten,
oder
für einen Komplexbildner oder Komplex der allgemeinen Formel III 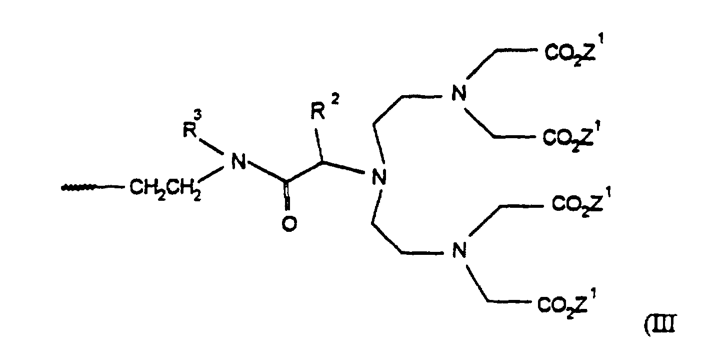 in der R³ und Z¹ die oben genannten Bedeutungen aufweisen und R² die Bedeutung von R¹ hat
oder
für einen Komplexbildner oder Komplex der allgemeinen Formel IV 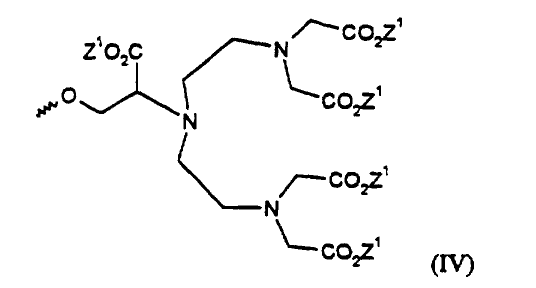 in der Z¹ die oben genannte Bedeutung hat
oder
für einen Komplexbildner oder Komplex der allgemeinen Formel V 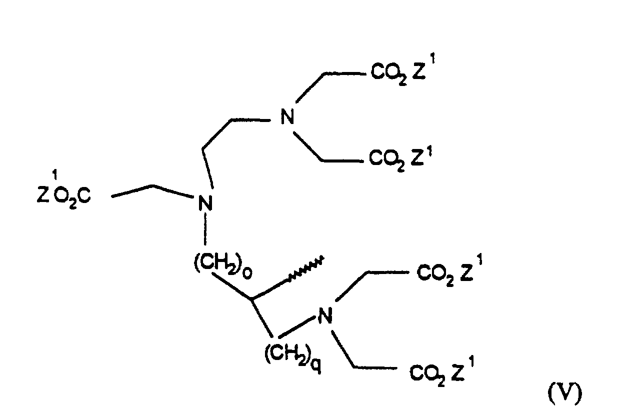 in der Z¹ die oben genannte Bedeutung hat und o und q für die Ziffern
O oder 1 stehen und die Summe o + q = 1 ergibt,
oder
für einen Komplexbildner oder Komplex der allgemeinen Formel VI 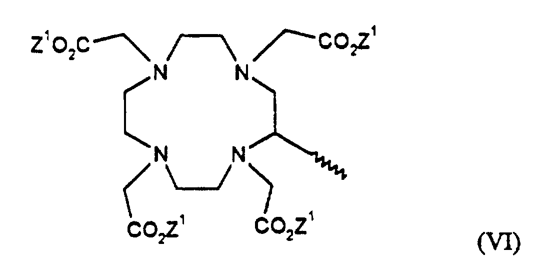 in der Z¹ die oben genannte Bedeutung hat
oder
für einen Komplexbildner oder Komplex der allgemeinen Formel VII 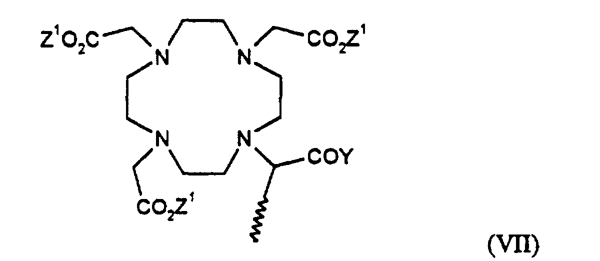 in der Z¹ und Y die oben genannten Bedeutungen haben
oder
für einen Komplexbildner oder Komplex der allgemeinen Formel VIII 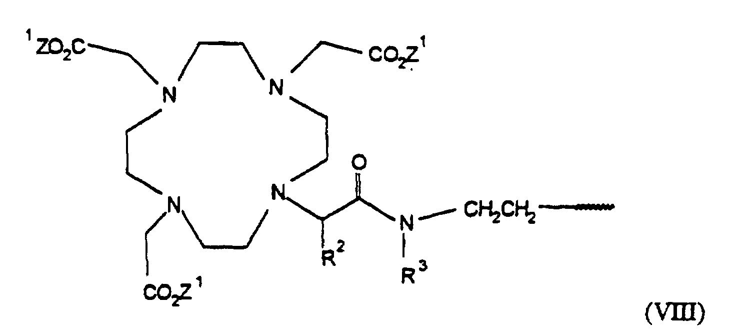 in der R³ und Z¹ die oben genannten Bedeutungen haben und R² die o.g. Bedeutung von R¹ hat.
oder
für einen Komplexbildner oder Komplex der allgemeinen Formel IX 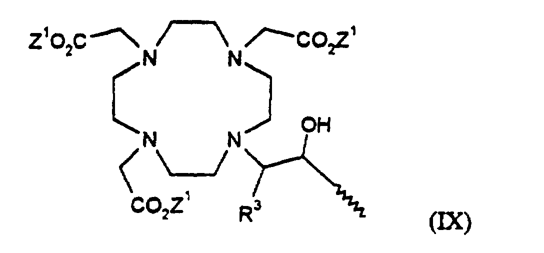 in der R³ und Z¹ die oben genannten Bedeutungen haben,
oder
für einen Komplexbildner oder Komplex der allgemeinen Formel X 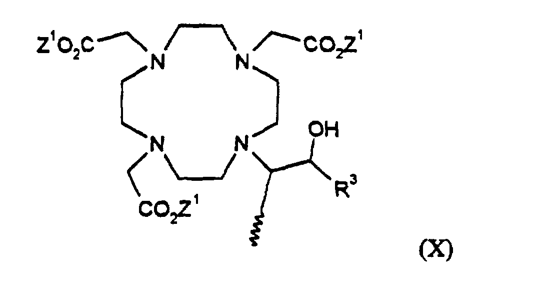 in der R³ und Z¹ die oben genannten Bedeutungen haben,
oder
für einen Komplexbildner oder Komplex der allgemeinen Formel XI 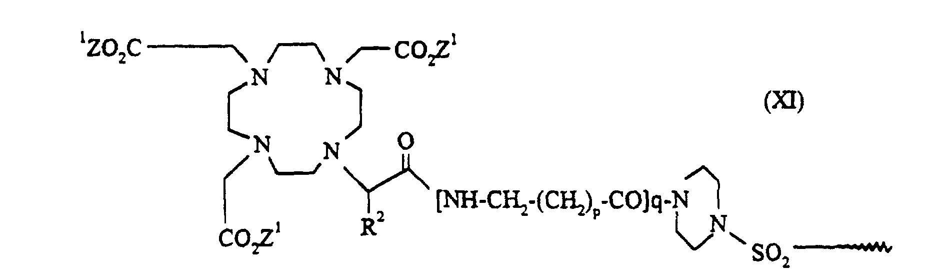 in der Z¹ p und q die oben genannte Bedeutung haben und R² die Bedeutung von R¹ hat.
oder
für einen Komplexbildner oder Komplex der allgemeinen Formel XII 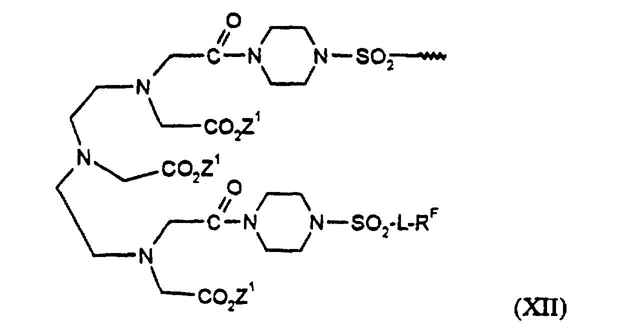 in der L, R^{F} und Z¹ die oben genannten Bedeutungen haben,
oder
für einen Komplexbildner oder Komplex der allgemeinen Formel XIII 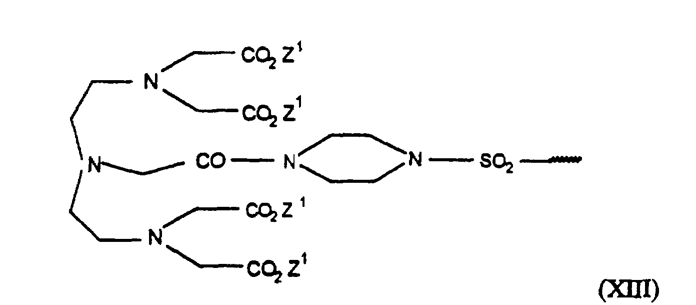 in der Z¹ die oben genannte Bedeutung hat.

2. Verbindungen gemäß Anspruch 1, **dadurch gekennzeichnet, daß** Z¹ für ein Wasserstoffatom steht.

3. Verbindungen gemäß Anspruch 1 oder 2, **dadurch gekennzeichnet, daß** n in der Formel -CₙF₂ₙ X für die Zahlen 4 - 15 steht.

4. Verbindungen gemäß einem der Ansprüche 1 bis 3, **dadurch gekennzeichnet, daß** X in der Formel -CₙF₂ₙ X ein Fluoratom bedeutet.

5. Verbindungen gemäß einem der Ansprüche 1 bis 4, **dadurch gekennzeichnet, daß** L für
-CH₂-
-CH₂CH₂-
-(CH₂)ₛ- s = 3 - 15
-CH₂-O-CH₂CH₂-
-CH₂-(O-CH₂-CH₂-)ₜ t = 2 - 6
-CH₂-NH-CO-
-CH₂-NH-CO-CH₂-N(CH₂COOH)-SO₂-
-CH₂-NH-CO-CH₂-N(C₂H₅)-SO₂-
-CH₂-NH-CO-CH₂-N(C₁₀H₂₁)-SO₂-
-CH₂-NH-CO-CH₂-N(C₆H₁₃)-SO₂-
-CH₂-NH-CO-(CH₂)₁₀-N(C₂H₅)-SO₂-
-CH₂-NH-CO-CH₂-N(-CH₂-C₆H₅)-SO₂-
-CH₂-NH-CO-CH₂-N(-CH₂-CH₂-OH)SO₂-
-CH₂-NHCO-(CH₂)₁₀-S-CH₂CH₂-
-CH₂NHCOCH₂-O-CH₂CH₂-
-CH₂NHCO(CH₂)₁₀-O-CH₂CH₂-
-CH₂-C₆H₄-O-CH₂CH₂-
-CH₂-O-CH₂-C(CH₂-OCH₂CH₂-C₆F₁₃)₂-CH₂-OCH₂-CH₂- 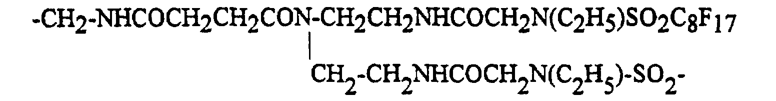
-CH₂-O-CH₂-CH(OC₁₀H₂₁)-CH₂-O-CH₂CH₂-
-(CH₂NHCO)₄-CH₂O-CH₂CH₂-
-(CH₂NHCO)₃-CH₂O-CH₂CH₂-
-CH₂-OCH₂C(CH₂OH)₂-CH₂-O-CH₂CH₂- 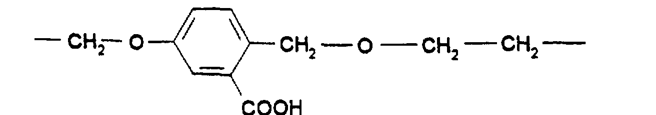
-CH₂NHCOCH₂N(C₆H₅)-SO₂-
-NHCO-CH₂-CH₂-
-NHCO-CH₂-O-CH₂CH₂-
-NH-CO-
-NH-CO-CH₂-N(CH₂COOH)-SO₂-
-NH-CO-CH₂-N(C₂H₅)-SO₂-
-NH-CO-CH₂-N(C₁₀H₂₁)-SO₂-
-NH-CO-CH₂-N(C₆H₁₃)-SO₂-
-NH-CO-(CH₂)₁₀-N(C₂H₅)-SO₂-
-NH-CO-CH₂-N(-CH₂-C₆H₅)-SO₂-
-NH-CO-CH₂-N(-CH₂-CH₂-OH)SO₂-
-NH-CO-CH₂-
-CH₂-O-C₆H₄-O-CH₂-CH₂-
-CH₂-C₆H₄-O-CH₂-CH₂-
-N(C₂H₅)-SO₂-
-N(C₆H₅)-SO₂-
-N(C₁₀H₂₁)-SO₂-
-N(C₆H₁₃)-SO₂-
-N(C₂H₄OH)-SO₂-
-N(CH₂COOH)-SO₂-
-N(CH₂C₆H₅)-SO₂-
-N-[CH(CH₂OH)₂]-SO₂-
-N-[CH(CH₂OH)CH(CH₂OH)]-SO₂-
steht.

6. Die Verbindungen gemäß Anspruch 1:
Gadolinium-Komplex von 10-[2-Hydroxy-4-aza-5-oxo-7-aza-7-(perfluoroctylsulfonyl)-nonyl]-1,4,7-tris(carboxymethyl)-1,4,7,10-tetraazacyclododecan,
Gadolinium-Komplex von 10-[2-Hydroxy-4-aza-5-oxo-7-oxa-10,10,11,11,12,12,13,13,14,14, 15,15,16,16,17,17,17-heptadecafluor-heptadecyl]-1,4,7-tris(carboxymethyl)-1,4,7,10-tetraazacyclododecan.

7. Verfahren zur Herstellung von perfluoralkylhaltigen Verbindungen der allgemeinen Formel I, **dadurch gekennzeichnet, daß** man
a) Verbindungen der allgemeinen Formel I mit A in der Bedeutung der allgemeinen Formel IX herstellt, indem man Verbindungen der allgemeinen Formel 20 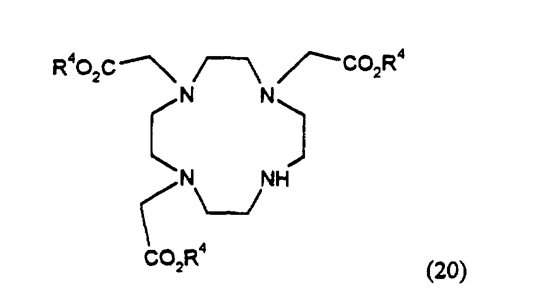 in der
R⁴ Wasserstoff, Methyl, Ethyl, Isopropyl, t-Butyl oder Benzyl bedeutet
mit Epoxiden der allgemeinen Formel 21 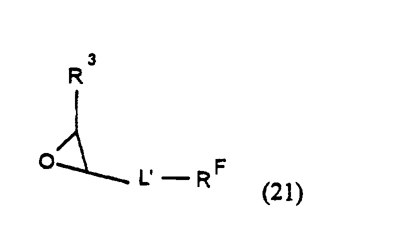 in der
R³ die Bedeutung von R¹, gegebenenfalls in geschützter Form, hat oder - (CH₂)ₘ-L-R^{F} bedeutet, wobei m 0, 1 oder 2 sein kann, L' die Bedeutung von L hat, gegebenenfalls in geschützter Form, und R^{F} eine perfluorierte Kohlenstoffkette ist,
in Alkoholen, Ethern, Wasser oder in Mischungen aus Wasser und einem organischen Lösungsmittel bei Temperaturen zwischen -10°C und 180°C unter Zusatz von anorganischen und/oder organischen Basen umsetzt, anschließend gegebenenfalls vorhandene Schutzgruppen abspaltet, die so erhaltenen Komplexbildner mit mindestens einem Metalloxid oder Metallsalz eines Elementes der Ordnungszahlen 21-29, 39, 42, 44 oder 57-83 bei Raumtemperatur oder erhöhter Temperatur umsetzt und anschließend - falls gewünscht - vorhandene acide Wasserstoffatome durch Kationen anorganischer und/oder organischer Basen, Aminosäuren oder Aminosäurenamide substituiert,
b) Verbindungen der allgemeinen Formel I mit A in der Bedeutung der allgemeinen Formel VIII herstellt, indem man Verbindungen der allgemeinen Formel 20 mit Verbindungen der allgemeinen Formel 28 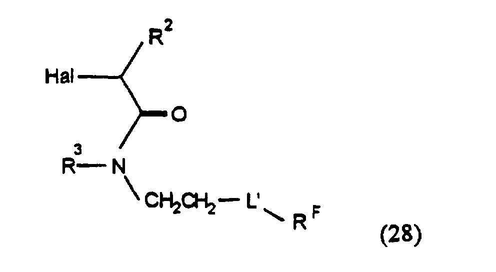 in der R² die Bedeutung von R¹ hat, Hal Chlor, Brom und Jod bedeutet und R^{F}, L' und R³ die oben genannten Bedeutungen haben,
in an sich bekannter Weise alkyliert, anschließend gegebenenfalls vorhandene Schutzgruppen abspaltet und mit den so erhaltenen Komplexbildnern weiter wie unter a) verfährt,
c) Verbindungen der allgemeinen Formel I mit A in der Bedeutung der allgemeinen Formel VII herstellt, indem man Verbindungen der allgemeinen Formel 20 mit Verbindungen der allgemeinen Formel 34 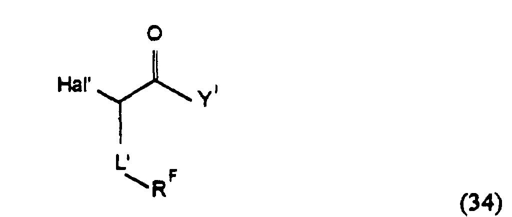 in der Hal' die Bedeutung von Hal, F,-OTs, OMs hat, Y' für die Reste OH und 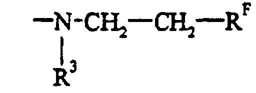 steht und L' und R^{F} die o.g. Bedeutung haben,
in an sich bekannter Weise umsetzt, anschließend gegebenenfalls vorhandene Schutzgruppen abspaltet und mit den so erhaltenen Komplexbildnern wie unter a) verfährt,
d) Verbindungen der allgemeinen Formel I mit A in der Bedeutung der allgemeinen Formel XI mit q in der Bedeutung der Ziffer 0 herstellt, indem man Verbindungen der allgemeinen Formel 20 mit Verbindungen der allgemeinen Formel 68 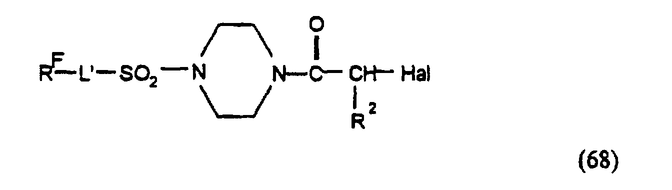 in der R^{F} , L' , R² und Hal die o. g. Bedeutungen haben ,
in einem organischen Lösungsmittel bei erhöhten Temperaturen mehrere Stunden umsetzt, anschließend ggf. vorhandene Schutzgruppen abspaltet und mit den so erhaltenen Komplexbildnern wie unter a) verfährt,
e) Verbindungen der allgemeinen Formel I mit A in der Bedeutung der allgemeinen Formel XI mit q in der Bedeutung der Ziffern 1, herstellt, indem man Verbindungen der allgemeinen Formel 20 mit Verbindungen der allgemeinen Formel 68a 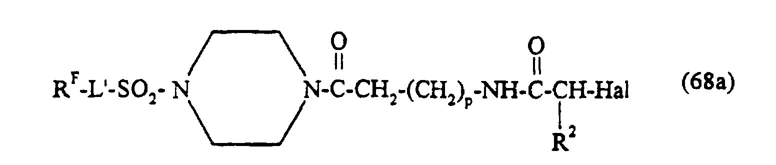 in der R^{F}, L', R³, p und Hal die oben genannten Bedeutungen haben, in einem organischen Lösungsmittel bei erhöhten Temperaturen mehrere Stunden umsetzt, anschließend ggf. vorhandene Schutzgruppen abspaltet und mit den so erhaltenen Komplexbildnern wie unter a) verfährt.

8. Verfahren zur Herstellung von perfluoralkylhaltigen Verbindungen der allgemeinen Formel I, **dadurch gekennzeichnet, daß** man
a) Verbindungen der allgemeinen Formel I mit A in der Bedeutung der allgemeinen Formel II herstellt, indem man für den Fall, daß Y in der allgemeinen Formel II für eine OH-Gruppe steht, Verbindungen der allgemeinen Formel 48 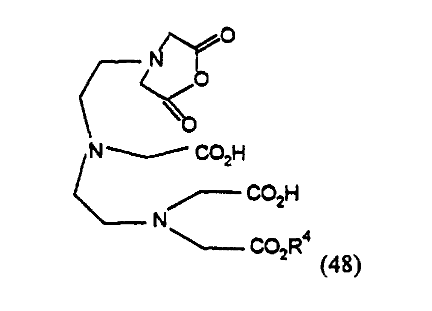 in der R⁴ die o.g. Bedeutung hat,
mit einem Amin der allgemeinen Formel 29 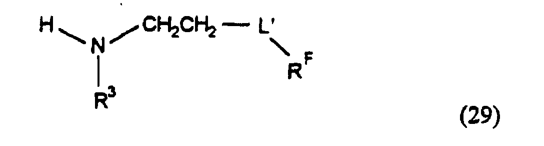 in der R³, L' und R^{F} die o. g. Bedeutungen haben,
in einem organischen Lösungsmittel, gegebenenfalls unter Zusatz von anorganischen und/oder organischen Basen, bei erhöhten Temperaturen umsetzt, anschließend gegebenenfalls vorhandene Schutzgruppen abspaltet, die so erhaltenen Komplexbildner mit mindestens einem Metalloxid oder Metallsalz eines Elementes der Ordnungszahlen 21-29, 39, 42, 44 oder 57-83 bei Raumtemperatur oder erhöhter Temperatur umsetzt und anschließend - falls gewünscht - vorhandene acide Wasserstoffatome durch Kationen anorganischer und/oder organischer Basen, Aminosäuren oder Aminosäurenamide substituiert
bzw. für den Fall, daß Y in der allgemeinen Formel II für die Gruppe 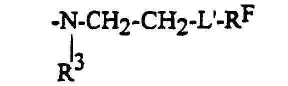 steht, das Bisanhydrid der Diethylentriamin-pentaessigsäure (Merck) der allgemeinen Formel 49 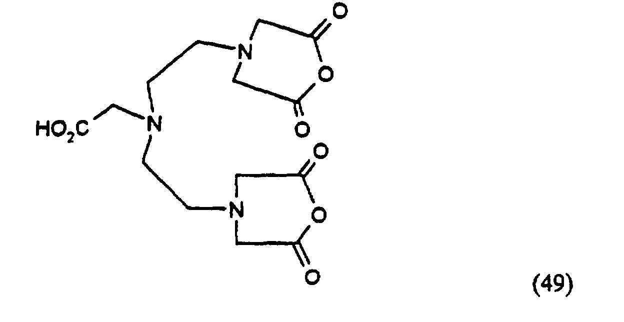 unter analogen Bedingungen mit einem Amin der Formel 29 umsetzt und weiter wie im ersten Fall verfährt,
b) Verbindungen der allgemeinen Formel I mit A in der Bedeutung der allgemeinen Formel XII herstellt, indem man das Bisanhydrid 49 mit Piperazinderivaten der allgemeinen Formel 67 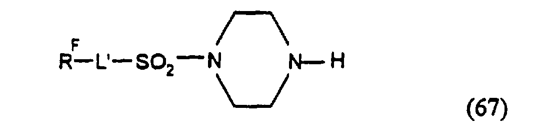 in der R^{F} und L' die o.g. Bedeutung haben,
unter gleichen Bedingungen wie unter a) umsetzt, anschließend gegebenenfalls vorhandene Schutzgruppen abspaltet und weiter wie unter a) verfährt.

9. Verfahren zur Herstellung von perfluoralkylhaltigen Verbindungen der allgemeinen Formel I, **dadurch gekennzeichnet, daß** man
a) Verbindungen der allgemeinen Formel I mit A in der Bedeutung der allgemeinen Formel III herstellt, indem man Halogencarbonsäurederivate der allgemeinen Formel 52 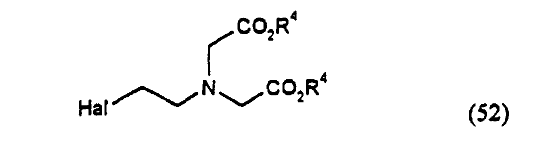 in der Hal und R⁴ die o.g. Bedeutung haben,
mit Verbindungen der allgemeinen Formel 51 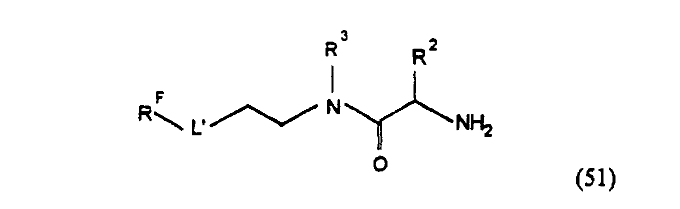 in der R^{F}, L', R² und R³ die o.g. Bedeutung haben,
in an sich bekannter Weise umsetzt, anschließend gegebenenfalls vorhandene Schutzgruppen abspaltet, die so erhaltenen Komplexbildner mit mindestens einem Metalloxid oder Metalisalz eines Elementes der Ordnungszahlen 21-29, 39, 42, 44 oder 57-83 bei Raumtemperatur oder erhöhter Temperatur umsetzt und anschließend - falls gewünscht - vorhandene acide Wasserstoffatome durch Kationen anorganischer und/oder organischer Basen, Aminosäuren oder Aminosäurenamide substituiert
b) Verbindungen der allgemeinen Formel I mit A in der Bedeutung der allgemeinen Formel XIII herstellt, indem man analog zu a) Halogencarbonsäurederivate der allgemeinen Formel 52 mit Piperazinderivaten der allgemeinen Formel 66 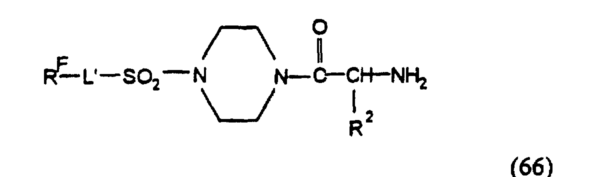 in der R^{F}, L' und R² die o.g. Bedeutung haben,
umsetzt, anschließend gegebenenfalls vorhandene Schutzgruppen abspaltet, und weiter wie unter a) verfährt.

10. Verfahren zur Herstellung von perfluoralkylhaltigen Verbindungen der allgemeinen Formel I, **dadurch gekennzeichnet,**
**daß** man Verbindungen mit A in der Bedeutung der allgemeinen Formel IV herstellt, indem man Hydroxysäuren bzw. -ester der allgemeinen Formel 56 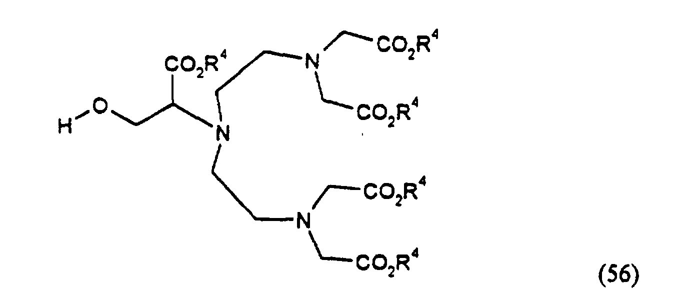 in der R⁴ die o. g. Bedeutung hat
mit Halogenverbindungen der allgemeinen Formel 55
Hal-L'-R^{F} (55)
in der R^{F}, L' und Hal die o. g. Bedeutungen haben,
in einer Mischung aus einem organischen Lösungsmittel und einem Puffer bei leicht alkalischem pH bei Raumtemperatur meherere Stunden umsetzt, anschließend gegebenenfalls vorhandene Schutzgruppen abspaltet, die so erhaltenen Komplexbildner mit mindestens einem Metalloxid oder Metallsalz eines Elementes der Ordnungszahlen 21-29, 39, 42, 44 oder 57-83 bei Raumtemperatur oder erhöhter Temperatur umsetzt und anschließend - falls gewünscht - vorhandene acide Wasserstoffatome durch Kationen anorganischer und/oder organischer Basen, Aminosäuren oder Aminosäurenamide substituiert.

11. Verfahren zur Herstellung von perfluoralkylhaltigen Verbindungen der allgemeinen Formel I, **dadurch gekennzeichnet, daß** man
a) Verbindungen der allgemeinen Formel I mit A in der Bedeutung der allgemeinen Formel V herstellt, indem man a-Halogencarbonsäureester oder -säuren der allgemeinen Formel 18
Hal-CH₂CO₂R⁴ (18)
in der Hal und R⁴ die o.g. Bedeutung haben,
mit Aminen der allgemeinen Formel 39 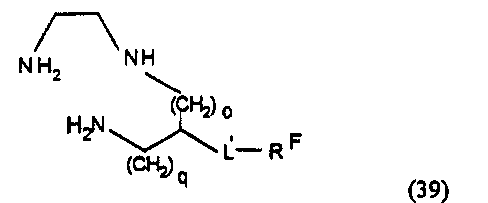 in der L', R^{F}, o und q die genannten Bedeutungen haben
in an sich bekannter Weise umsetzt, anschließend gegebenenfalls vorhandene Schutzgruppen abspaltet, die so erhaltenen Komplexbildner mit mindestens einem Metalloxid oder Metallsalz eines Elementes der Ordnungszahlen 21-29, 39, 42, 44 oder 57-83 bei Raumtemperatur oder erhöhter Temperatur umsetzt und anschließend - falls gewünscht - vorhandene acide Wasserstoffatome durch Kationen anorganischer und/oder organischer Basen, Aminosäuren oder Aminosäurenamide substituiert,
b) Verbindungen der allgemeinen Formel I mit A in der Bedeutung der allgemeinen Formel VI herstellt, indem man a-Halogencarbonsäureester oder-säuren der allgemeinen Formel 18 mit Verbindungen der allgemeinen Formel 36 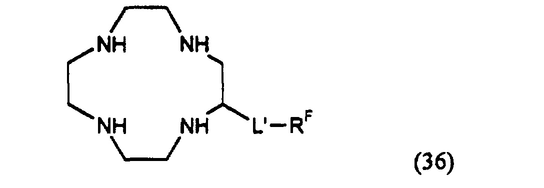 in der L' und R^{F} die o.g. Bedeutungen haben,
in an sich bekannter Weise umsetzt und weiter wie unter a) verfährt.
c) Verbindungen der allgemeinen Formel I mit A in der Bedeutung der allgemeinen Formel X herstellt, indem man a-Halogencarbonsäureester oder -säuren der allgemeinen Formel 18 mit Verbindungen der allgemeinen Formel 70 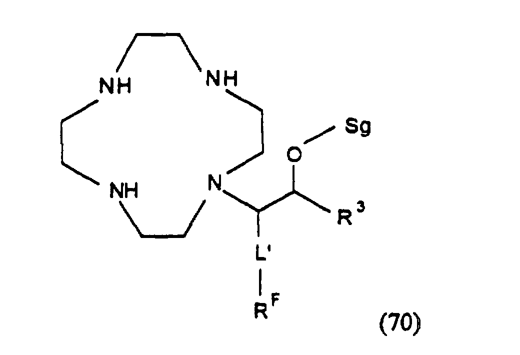 in der R^{F} , L', R³ die oben genannte Bedeutung haben und Sg in
der Bedeutung einer Schutzgruppe steht,
in an sich bekannter Weise umsetzt und weiter wie unter a) verfährt.

12. Pharmazeutische Mittel enthaltend mindestens eine physiologisch verträgliche Verbindung gemäß Anspruch 1, gegebenenfalls mit den in der Galenik üblichen Zusätzen.

13. Verwendung von mindestens einer physiologisch verträglichen Verbindung gemäß Anspruch 1 oder eines pharmazeutischen Mittels gemäß Anspruch 12 zur Herstellung von Kontrastmitteln in der ¹H-NMR-Diagnostik und -Spektroskopie.

14. Verwendung von mindestens einer physiologisch verträglichen Verbindung gemäß Anspruch 1 oder eines pharmazeutischen Mittels gemäß Anspruch 12 zur Herstellung von Kontrastmitteln in der Röntgendiagnostik.

15. Verwendung von mindestens einer physiologisch verträglichen Verbindung gemäß Anspruch 1 oder eines pharmazeutischen Mittels gemäß Anspruch 12 zur Herstellung von pharmazeutischen Mitteln für Radio-Diagnostik und -Therapie.

16. Verwendung gemäß Anspruch 13 oder 14 zur Herstellung von blood-pool-agents.

17. Verwendung gemäß Anspruch 13 oder 14 zur Herstellung von Lymphographika.

## Claims

1. Perfluoroalkyl-containing compounds of general formula I
R^{F}-L-A (I)
in which
R^{F} is a perfluorinated, straight-chain or branched carbon chain with formula -CₙF₂ₙX, in which
X represents a terminal fluorine, chlorine, bromine, iodine or hydrogen atom, and n stands for numbers 4-30,
L means a direct bond, a methylene group, an -NHCO group, a group 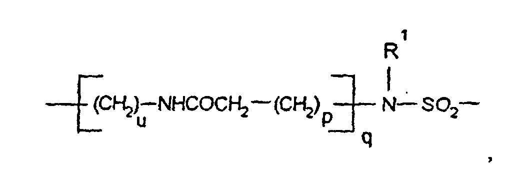 whereby p means numbers 0 to 10, q and u, independently of one another, mean numbers 0 or 1 and
R¹ is a hydrogen atom, a methyl group, a -CH₂-OH group, a -CH₂-CO₂H group or a C₂-C₁₅ chain, which optionally is interrupted by 1 to 3 oxygen atoms, 1 to 2 〉CO groups or an optionally substituted aryl group and/or is substituted with 1 to 4 hydroxyl groups, 1 to 2 C₁-C₄ alkoxy groups, 1 to 2 carboxy groups, a group -SO₃H,
or L is a straight-chain, branched, saturated or unsaturated C₂-C₃₀ carbon chain, which optionally contains 1 to 10 oxygen atoms, 1 to 3 -NR¹ groups, 1 to 2 sulfur atoms, a piperazine, a -CONR¹ group, an -NR¹CO group, an -SO₂ group, an -NR¹-CO₂ group, 1 to 2 -CO-groups, a group 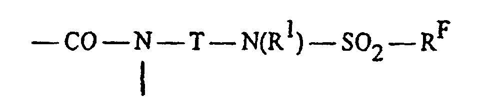 or 1 to 2 optionally substituted aryls and/or is interrupted by these groups and/or is optionally substituted with 1 to 3 -OR¹ groups, 1 to 2 oxo groups, 1 to 2 -NH-COR¹ groups, 1 to 2 -CONHR¹ groups, 1 to 2 -(CH₂)ₚ-CO₂H groups, 1 to 2 groups of -(CH₂)ₚ-(O)_{q}-CH₂CH₂-R^{F}, whereby
R¹, R^{F} and p and q have the above-indicated meanings, and
T means a C₂-C₁₀ chain, which optionally is interrupted by 1 to 2 oxygen atoms or 1 to 2 -NHCO groups,
A stands for a complexing agent or metal complex or their salts of organic and/or inorganic bases or amino acids or amino acid amides, specifically for a complexing agent or complex of general formula II 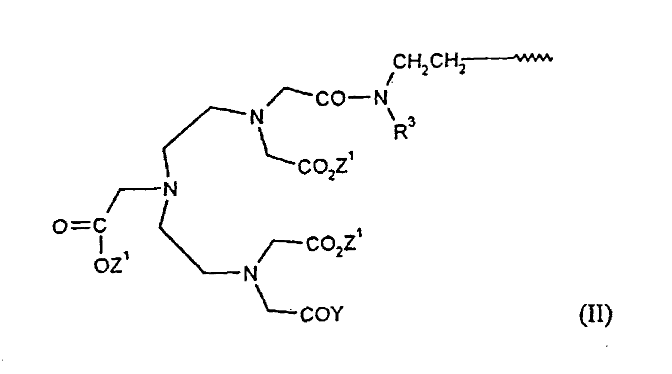 in which R³, Z¹ and Y are independent of one another, and
R³ has the meaning of R¹ or means -(CH₂)m-L-R^{F}, whereby m is 0, 1 or 2 and L and R^{F} have the above-mentioned meaning,
Z¹ independently of one another, mean a hydrogen atom or a metal ion equivalent of atomic numbers 21-29, 39, 42, 44 or 57-83,
Y means -OZ¹ or 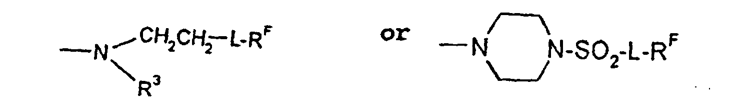 whereby Z¹, L, R^{F} and R³ have the above-mentioned meanings,
or
A stands for a complexing agent or complex of general formula III 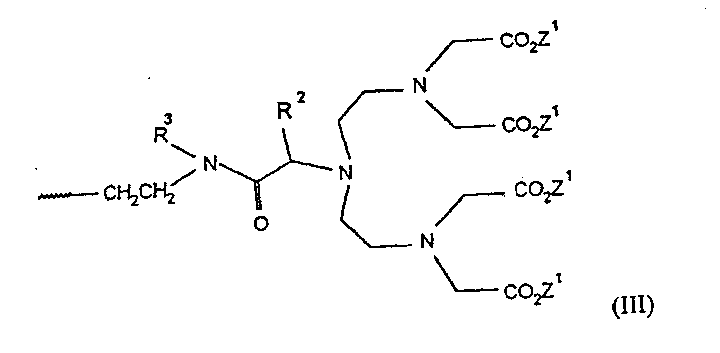 in which R³ and Z¹ have the above-mentioned meanings and R² has the meaning of R¹,
or
A stands for a complexing agent or complex of general formula IV 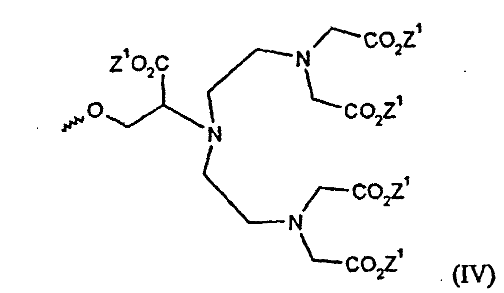 in which Z¹ has the above-mentioned meaning, or
A stands for a complexing agent or complex of general formula V 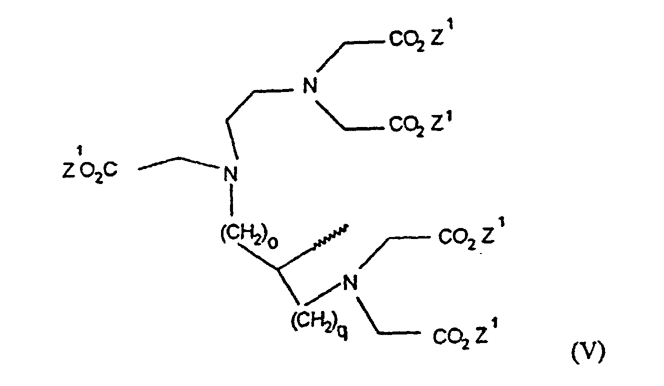 in which Z¹ has the above-mentioned meaning, and o and q stand for numbers 0 or 1 and the sum
o + q = 1 results,
or
A stands for a complexing agent or complex of general formula VI 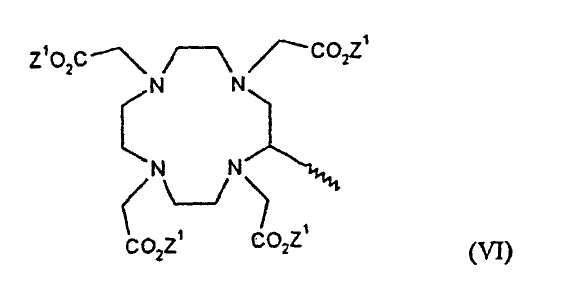 in which Z¹ has the above-mentioned meaning
or
A stands for a complexing agent or complex of general formula VII 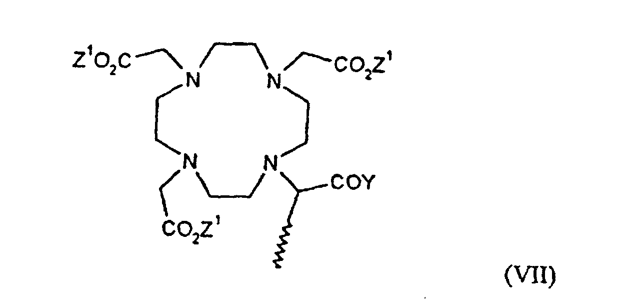 in which Z¹ and Y have the above-mentioned meanings
or
A stands for a complexing agent or complex of general formula VIII 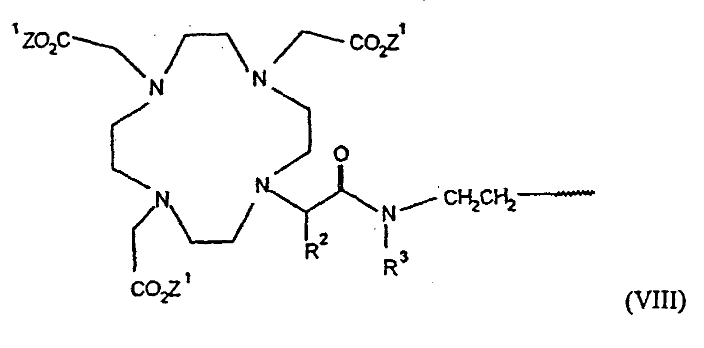 in which R³ and Z¹ have the above-mentioned meanings, and R² has the above-mentioned meaning of R¹,
or
A stands for a complexing agent or complex of general formula IX 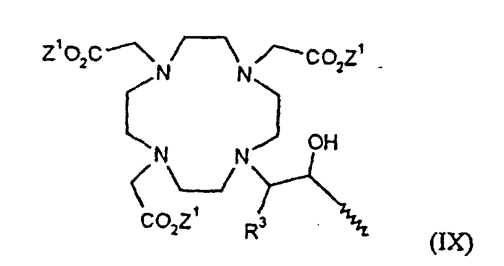 in which R³ and Z¹ have the above-mentioned meanings,
or
A stands for a complexing agent or complex of general formula X 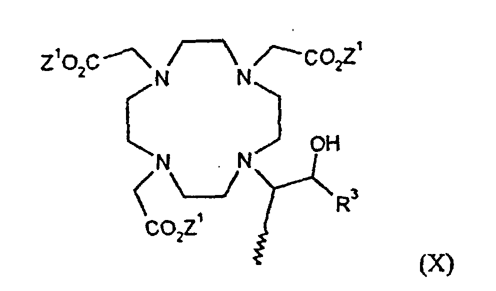 in which R³ and Z¹ have the above-mentioned meanings,
or
A stands for a complexing agent or complex of general formula XI 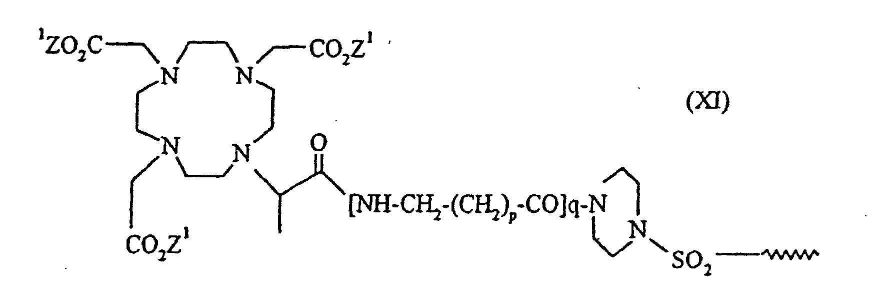 in which Z¹, p and q have the above-mentioned meanings and R² has the meaning of R¹,
or
A stands for a complexing agent or complex of general formula XII 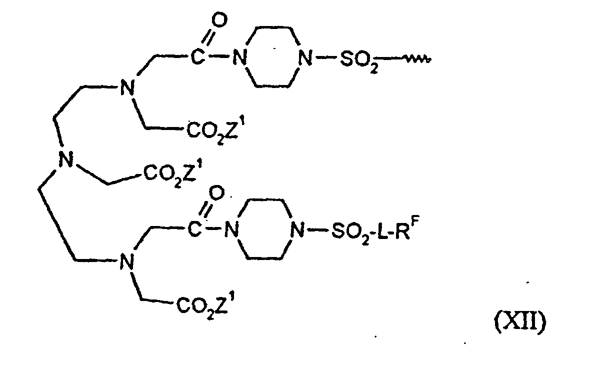 in which L, R^{F} and Z¹ have the above-mentioned meanings,
or
A stands for a complexing agent or complex of general formula XIII 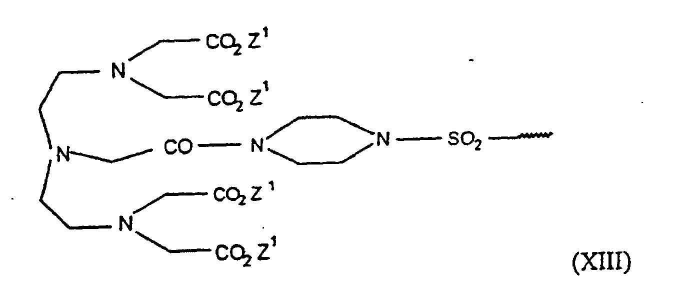 in which Z¹ has the above-mentioned meaning.

2. Compounds according to claim 1, **characterized in that** Z¹ stands for a hydrogen atom.

3. Compounds according to claim 1 or 2, wherein n in formula -CₙF₂ₙ X stands for numbers 4-15.

4. Compounds according to one of claims 1 to 3, wherein X in formula -CₙF₂ₙ X means a fluorine atom.

5. Compounds according to one of claims 1 to 4, wherein L stands for
-CH₂-
-CH₂CH₂-
-(CH₂)ₛ- s = 3 - 15
-CH₂-O-CH₂CH₂-
-CH₂-(O-CH₂-CH₂-)ₜ t = 2 - 6
-CH₂-NH-CO-
-CH₂-NH-CO-CH₂-N(CH₂COOH)-SO₂-
-CH₂-NH-CO-CH₂-N(C₂H₅)-SO₂-
-CH₂-NH-CO-CH₂-N(C₁₀H₂₁)-SO₂-
-CH₂-NH-CO-CH₂-N(C₆H₁₃)-SO₂-
-CH₂-NH-CO-(CH₂)₁₀-N(C₂H₅)-SO₂-
-CH₂-NH-CO-CH₂-N(-CH₂-C₆H₅)-SO₂-
-CH₂-NH-CO-CH₂-N(-CH₂-CH₂-OH)SO₂-
-CH₂-NHCO-(CH₂)₁₀-S-CH₂CH₂-
-CH₂NHCOCH₂-O-CH₂CH₂-
-CH₂NHCO(CH₂)₁₀-O-CH₂CH₂-
-CH₂-C₆H₄-O-CH₂CH₂-
-CH₂-O-CH₂-C(CH₂-OCH₂CH₂-C₆F₁₃)₂-CH₂-OCH₂-CH₂- 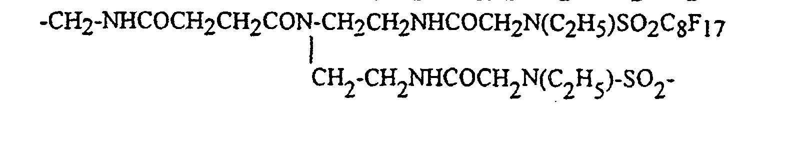
-CH₂-O-CH₂-CH(OC₁₀H₂₁)-CH₂-O-CH₂CH₂-
-(CH₂NHCO)₄-CH₂O-CH₂CH₂-
-(CH₂NHCO)₃-CH₂O-CH₂CH₂-
-CH₂-OCH₂C(CH₂OH)₂-CH₂-O-CH₂CH₂- 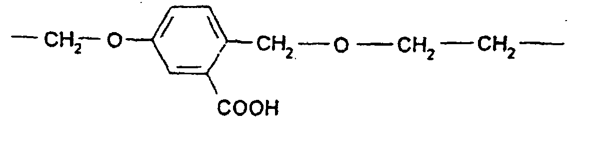
-CH₂NHCOCH₂N(C₆H₅)-SO₂-
-NHCO-CH₂-CH₂-
-NHCO-CH₂-O-CH₂CH₂-
-NH-CO-
-NH-CO-CH₂-N(CH₂COOH)-SO₂-
-NH-CO-CH₂-N(C₂H₅)-SO₂-
-NH-CO-CH₂-N(C₁₀H₂₁)-SO₂-
-NH-CO-CH₂-N(C₆H₁₃)-SO₂-
-NH-CO-(CH₂)₁₀-N(C₂H₅)-SO₂-
-NH-CO-CH₂-N(-CH₂-C₆H₅)-SO₂-
-NH-CO-CH₂-N(-CH₂-CH₂-OH)SO₂-
-NH-CO-CH₂-
-CH₂-O-C₆H₄-O-CH₂-CH₂-
-CH₂-C₆H₄-O-CH₂-CH₂-
-N(C₂H₅)-SO₂-
-N(C₆H₅)-SO₂-
-N(C₁₀H₂₁)-SO₂-
-N(C₆H₁₃)-SO₂-
-N(C₂H₄OH)-SO₂-
-N(CH₂COOH)-SO₂-
-N(CH₂C₆H₅)-SO₂-
-N-[CH(CH₂OH)₂]-SO₂-
-N-[CH(CH₂OH)CH(CH₂OH)]-SO₂-

6. The compounds according to claim 1:
Gadolinium complex of 10-[2-hydroxy-4-aza-5-oxo-7-aza-7-(perfluorooctylsulfonyl)-nonyl]-1,4,7-tris(carboxymethyl)-1,4,7,10-tetraazacyclododecane,
gadolinium complex of 10-[2-hydroxy-4-aza-5-oxo-7-oxa-10,10,11,11,12,12,13,13,14,14,15,15,16,16,17,17,17-heptadecafluoro-heptadecyl]-1,4,7-tris(carboxymethyl)-1,4,7,10-tetraazacyclododecane.

7. Process for the production of perfluoroalkyl-containing compounds of general formula I, wherein
a) Compounds of general formula I with A in the meaning of general formula IX are produced by compounds of general formula 20 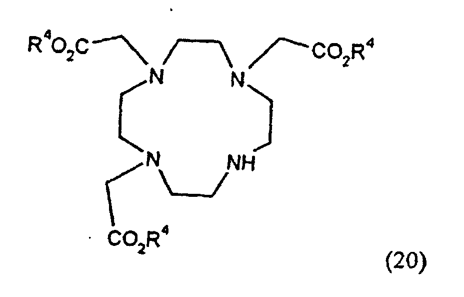 in which
R⁴ means hydrogen, methyl, ethyl, isopropyl, t-butyl or benzyl,
being reacted with epoxides of general formula 21 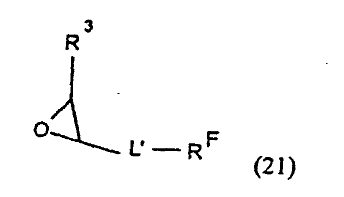 in which
R³ has the meaning of R¹, optionally in protected form, or means -(CH₂)ₘ-L-R^{F}, whereby m can be 0, 1 or 2, L' has the meaning of L, optionally in protected form, and R^{F} is a perfluorinated carbon chain,
in alcohols, ethers, water or in mixtures of water and an organic solvent at temperatures of between -10°C and 180°C with the addition of inorganic and/or organic bases, then optionally present protective groups being cleaved, the thus obtained complexing agents being reacted with at least one metal oxide or metal salt of an element of atomic numbers 21-29, 39, 42, 44 or 57-83 at room temperature or elevated temperature, and then -- if desired -- acidic hydrogen atoms that are present being substituted by cations of inorganic and/or organic bases, amino acids or amino acid amides,
b) compounds of general formula I with A in the meaning of general formula VIII are produced by compounds of general formula 20 being alkylated in a way known in the art with compounds of general formula 28 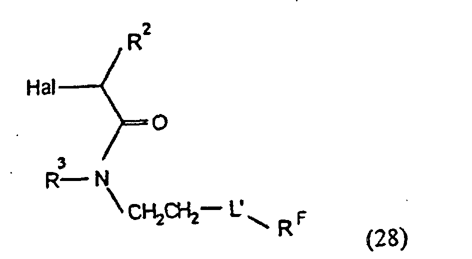 in which R² has the meaning of R¹, Hal meant chlorine, bromine and iodine, and R^{F}, L' and R³ have the above-mentioned meanings,
then optionally present protective groups being cleaved and the procedure further being performed as under a) with the thus obtained complexing agents,
c) compounds of general formula I with A in the meaning of general formula VII are produced by compounds of general formula 20 being reacted in a way known in the art with compounds of general formula 34 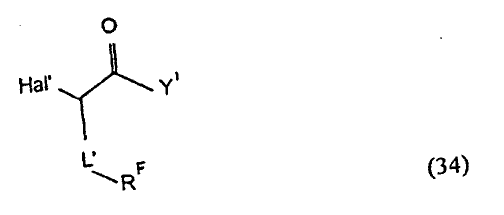 in which Hal' has the meaning of Hal, F, -OTs, OMs, Y' stands for radicals -OH and 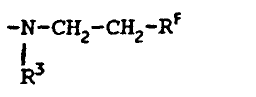 and L' and R^{F} have the above-mentioned meaning, then optionally present protective groups being cleaved and the procedure further being performed as under a) with the thus obtained complexing agents,
d) compounds of general formula I with A in the meaning of general formula XI with q in the meaning of number 0 are produced by compounds of general formula 20 being reacted with compounds of general formula 68 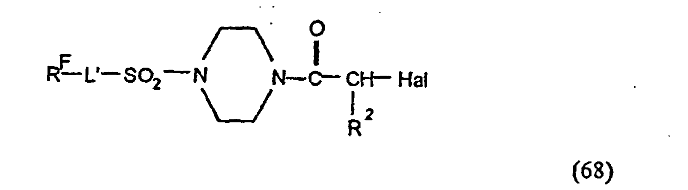 in which R^{F}, L', R² and Hal have the above-mentioned meanings,
in an organic solvent at elevated temperatures for several hours, then optionally present protective groups being cleaved and the procedure further being performed as under a) with the thus obtained complexing agents,
e) compounds of general formula I with A in the meaning of general formula XI with q in the meaning of number 1 are produced by compounds of general formula 20 being reacted with compounds of general formula 68a 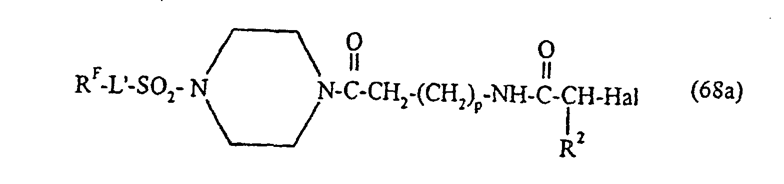 in which R^{F}, L', R³, p and Hal have the above-mentioned meanings,
in an organic solvent at elevated temperatures for several hours, then optionally present protective groups being cleaved and the procedure being performed as under a) with the thus obtained complexing agents.

8. Process for the production of perfluoroalkyl-containing compounds of general formula I, wherein
a) compounds of general formula I with A in the meaning of general formula II are produced by, if Y in general formula II stands for an OH group, compounds of general formula 48 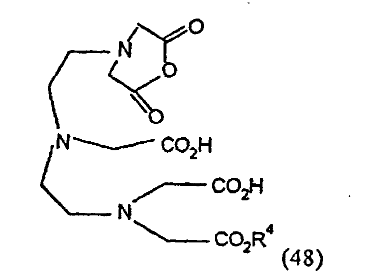 in which R⁴ has the above-mentioned meaning, being reacted with an amine of general formula 29 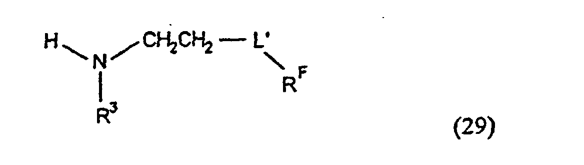 in which R³, L' and R^{F} have the above-mentioned meanings, in an organic solvent, optionally with the addition of inorganic and/or organic bases, at elevated temperatures, then optionally present protective groups being cleaved, the thus obtained complexing agents being reacted with at least one metal oxide or metal salt of an element of atomic numbers 21-29, 39, 42, 44 or 57-83 at room temperature or elevated temperature, and then -- if desired -- acidic hydrogen atoms that are present being substituted by cations of inorganic and/or organic bases, amino acids or amino acid amides, or, if Y in general formula II stands for group 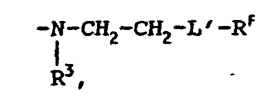 the bisanhydride of the diethylenetriamine-pentaacetic acid (Merck) of general formula 49 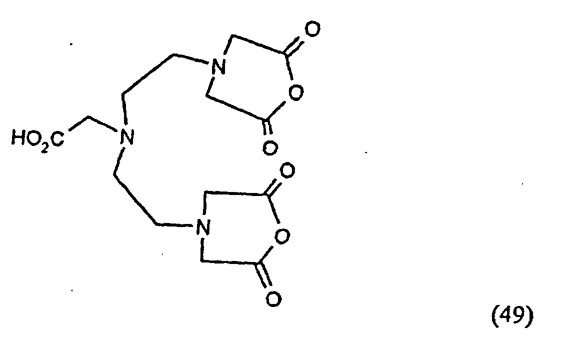 being reacted under analogous conditions with an amine of formula 29 and the procedure further being performed as in the first case,
b) compounds of general formula I with A in the meaning of general formula XII are produced by bisanhydride 49 being reacted with piperazine derivatives of general formula 67 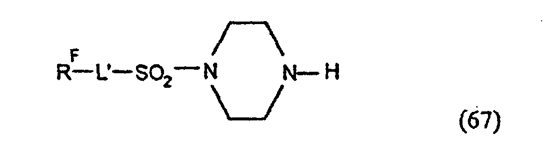 in which R^{F} and L' have the above-mentioned meaning,
under the same conditions as under a), then optionally present protective groups being cleaved and the procedure further being performed as under a).

9. Process for the production of perfluoroalkyl-containing compounds of general formula I, wherein
a) compounds of general formula I with A in the meaning of general formula III are produced by halocarboxylic acid derivatives of general formula 52 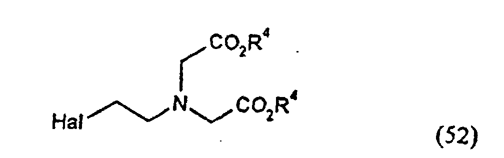 in which Hal and R⁴ have the above-mentioned meaning,
being reacted in a way known in the art with compounds of general formula 51 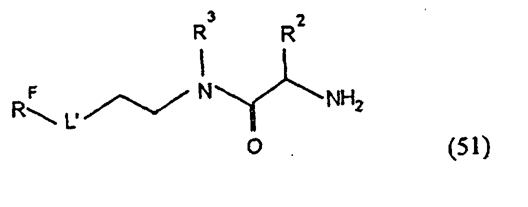 in which R^{F}, L', R² and R³ have the above-mentioned meaning,
then optionally present protective groups being cleaved, the thus obtained complexing agents being reacted with at least one metal oxide or metal salt of an element of atomic numbers 21-29, 39, 42, 44 or 57-83 at room temperature or elevated temperature and then -- if desired -- acidic hydrogen atoms that are present being substituted by cations of inorganic and/or organic bases, amino acids or amino acid amides,
b) compounds of general formula I with A in the meaning of general formula XIII are produced by halocarboxylic acid derivatives of general formula 52 being reacted analogously to a) with piperazine derivatives of general formula 66 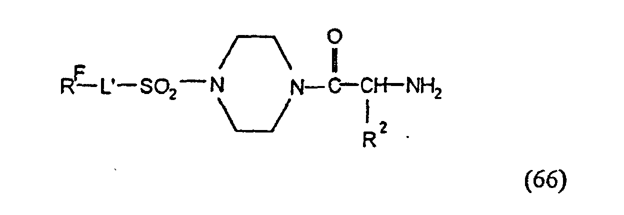 in which R^{F}, L' and R² have the above-mentioned meaning,
then optionally present protective groups being cleaved and the procedure further being performed as under a).

10. Process for the production of perfluoroalkyl-containing compounds of general formula I, wherein compounds with A in the meaning of general formula IV are produced by hydroxy acids or hydroxy esters of general formula 56 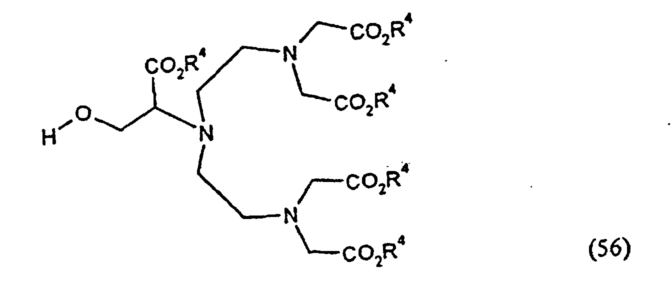 in which R⁴ has the above-mentioned meaning, being reacted with halogen compounds of general formula 55
Hal-L'-R^{F} (55)
in which R^{F}, L' and Hal have the above-mentioned meanings,
in a mixture consisting of an organic solvent and a buffer at slightly alkaline pH at room temperature for several hours, then optionally present protective groups being cleaved, the thus obtained complexing agents being reacted with at least one metal oxide or metal salt of an element of atomic numbers 21-29, 39, 42, 44 or 57-83 at room temperature or elevated temperature and then -- if desired -- acidic hydrogen atoms that are present being substituted by cations of inorganic and/or organic bases, amino acids or amino acid amides.

11. Process for the production of perfluoroalkyl-containing compounds of general formula I, wherein
a) Compounds of general formula I with A in the meaning of general formula V are produced by α-halocarboxylic acid esters or α-halocarboxylic acids of general formula 18
Hal-CH₂CO₂R⁴ (18)
in which Hal and R⁴ have the above-mentioned meaning,
being reacted in a way known in the art with amines of general formula 39 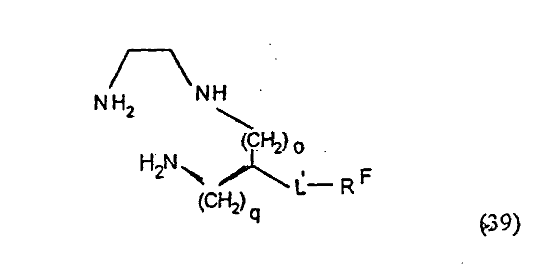 in which L', R^{F}, o and q have the mentioned meanings,
then optionally present protective groups being cleaved, the thus obtained complexing agents being reacted with at least one metal oxide or metal salt of an element of atomic numbers 21-29, 39, 42, 44 or 57-83 at room temperature or elevated temperature and then -- if desired -- acidic hydrogen atoms that are present being substituted by cations of inorganic and/or organic bases, amino acids or amino acid amides,
b) compounds of general formula I with A in the meaning of general formula VI are produced by α-halocarboxylic acid esters or α-halocarboxylic acids of general formula 18 being reacted in a way known in the art with compounds of general formula 36 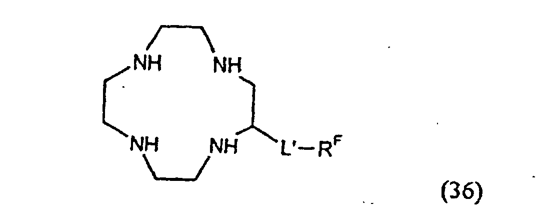 in which L' and R^{F} have the above-mentioned meanings,
and the procedure further is performed as under a),
c) compounds of general formula I with A in the meaning of general formula X are produced by α-halocarboxylic acid esters or α-halocarboxylic acids of general formula 18 being reacted in a way known in the art with compounds of general formula 70 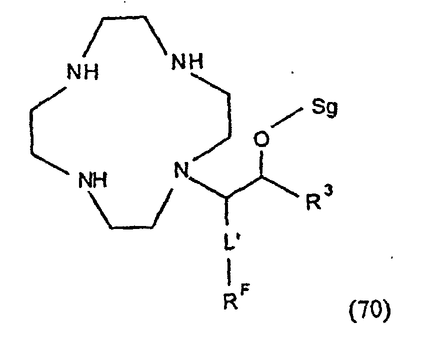 in which R^{F}, L', R³ have the above-mentioned meaning, and Sg is in the meaning of a protective group,
and the procedure further is performed as under a).

12. Pharmaceutical agents that contain at least one physiologically compatible compound according to claim 1, optionally with the additives that are commonly used in galenicals.

13. Use of at least one physiologically compatible compound according to claim 1 or a pharmaceutical agent according to claim 12 for the production of a contrast agent in the ¹H-NMR-diagnosis and ¹H-NMR -spectroscopy.

14. Use of at least one physiologically compatible compound according to claim 1 or a pharmaceutical agent according to claim 12 for the production of a contrast medium in diagnostic radiology.

15. Use of at least one physiologically compatible compound according to claim 1 or a pharmaceutical agent according to claim 12 for the production of pharmaceutical agents for radiodiagnosis and radiotherapy.

16. Use according to claims 13 or 14 for the production of blood-pool agents.

17. Use according to claims 13 or 14 for the production of lymphographic agents.

## Revendications

1. Composés d'alkyle perfluorique de formule générale I
R^{F}-L-A I
où
R^{F} est une chaîne carbonique perfluorée, linéaire ou ramifiée de formule -CₙF₂ₙX,
X représentant un atome de fluor, de chlore, de brome, d'iode ou d'hydrogène et n un nombre de 4 à 30,
L est une liaison directe, un groupe méthylène, un groupe -NHCO, un groupe 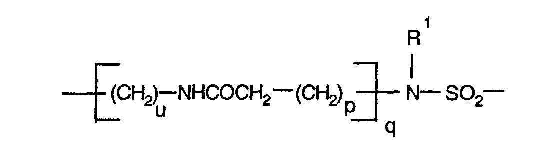 où p représente un nombre de 0 à 10, q et u le nombre 0 ou 1, indépendamment l'un de l'autre,
R¹ étant un atome d'hydrogène, un groupe méthylène, un groupe -CH₂-OH, un groupe -CH₂- CO₂H ou une chaîne C₂-C₁₅, laquelle est le cas échéant interrompue par 1 à 3 atomes d'oxygène, 1 ou 2 groupes >CO ou un groupe aryle éventuellement substitué, et/ou est substituée par 1 à 4 hydroxyles, 1 ou 2 groupes alcoxyles, 1 ou 2 groupes carboxyles, un groupe -SO₃H,
ou bien une chaîne carbonique linéaire, ramifiée, saturée ou insaturée C₂-C₃₀, laquelle contient le cas échéant 1 à 10 atomes d'oxygène, 1 à 3 groupes NR¹, 1 à 2 atomes de soufre, une pipérazine, un groupe -CONR¹, un groupe -NR¹CO, un groupe -SO₂, un groupe -NR¹-SO₂, 1 ou 2 groupes CO, un groupe 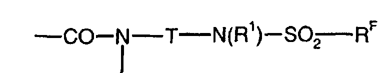 ou 1 ou 2 aryles éventuellement substitués
et/ou est interrompue par ces groupes, et/ou est éventuellement substituée par 1 à 3 groupes -OR¹, 1 ou 2 groupes d'oxo, 1 ou 2 groupes -NH-COR¹, 1 ou 2 groupes -CONHR¹, 1 ou 2 groupes -(CH₂)-CO₂H, 1 ou 2 groupes - (CH₂)ₚ-(O)_{q}-CH₂CH₂-R^{F},
où
R¹, R^{F}, p et q ont les sens exposés plus haut, et où
T renvoie à une chaîne C₂-C₁₀, laquelle est le cas échéant interrompue par 1 ou 2 atomes d'oxygène ou 1 ou 2 groupes - NHCO,
A représentant un complexant, un complexe métallique ou leurs sels de bases organiques et/ou inorganiques, aminoacides ou amides d'aminoacides, et cela pour un complexant ou complexe de formule générale II 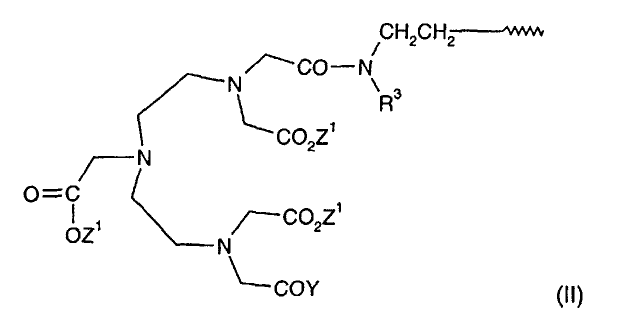 où R³, Z¹ et Y sont indépendants l'un de l'autre et où
R³ représente R¹ ou -(CH₂)ₘ-L- R^{F}, m étant 0, 1 ou 2 et L et R^{F} ayant le sens exposé plus haut,
Z¹ renvoyant, indépendamment l'un de l'autre, à un atome d'hydrogène ou à un équivalent ion-métallique de nombre atomique 21-29, 39, 42, 44 ou 57-83,
Y représentant -OZ¹ ou 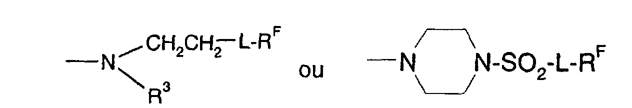 Z¹, L, RF et R³ ayant les sens exposés plus haut,
ou bien
pour un complexant ou un complexe de formule générale III 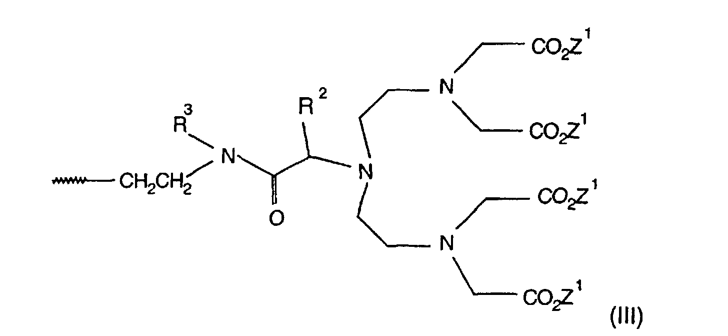 où R³ et Z¹ ont les sens exposés plus haut et où R² représente R¹,
ou bien
pour un complexant ou un complexe de formule générale IV 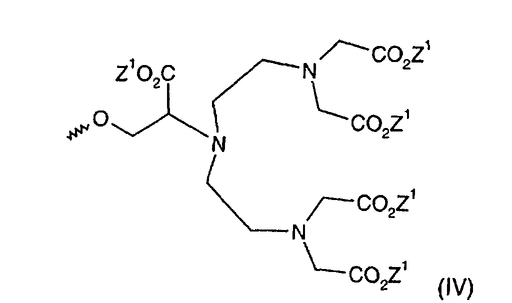 où Z¹ a le sens exposé plus haut,
ou bien
pour un complexant ou un complexe de formule générale V 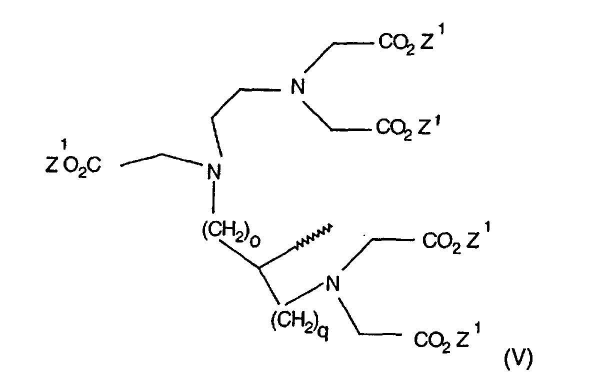 où Z¹ a le sens exposé plus haut et o et q représentent les chiffres 0 ou 1, le total de o + q étant égal à 1,
ou bien
pour un complexant ou un complexe de formule générale VI 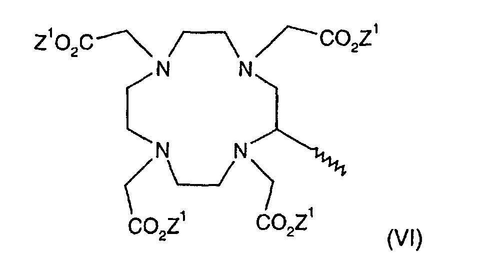 où Z¹ a le sens exposé plus haut,
ou bien
pour un complexant ou un complexe de formule générale VII 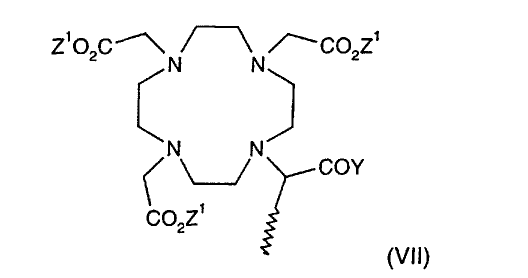 où Z¹ et Y ont les sens exposés plus haut,
ou bien
pour un complexant ou un complexe de formule générale VIII 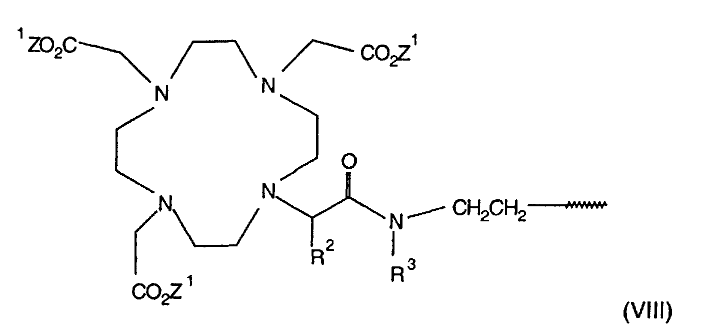 où R³ et Z¹ ont les sens exposés plus haut et R² représente R¹ comme exposé plus haut,
ou bien
pour un complexant ou un complexe de formule générale IX 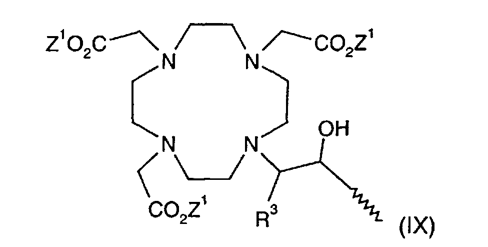 où R³ et Z¹ ont les sens exposés plus haut,
ou bien
pour un complexant ou un complexe de formule générale X 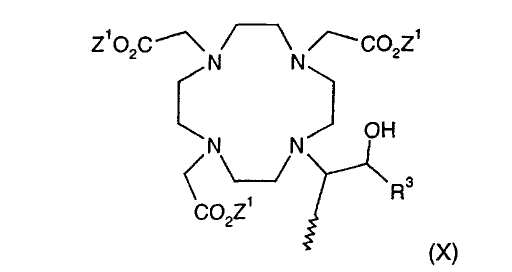 où R³ et Z¹ ont les sens exposés plus haut,
ou bien
pour un complexant ou un complexe de formule générale XI 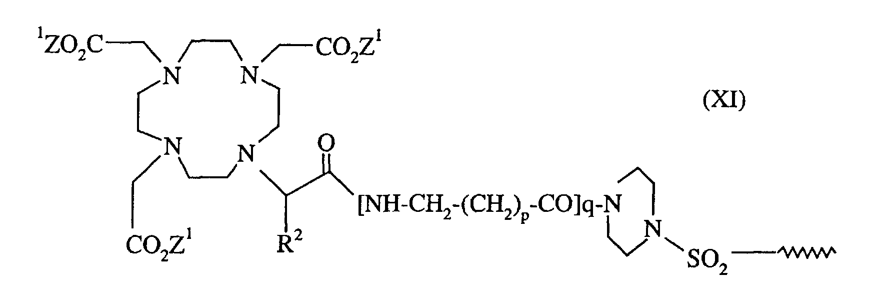 où Z¹ p et q ont le sens exposé plus haut et R² représente R¹,
ou bien
pour un complexant ou un complexe de formule générale XII 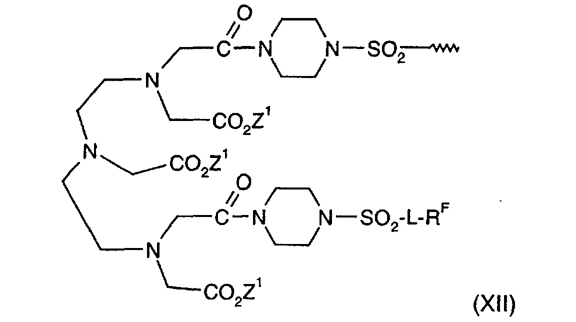 où L, R^{F} et Z¹ ont les sens exposés plus haut,
ou bien
pour un complexant ou un complexe de formule générale XIII 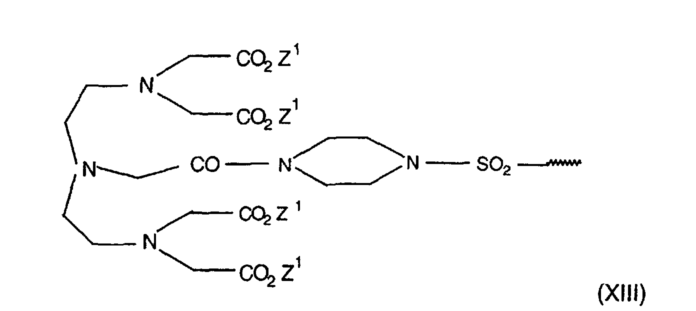 où Z¹ a le sens exposé plus haut,

2. Composés selon la revendication 1, **caractérisés en ce que** Z¹ représente un atome d'hydrogène.

3. Composés selon la revendication 1 ou 2, **caractérisés en ce que** n représente les nombres 4 à 15 dans la formule -CₙF₂ₙX.

4. Composés selon l'une des revendications 1 à 3, **caractérisés en ce que** X représente un atome de fluor dans la formule -CₙF₂ₙX.

5. Composés selon l'une des revendications 1 à 4, **caractérisés en ce que** L représente
-CH₂-
-CH₂CH₂-
-(CH₂)ₛ- s = 3 - 15
-CH₂-O-CH₂CH₂-
-CH₂-(O-CH₂-CH₂-)ₜ t = 2-6
-CH₂-NH-CO-
-CH₂-NH-CO-CH₂-N(CH₂COOH)-SO₂-
-CH₂-NH-CO-CH₂-N(C₂H₅)-SO₂-
-CH₂-NH-CO-CH₂-N(C₁₀H₂₁)-SO₂-
-CH₂-NH-CO-CH₂-N(C₆H₁₃)-SO₂-
-CH₂-NH-CO-(CH₂)₁₀-N(C₂H₅)-SO₂-
-CH₂-NH-CO-CH₂-N(-CH₂-C₆H₅)-SO₂-
-CH₂-NH-CO-CH₂-N(-CH₂-CH₂-OH)SO₂-
-CH₂-NHCO-(CH₂)₁₀-S-CH₂CH₂-
-CH₂NHCOCH₂-O-CH₂CH₂-
-CH₂NHCO(CH₂)₁₀-O-CH₂CH₂-
-CH₂-C₆H₄-O-CH₂CH₂-
-CH₂-O-CH₂-C(CH₂-OCH₂CH₂-C₆F₁₃)₂-CH₂-OCH₂-CH₂- 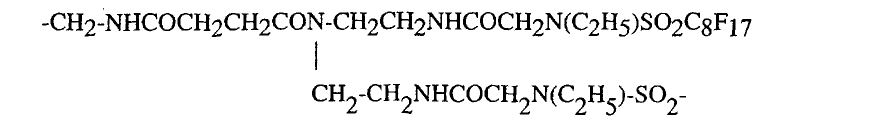
-CH₂-O-CH₂-CH(OC₁₀H₂₁)-CH₂-O-CH₂CH₂-
-(CH₂NHCO)₄-CH₂O-CH₂CH₂-
-(CH₂NHCO)₃-CH₂O-CH₂CH₂-
-CH₂-OCH₂C(CH₂OH)₂-CH₂-O-CH₂CH₂- 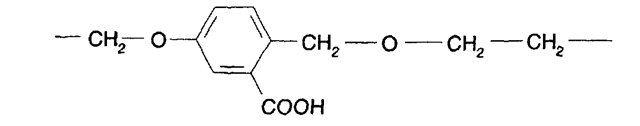
-CH₂NHCOCH₂N(C₆H₅)-SO₂-
-NHCO-CH₂-CH₂-
-NHCO-CH₂-O-CH₂CH₂-
-NH-CO-
-NH-CO-CH₂-N(CH₂COOH)-SO₂-
-NH-CO-CH₂-N(C₂H₅)-SO₂-
-NH-CO-CH₂-N(C₁₀H₂₁)-SO₂-
-NH-CO-CH₂-N(C₆H₁₃)-SO₂-
-NH-CO-(CH₂)₁₀-N(C₂H₅)-SO₂-
-NH-CO-CH₂-N(-CH₂-C₆H₅)-SO₂-
-NH-CO-CH₂-N(-CH₂-CH₂-OH)SO₂-
-NH-CO-CH₂-
-CH₂-O-C₆H₄-O-CH₂-CH₂-
-CH₂-C₆H₄-O-CH₂-CH₂-
-N(C₂H₅)-SO₂-
-N(C₆H₅)-SO₂-
-N(C₁₀H₂₁)-SO₂-
-N(C₆H₁₃)-SO₂-
-N(C₂H₄OH)-SO₂-
-N(CH₂COOH)-SO₂-
-N(CH₂C₆H₅)-SO₂-
-N-[CH(CH₂OH)₂]-SO₂-
-N-[CH(CH₂OH)CH(CH₂OH)]-SO₂-

6. Composés selon la revendication 1 :
complexe gadolinium de 10-[2-hydroxy-4-aza-5-oxo-7-aza-7-(perfluoroctylsulfonyle)-nonyle]-1,4,7-tris(carboxyméthyle)-1,4,7,10-tétraazacyclododécane,
complexe gadolinium de 10-[2-hydroxy-4-aza-5-oxo-7-oxa-10,10,11,11,12,12,13,13,14,14, 15,15,16,16,17,17,17-heptadécafluor-heptadécyle]-1,4,7-tris(carboxyméthyle)-1,4,7,10-tétraazacyclododécane.

7. Procédé pour la préparation de composés d'alkyle perfluorique de formule générale I, **caractérisé en ce que**
a) l'on prépare des composés de formule générale I avec A dans le sens de la formule générale IX en transformant des composés de formule générale 20 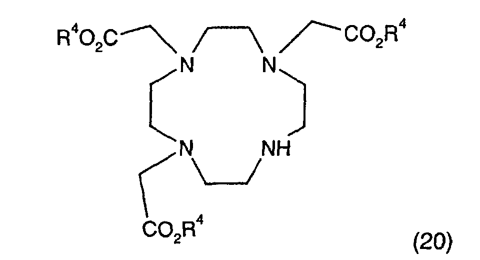 où R⁴ représente de l'hydrogène, du méthyle, de l'éthyle, de l'isopropyle, du t-butyle
ou du benzyle
avec des époxydes de formule générale 21 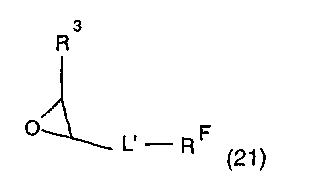 où
R³ a le sens de R¹, le cas échéant sous forme protégée, ou de -(CH₂)ₘ-L- R^{F}, m pouvant être 0, 1 ou 2, L' ayant le sens de L, le cas échéant sous forme protégée, et R^{F} étant une chaîne carbonique perfluorée,
dans des alcools, éthers, de l'eau ou des mélanges d'eau et d'un solvant organique à température comprise entre -10° C et 180° C en additionnant des bases organiques et/ou inorganiques, puis en séparant les groupes protecteurs éventuellement existants, en transformant les complexants ainsi obtenus avec au moins un oxyde ou un sel métallique d'un élément de nombre atomique 21 à 29, 39, 42, 44 ou 57-83 à température ambiante ou élevée, puis en substituant - si cela est souhaité - les atomes d'hydrogène acides existants par des cations de bases inorganiques et/ou organiques, aminoacides ou amides d'aminoacides,
b) l'on prépare des composés de formule générale I avec A dans le sens de la formule générale VII en alkylant de manière connue en soi des composés de formule générale 20 avec des composés de formule générale 28 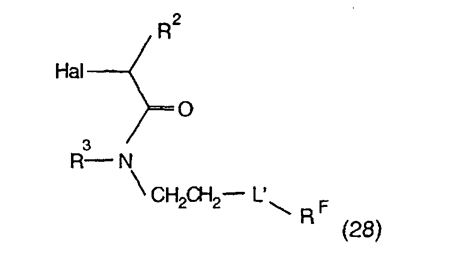 où R² a le sens de R¹, Hal a le sens de chlore, brome et iode et R^{F}, L'et R¹ ont les sens exposés plus haut,
puis en séparant les groupes protecteurs éventuellement existants, et en procédant avec les complexants ainsi obtenus comme en a),
c) l'on prépare des composés de formule générale I avec A dans le sens de la formule générale VII en transformant de manière connue en soi des composés de formule générale 20 avec des composés de formule générale 34 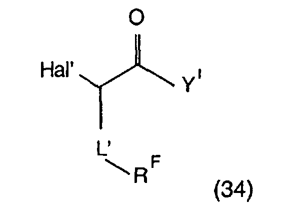 où R² a le sens de R, Hal¹ a le sens de Hal, F, -OTs, OMs, Y' représente l'OH résiduel et 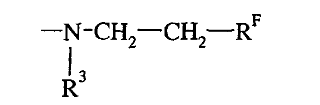 et L' et R^{F} ont les sens exposés plus haut,
puis en séparant les groupes protecteurs éventuellement existants, et en procédant avec les complexants ainsi obtenus comme en a),
d) l'on prépare des composés de formule générale I avec A dans le sens de la formule générale XI avec q représentant le chiffre 0, en transformant des composés de formule générale 20 avec des composés de formule générale 68 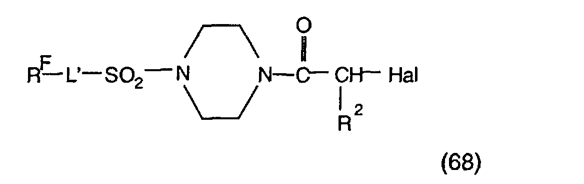 où R^{F}, L', R² et Hal ont les sens exposés plus haut,
dans un solvant organique à température élevée pendant plusieurs heures, puis en séparant les groupes protecteurs éventuellement existants, et en procédant avec les complexants ainsi obtenus comme en a),
e) l'on prépare des composés de formule générale I avec A dans le sens de la formule générale XI avec q représentant les chiffres 1, en transformant des composés de formule générale 20 avec des composés de formule générale 68a 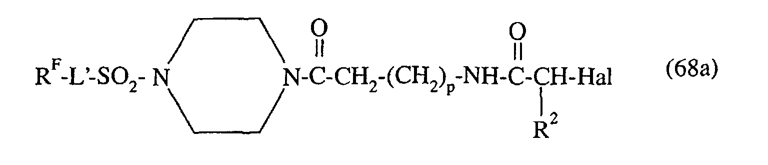
où R^{F}, L', R³, p et Hal ont les sens exposés plus haut, dans un solvant organique à température élevée pendant plusieurs heures, puis en séparant les groupes protecteurs éventuellement existants, et en procédant avec les complexants ainsi obtenus comme en a),

8. Procédé pour la préparation de composés d'alkyle perfluorique de formule générale I,
**caractérisé en ce que**
a) l'on prépare des composés de formule générale I avec A dans le sens de la formule générale II, dans le cas où Y représente un groupe OH dans la formule générale II, en transformant des composés de formule générale 48, 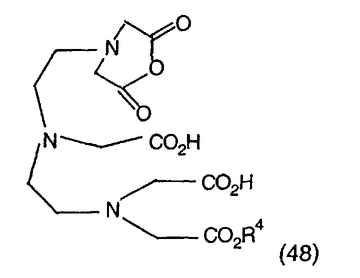 où R⁴ a le sens exposé plus haut,
avec une amine de formule générale 29 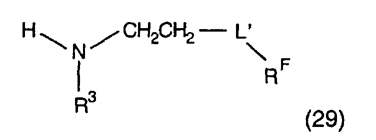 où R³, L' et R^{F} ont les sens exposés plus haut,
dans un solvant organique, en additionnant le cas échéant des bases inorganiques et/ou organiques, à température élevée, puis en séparant les groupes protecteurs éventuellement existants, en transformant les complexants ainsi obtenus avec au moins un oxyde ou un sel métallique d'un élément de nombre atomique 21 à 29, 39, 42, 44 ou 57-83 à température ambiante ou élevée, puis en substituant - si cela est souhaité - les atomes d'hydrogène acides existants par des cations de bases inorganiques et/ou organiques, aminoacides ou amides d'aminoacides,
ou, dans le cas où Y représente le groupe 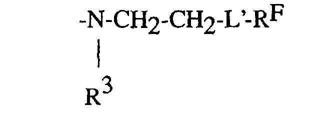 dans la formule générale II, en transformant le bisanhydride d'acide diéthylènetriamine-pentacétique (Merck) de formule générale 49 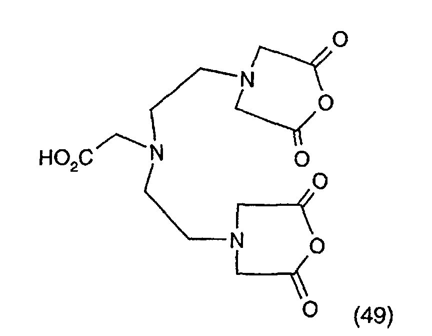 dans des conditions analogues avec une amine de formule 29 et en procédant ensuite comme dans le premier cas,
b) l'on prépare des composés de formule générale I avec A dans le sens de la formule générale XII, en transformant le bisanhydride 49 avec des dérivés pipérazine de formule générale 67 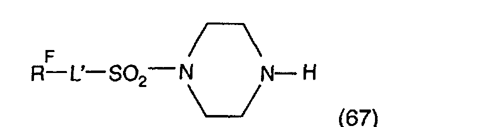
où R^{F} et L' ont le sens exposé plus haut,
dans les mêmes conditions qu'en a), puis en séparant les groupes protecteurs éventuellement existants, et en procédant ensuite comme en a).

9. Procédé pour la préparation de composés d'alkyle perfluorique de formule générale 1,
**caractérisé en ce que**
a) l'on prépare des composés de formule générale I avec A dans le sens de la formule générale III, en transformant de manière connue en soi des dérivés d'hydracide halogéné de formule générale 52, 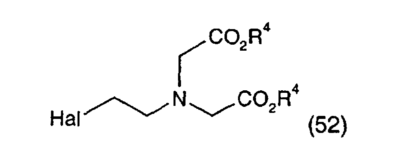 où Hal et R⁴ ont le sens exposé plus haut,
avec des composés de formule générale 51 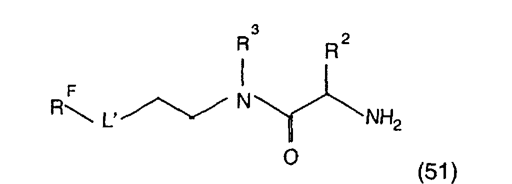 où R^{F}, L', R² et R³ ont les sens exposés plus haut,
puis en séparant les groupes protecteurs éventuellement existants, en transformant les complexants ainsi obtenus avec au moins un oxyde ou un sel métallique d'un élément de nombre atomique 21 à 29, 39, 42, 44 ou 57-83 à température ambiante ou élevée, puis en substituant - si cela est souhaité - les atomes d'hydrogène acides existants par des cations de bases inorganiques et/ou organiques, aminoacides ou amides d'aminoacides,
b) l'on prépare des composés de formule générale I avec A dans le sens de la formule générale XIII, en transformant de manière analogue à a) des dérivés d'hydracide halogéné de formule générale 52 avec des dérivés pipérazine de formule générale 66, 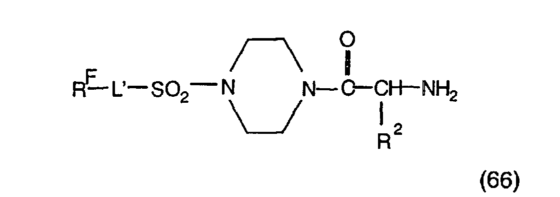
où R^{F}, L', et R² ont les sens exposés plus haut,
puis en séparant les groupes protecteurs éventuellement existants, et en procédant ensuite comme en a).

10. , Procédé pour la préparation de composés d'alkyle perfluorique de formule générale I,
**caractérisé en ce que**
l'on prépare des composés avec A dans le sens de la formule générale IV, en transformant des acides ou esters hydroxyliques de formule générale 56, 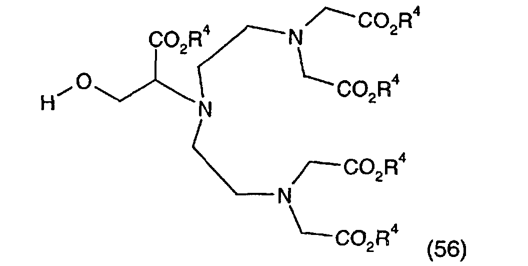 où R⁴ a le sens exposé plus haut,
avec des composés halogénés de formule générale 55
Hal - L'- R^{F} (55)
où R^{F}, L' et Hal ont les sens exposés plus haut,
dans un mélange d'un solvant organique et d'un tampon avec un pH légèrement alcalin, pendant plusieurs heures à température ambiante, puis en séparant les groupes protecteurs éventuellement existants, en transformant les complexants ainsi obtenus avec au moins un oxyde ou un sel métallique d'un élément de nombre atomique 21 à 29, 39, 42, 44 ou 57-83 à température ambiante ou élevée, puis en substituant - si cela est souhaité - les atomes d'hydrogène acides existants par des cations de bases inorganiques et/ou organiques, aminoacides ou amides d'aminoacides.

11. Procédé pour la préparation de composés d'alkyle perfluorique de formule générale I,
**caractérisé en ce que**
a) l'on prépare des composés de formule générale I avec A dans le sens de la formule générale V, en transformant de manière connue en soi des acides ou esters d'a-hydracide halogéné de formule générale 18,
Hal-CH₂CO₂R⁴ (18)
où Hal et R⁴ ont le sens exposé plus haut,
avec des amines de formule générale 39 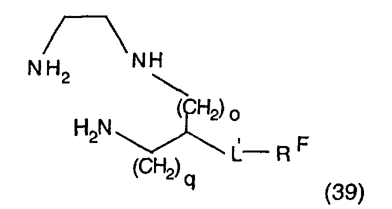 où L', R^{F}, o et q ont les sens exposés plus haut,
puis en séparant les groupes protecteurs éventuellement existants, en transformant les complexants ainsi obtenus avec au moins un oxyde ou un sel métallique d'un élément de nombre atomique 21 à 29, 39, 42, 44 ou 57-83 à température ambiante
ou élevée, puis en substituant - si cela est souhaité - les atomes d'hydrogène acides existants par des cations de bases inorganiques et/ou organiques, aminoacides ou amides d'aminoacides,
b) l'on prépare des composés de formule générale I avec A dans le sens de la formule générale VI, en transformant de manière connue en soi des acides ou esters d'a-hydracide halogéné de formule générale 18 avec des composés de formule générale 36, 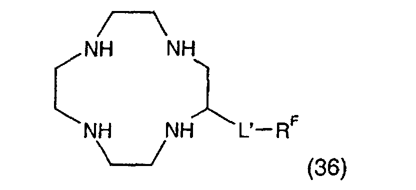 où L' et R^{F} ont les sens exposés plus haut,
et en procédant ensuite comme en a).
c) l'on prépare des composés de formule générale I avec A dans le sens de la formule générale X, en transformant de manière connue en soi des acides ou esters d'a-hydracide halogéné de formule générale 18 avec des composés de formule générale 70, 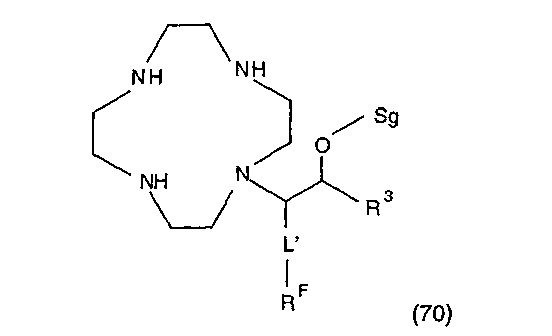
où R^{F}, L', R³ ont le sens exposé plus haut, et Sg représente un groupe protecteur,
et en procédant ensuite comme en a).

12. Agent pharmaceutique contenant au moins un composé physiologiquement compatible selon la revendication 1, le cas échéant avec les additifs usuels en galénique.

13. Application d'au moins un composé physiologiquement compatible selon la revendication 1, ou d'un agent pharmaceutique selon la revendication 12, pour la préparation d'agents contrastants en diagnostic et spectroscopie ¹H-NMR.

14. Application d'au moins un composé physiologiquement compatible selon la revendication 1, ou d'un agent pharmaceutique selon la revendication 12 pour la préparation d'agents contrastants en diagnostic aux rayons X.

15. Application d'au moins un composé physiologiquement compatible selon la revendication 1, ou d'un agent pharmaceutique selon la revendication 12 pour la préparation d'agents pharmaceutiques pour le diagnostic et la thérapie radiographiques.

16. Application selon la revendication 13 ou 14 pour la préparation d'agents blood-pool.

17. Application selon la revendication 13 ou 14 pour la préparation de lymphographies.
